# EUROPEAN PATENT APPLICATION

(11) **EP 4 752 155 A1**
(43) Date of publication of application: **03.06.2026**
(21) Application number: 24216433.3
(22) Date of filing: 29.11.2024
(51) Int. Cl.: C07K 14/47, G01N 33/58, C12N 15/85, C12Q 1/6897

(54) **CELLULAR IRON BIOSENSORS**

(71) Applicant: Medizinische Universität Graz, 8010 Graz (AT)
(72) Inventor: AKYOL, Ali, 8010 Graz (AT); GRAIER, Wolfgang, 8010 Graz (AT); MALLI, Roland, 8010 Graz (AT)
(74) Representative: Maiwald GmbH

(57) **Abstract**

The present invention relates to genetically encoded cellular iron biosensors, in particular to nucleic acids encoding the same, vectors and cells comprising the nucleic acid(s), *in vitro* methods for measuring, and optionally monitoring, cellular Fe²⁺ and/or cellular iron-sulfur cluster (ISC), *in vitro* screening methods for a compound that affects cellular Fe²⁺ and/or cellular ISC, and to *in vitro* methods for identifying a dosage and/or formulation of a compound that affects cellular Fe²⁺ and/or cellular ISC.

## Description

### TECHNICAL FIELD

The present invention relates to genetically encoded cellular iron biosensors, in particular to nucleic acids encoding the same, vectors and cells comprising the nucleic acid(s), *in vitro* methods for measuring, and optionally monitoring, cellular Fe²⁺ and/or cellular iron-sulfur cluster (ISC), *in vitro* screening methods for a compound that affects cellular Fe²⁺ and/or cellular ISC, and to *in vitro* methods for identifying a dosage and/or formulation of a compound that affects cellular Fe²⁺ and/or cellular ISC.

### BACKGROUND OF THE INVENTION

Iron is an essential trace element that plays a central role in many biological processes. Dysregulation of cellular iron balance can lead to serious health problems, including neurodegenerative diseases, cancer and cell aging. The critical role of cellular iron homeostasis in maintaining proper cellular function is well recognized, and disturbances in iron regulation can lead to cellular dysfunction and various diseases. However, the current understanding of the dynamic changes in cellular iron levels remains limited.

Several approaches have been developed for assessing cellular iron, e.g. using fluorescence microscopy. Some approaches are based on low molecular weight fluorescent iron indicators. These indicators can interact directly with iron ions in cells. For example, calcein can be used as a fluorophore, which is quenched when interacting with Fe²⁺ ions inside the cells, but less so with Fe³⁺. Conversely, fluorophores such as FeRhoNox can show increased fluorescence upon reaction with Fe²⁺ and serve as "turn-on" iron indicators. However, these low molecular weight fluorescent iron indicators have drawbacks, including cytotoxicity, subcellular accumulation, unfavourable iron affinities, and irreversible changes due to iron binding, which make a dynamic assessment of iron changes difficult. Low molecular weight fluorescent iron indicators respond typically only to large, non-physiological changes in a cell's labile Fe²⁺ pool and thus, offer little insight into physiological changes in iron status and cellular iron availability. Other approaches are based on genetically encoded biosensors such as nitric oxide (NO) sensors that undergo a conformational change upon target ion binding. For example, NO biosensors utilize fluorescence quenching by nitrosylation. NO biosensors typically comprise a GAF domain, which is a bacterial non-heme iron binding site. The integration of Fe²⁺ into the GAF domain, which is essential for their functionality, may enable estimation of the (sub-)cellular iron content. However, NO sensors require an excess of reactive NO radicals to trigger the fluorescence quenching signal, which limits its qualitative assessment of cellular Fe²⁺ and dynamic measurement capabilities. Thus, currently available methods for assessing cellular iron status are not yet fully adequate, which limits the understanding cellular of iron homeostasis and its effects on physiological and pathological processes, and thus, its role in health and disease. Hence, there is still a need to have at hand alternative solutions for being able to assess cellular Fe²⁺ its and/or changes.

### DETAILED DESCRIPTION OF THE INVENTION

The present application addresses the need for cellular Fe²⁺ biosensors that can translate dynamic changes in cellular iron status into a measurable signal by providing the embodiments recited in the claims.

In particular, a genetically encoded biosensor system that is capable of measuring, and optionally monitoring and/or visualising, iron status of a cell has been developed. The genetically encoded Fe²⁺ biosensor system, herein referred to also as Live-Cell Iron Mapping System (LIMS), comprises Module 1 and/or Module 2. Modules 1 and 2 refer to genetically encoded biosensors with high specificity and sensitivity for cellular Fe²⁺ and/or cellular ISC. LIMS does not leverage FRET or quenching effects resulting from conformational changes of a fusion protein comprising a reporter domain. LIMS leverages effects of Fe²⁺-sensitive domains, iron responsible elements (IREs) and/or Fe²⁺ and/or ISC sensitive regions on the amount of a reporter protein domain within a cell. More specifically, LIMS leverages effects of cellular Fe²⁺ and/or ISC on transcription, mRNA stability, translation and/or polypeptide stability of a polypeptide that comprises or is a reporter protein domain. Thus, an intensity of a signal obtained from a reporter protein domain can be indicative of an amount of cellular Fe²⁺ and/or ISC. More specifically, Module 1 is based on changes in the amount of a polypeptide, which comprises a reporter protein domain fused to an Fe²⁺-sensitive domain, in dependency of the cellular labile iron pool (LIP). Thus, Module 1 can be especially advantageous for assessing cellular LIP and/or changes thereof. Module 2 is based on changes in the amount of a reporter protein domain, via i) IREs and/or ii) Fe²⁺ and/or ISC sensitive regions, wherein said changes are caused by changes in transcription, mRNA stability and/or translation in dependency of cellular Fe²⁺ and/or ISC availability. Thus, Module 2 can be especially advantageous for assessing cellular iron excess or deficiency. By leveraging advantages of Module 1 and/or Module 2, LIMS can enable dynamic measurements, optionally also monitoring and/or visualization, of cellular iron homeostasis and its regulation. Thus, LIMS can provide comprehensive insights into iron dynamics in living cells *in vitro.*

Herein, the term "iron" refers to ferrous iron if not explicitly specified otherwise. Furthermore, the terms "ferrous iron", "Fe²⁺", "Fe²⁺", and "Fe (II)" are used interchangeably herein.

### Cellular iron status regulation

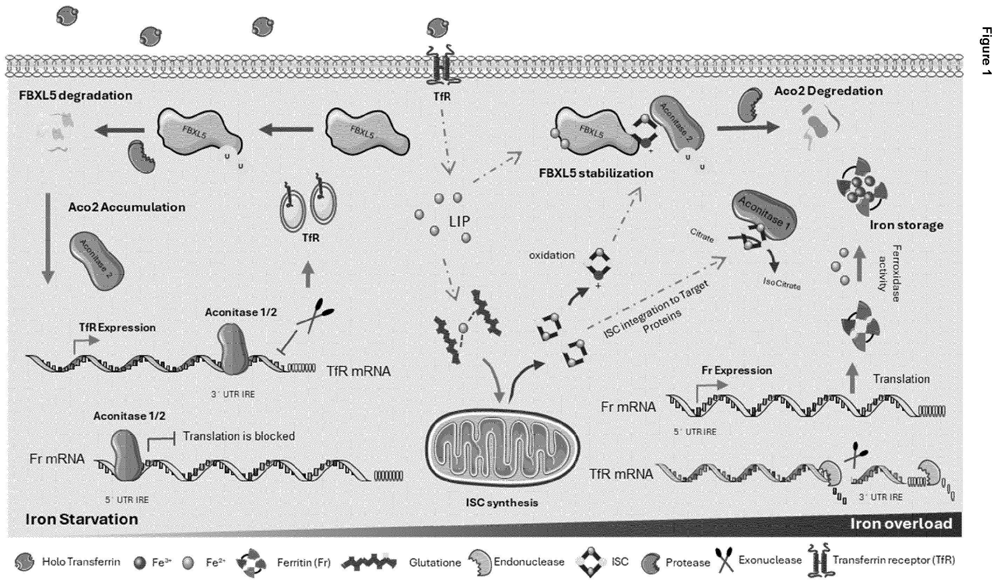

Within a cell, Fe²⁺ is present in the form of a labile iron pool (LIP; cf. **Figure** 1). LIP can rise through extracellular intake of Fe²⁺ or ferritin recycling (ferritinophagy). The regulation of the cellular iron status is controlled by the expression of key proteins, such as the transferrin receptor (TfR) and ferritin (Fr), to maintain optimal iron levels within a cell. As illustrated and summarized in **Figure** 1, a feedback loop is involved in the regulation of cellular Fe²⁺. The respective regulatory mechanism is assumed to involve iron-responsive elements (IREs) and iron regulatory proteins (IRPs), including aconitase 1 (Aco1, also known as IRP1) and aconitase 2 (Aco2, also called IRP2). In contrast to IRP1, IRP2 lacks an iron-sulfur cluster (ISC) binding domain. Thus, IRP2 can, e.g., modulate Fr synthesis and stabilize TfR mRNA differently from IRP1. Stability of IRP2 appears to be regulated by FBXL5, a protein that is assumed to target IRP2 for proteasomal degradation in the presence of Fe²⁺. In low-iron conditions, FBXL5 is degraded through ubiquitination, which leads to an accumulation of IRP2 and a promotion of TfR expression to increase iron uptake. With rising LIP, IRP2 may be progressively downregulated. The resulting stabilization of FBXL5 potentially is assumed to mark a level of iron availability sufficient for the cell. It is further hypothesized that oxidized ISC levels may be indicative of ISC needs, and thus potentially also Fe²⁺ needs, of the cell being met. This, oxidized ISC may enable both IRPs to detach from their respective target mRNAs, thereby potentially promoting Fr synthesis to lower excess LIP.

### Module 1

In a first aspect, the present invention relates to a nucleic acid, comprising
a. A promoter region;
b. A first coding region encoding a first polypeptide,
   wherein the first polypeptide comprises a first reporter protein domain and a first Fe²⁺-sensitive domain;
c. A polypeptide separator region; and
d. A second coding region encoding a second polypeptide,
   wherein the second polypeptide comprises a second reporter protein domain, and wherein the first reporter protein domain and the second reporter protein domain are different.

It has surprisingly been found that advantages conferred by a Fe²⁺-sensitive domain fused to a reporter protein domain can be successfully leveraged for an Fe²⁺ biosensor. When the Fe²⁺-sensitive domain causes stabilization or degradation of the fusion polypeptide or at least its reporter protein domain in dependency of cellular Fe²⁺, a signal obtained from the reporter protein domain can be indicative for cellular Fe²⁺. Thus, a biosensor according to Module 1 is advantageously for and/or capable of measuring changes in the cellular labile iron pool (LIP) via changes in the amount of cellular Fe²⁺. Hence, Module 1 can offer several advantages, including high sensitivity and specificity to cellular Fe²⁺. Module 1 can enable monitoring of cellular LIP dynamics and/or of cellular iron changes with temporal and/or spatial resolution. Module 1 can be suitable for long-term studies and live cell imaging *in vitro.* Module 1 can also be advantageous for screening iron-based therapeutics such as nanoparticle-based approaches.

Herein, the term "nucleic acid" refers to a chain of nucleotides and is interchangeably used with the term "polynucleotide". A skilled person has the general knowledge that nucleic acids are polynucleotides, which can be hydrolysed into monomeric "nucleotides". The monomeric nucleotides can be hydrolysed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means. The term "polynucleotide" as used herein is to be interpreted broadly and thus, includes single or double-stranded polymer of deoxyribonucleotide or ribonucleotide bases, DNA and RNA, including modified DNA and RNA. The term "nucleic acid" also refers to a consecutive list of abbreviations, letters, characters or words, which represent nucleotides. The terms "nucleic acid" and "nucleotide" both encompass naturally occurring or synthetic or artificial nucleic acids or nucleotides, including but not limited to deoxyribonucleotides or ribonucleotides or any nucleotide analogue and polymers or hybrids thereof in either single- or double-stranded, sense or antisense form. Unless otherwise indicated, a particular nucleic acid implicitly encompasses the sequence indicated, conservatively modified variants thereof (e.g., degenerate codon substitutions) and complementary sequences.

Herein, the term "DNA" refers to the usual abbreviation for deoxyribonucleic acid. It is a nucleic acid molecule, i.e. a polymer consisting of nucleotide monomers. These nucleotides are usually deoxy-adenosine-monophosphate, deoxy-thymidine-monophosphate, deoxy-guanosine-monophosphate and deoxy-cytidine-monophosphate monomers or analogs thereof which are - by themselves - composed of a sugar moiety (deoxyribose), a base moiety and a phosphate moiety, and polymerize by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first strand typically hybridize with the nucleotides of the second strand, e.g. by A/T-base-pairing and G/C-base-pairing.

Herein, the term "RNA" relates to a nucleic acid molecule which includes ribonucleotide monomers. For example, the RNA may contain all or a majority of ribonucleotide monomers. RNA encompasses mRNA. Herein, the term "mRNA" relates to an RNA transcript which encodes a peptide or a polypeptide. An mRNA may contain a cap structure, a 5' untranslated region (5'-UTR), a peptide coding region, a 3' untranslated region (3'-UTR) and a polyA tail. An RNA may be produced by *in vitro* transcription or chemical synthesis. These methods are known by the skilled person in the art.

### Module 1 - Promoter region

Herein, the term "promoter region" refers to a DNA sequence which, when operably linked to a coding region is capable of controlling the transcription of the coding region into RNA. A promoter region is located 5' (i.e., upstream), proximal to the transcriptional start site of a nucleotide sequence of interest whose transcription into RNA it controls and provides a site for specific binding by RNA polymerase and other transcription factors for initiation of transcription. The promoter region does not comprise exon and/or intron regions or 5' untranslated regions. The promoter region may for example be heterologous or homologous to the respective cell. A polynucleotide sequence is "heterologous to" an organism or a second polynucleotide sequence if it originates from a foreign species, or, if from the same species, is modified from its original form. For example, a promoter region operably linked to a heterologous coding region refers to a coding region from a species different from that from which the promoter region was derived, or, if from the same species, a coding region which is not naturally associated with the promoter region (e.g. a genetically engineered coding region or an allele from a different ecotype or variety).

Herein, the term "natural" means with respect to an organism, polypeptide, or nucleic acid, that said organism is naturally occurring or available in at least one naturally occurring organism which is not changed, mutated, or otherwise manipulated by man.

The term "operably linked" is to be understood as meaning, for example, the sequential arrangement of a regulatory element (e.g. a promoter region) with a coding region to be expressed and, if appropriate, further regulatory elements (such as e.g., a terminator) in such a way that each of the regulatory elements can fulfil its intended function to allow, modify, facilitate or otherwise influence expression of said coding region. To this end, direct linkage in the chemical sense is not necessarily required. Genetic control sequences such as, e.g., enhancer sequences, can also exert their function on the target sequence from positions which are further away, or indeed from other DNA molecules. Preferred arrangements are those in which the coding region to be expressed recombinantly is positioned downstream, i.e. 3' located of, the promoter region, so that the two regions are linked covalently to each other. Operable linkage can be generated by means of customary recombination and cloning techniques as described (e.g., in Maniatis T, Fritsch EF and Sambrook J (1989) Molecular Cloning: A Laboratory Manual, 2nd Ed., Cold Spring Harbor Laboratory, Cold Spring Harbor (NY); Silhavy et al. (1984) Experiments with Gene Fusions, Cold Spring Harbor Laboratory, Cold Spring Harbor (NY); Ausubel et al. (1987) Current Protocols in Molecular Biology, Greene Publishing Assoc. and Wiley Interscience). Further sequences, which, e.g., act as a linker with specific cleavage sites for restriction enzymes, or as a signal peptide, may also be positioned between promoter region and coding region. The insertion of sequences may also lead to the expression of fusion proteins. For example, a linkage of a regulatory region, e.g. a promoter region, and a coding region to be expressed, can exist in a vector-integrated form and can be inserted into a genome, e.g. by transfection, transduction, and/or transformation.

Herein, the term "expression" refers to the biosynthesis of a coding region product, preferably to the transcription and/or translation of a coding region within a nucleic acid in a cell. For example, in the case of a structural gene, expression may involve transcription of the structural gene into mRNA and - optionally - the subsequent translation of mRNA into one or more polypeptides. In other cases, expression may refer only to the transcription of a DNA sequence encoding and/or harbouring an RNA molecule.

In an embodiment of the first aspect, the nucleic acid comprises a) to d) in 5' to 3' direction. Additionally or alternatively, the nucleic acid comprises a) to d), wherein b), c) and d) are under control of a).

In an embodiment of the first aspect, the promoter region results in a moderate expression of b), c) and d), operably linked thereto. A promoter region that results in a moderate expression may be understood as a moderate promoter. An example of a moderate promoter is a human phosphoglycerate kinase (hPGK)promoter. A moderate expression can be advantageous as it can facilitate maintaining endogenous regulation mechanisms, including regulation mechanisms via proteasomal degradation. Thus, a moderate promoter can advantageously ensure that at least the signal of the first reporter protein domain reflects cellular Fe²⁺ levels via the Fe²⁺-sensitive domain comprised in the first polypeptide.

In an embodiment of the first aspect, the promoter region comprises or consists of an hPGK promoter. For example, the promoter region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 1. For example, the promoter region can comprise or consist of the sequence set forth in SEQ ID NO: 1. For example, the promoter region can comprise or consist of a sequence having the same biological function as the sequence set forth in SEQ ID NO: 1.

Herein, the term "sequence identity" when used in respect to the comparison of two or more nucleic acid or amino acid sequences means that the sequences share a certain degree of sequence similarity, the sequences being partially identical. Sequence variants may be defined by their sequence identity when compared to a parent sequence. Sequence identity is usually provided as "% sequence identity" or "% identity". To determine the percent-identity between two sequences in a first step a pairwise sequence alignment is generated between those two sequences, wherein the two sequences are aligned over their complete length (global alignment) or only partially (local alignment). For example, sequence similarity or identity can be determined by searching against databases such as FASTA, BLAST, etc., but hits should be retrieved and aligned pairwise to compare sequence identity, e.g. using default setting. For example, for amino acid sequences the alignment may be generated with a program implementing the Needleman and Wunsch algorithm (J. Mol. Biol. (1979) 48, p. 443-453), preferably by using the program "NEEDLE" (The European Molecular Biology Open Software Suite (EMBOSS)) with the programs default parameters (gapopen=10.0, gapextend=0.5 and matrix=EBLOSUM62). The preferred alignment for the purpose of this invention is that alignment, from which the highest sequence identity can be determined.

In an embodiment of the first aspect, the promoter region results in a strong expression of b), c) and d), operably linked thereto. A promoter region that results in a strong expression may be understood as a strong promoter. An example of a strong promoter is a cytomegalovirus (CMV) promoter. A strong expression can be advantageous as it can result in a higher amount of reporter protein domains compared to an expression under a moderate promoter region. Thus, a strong promoter can facilitate reporter protein domain signal detection. Accordingly, the promoter region can additionally or alternatively comprise a cytomegalovirus (CMV) promoter.

### Module 1 - Coding reqion(s), polypeptide(s), and reporter protein (domain(s))

Herein, the term "coding region" of a nucleic acid refers to the portion of the nucleic acid, which is transcribed and translated in a sequence-specific manner to produce a particular polypeptide when placed under the control of appropriate regulatory sequences. The coding region is said to encode such a polypeptide and thus, the amino acids found in the nascent polypeptide as a result of translation of an mRNA. In eukaryotes, the coding region is bounded on the 5'-side by the nucleotide triplet "ATG, which encodes the initiator methionine, and on the 3'-side by one of the three triplets, which specify a stop codon (i.e., TAA, TAG, TGA). In addition to containing introns, genomic forms of a gene may also include sequences located on both the 5'- and 3'-end of the sequences which are present on the RNA transcript. These sequences may be referred to as "flanking" sequences or regions (these flanking sequences are located 5' or 3' to the non-translated sequences present on the mRNA transcript). The 5'-flanking region may contain regulatory sequences such as promoter regions and enhancers which control or influence the transcription of the gene. The 3'-flanking region may contain sequences which direct the termination of transcription, post-transcriptional cleavage and polyadenylation.

Herein, the term "encodes" or "encoding" refers to the inherent property of specific sequences of nucleotides in a polynucleotide, such as a coding region, a gene, a cDNA, or an mRNA, to serve as templates for synthesis of other polymers and macromolecules in biological processes having either a defined sequence of nucleotides (i.e., rRNA, tRNA, mRNA, or miRNA) or a defined sequence of amino acids and the biological properties resulting therefrom. On a general level, a coding region encodes a polypeptide if transcription and translation of mRNA corresponding to that coding region produces the polypeptide in a cell or in another biological system. Both the coding strand, the nucleotide sequence of which is identical to the mRNA sequence, and the non-coding strand, used as the template for transcription of a coding region, can be referred to as encoding the polypeptide of that coding region.

Herein, the terms "polypeptide" and "protein" are used interchangeably to refer to a polymer or oligomer of consecutive amino acid residues. Thus, the term refers to a compound comprised of amino acid residues covalently linked by peptide bonds. A polypeptide must contain at least two amino acids, and no limitation is placed on the maximum number of amino acids that can comprise a protein's or peptide's sequence. Thus, a "polypeptide" comprises two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. A polypeptide includes, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

Herein, the term "reporter protein" refers to a polypeptide that is suitable for obtaining a read-out indicative for an amount, a change thereof or the like of a target under study. Preferably, the read-out is a signal and the intensity of the signal is indicative for an amount, a change thereof or the like of a target under study. Herein, the term "intensity of a signal" may encompass quantity and/or intensity depending on the nature of the signal. The signal can preferably be measured and/or visualized using standard techniques known to the person skilled in the art. Preferably, the signal can be analyzed spectrally. For example, a suitable signal can be fluorescence and an intensity of fluorescence may be indicative for an amount, a change thereof or the like of a target under study. A suitable reporter protein can be, e.g., a fluorescent protein, a luciferase, a tag, an antigen and the like or any combination thereof. Using a fluorescent protein as a reporter protein can be advantageous as fluorescence can be easily measured, and optionally monitored, for example using imaging systems, by applying microscopy techniques and/or by applying image analysis software. Fluorescence microscopy can offer the advantage of enabling live cell imaging, dynamic real-time monitoring and/or non-invasive analyses including fluorescence measurement. Herein, a reporter protein can be a "reporter protein domain" when comprised in a polypeptide. For example, a reporter protein can be a reporter protein domain within a polypeptide, wherein said polypeptide comprises a further domain such as a Fe²⁺-sensitive domain. For example, in case of a polypeptide consisting of a reporter protein domain, said polypeptide can be seen as a single domain polypeptide and thus, said polypeptide can also be understood as a reporter protein. Hence, the terms "reporter protein" and "reporter protein domain" are to be understood as being used interchangeably when comprised in a single domain polypeptide. Furthermore, when a sequence encoding a reporter protein domain is operably linked to a promoter region, the reporter protein domain can function as a mean for measuring promoter region activity. Hence, activity of a promoter region can be evaluated by, e.g., operably linking a sequence encoding a reporter protein domain and a promoter region to generate a reporter construct, introducing the reporter construct into the genome of a cell and detecting expression of the sequence encoding the reporter protein domain (e.g., detecting mRNA, protein, or the activity of a reporter protein). Various reporter proteins as well as various methods of integrating one or more thereof in a construct of choice are well known to those skilled in the art.

In an embodiment of the first aspect, the reporter protein domain is a fluorescent protein domain. For example, the first reporter protein domain is a first fluorescent protein domain. Additionally or alternatively, the second reporter protein domain is a second fluorescent protein domain. Preferably, the first reporter protein domain is a first fluorescent protein domain and the second reporter protein domain is a second fluorescent protein domain.

Accordingly, in an embodiment of the first aspect the nucleic acid comprises
a. A promoter region;
b. A first coding region encoding a first polypeptide,
   wherein the first polypeptide comprises a first fluorescent protein domain and a first Fe²⁺-sensitive domain;
c. A polypeptide separator region, preferably wherein the polypeptide separator region is a ribosome skipping region; and
d. A second coding region encoding a second polypeptide,
   wherein the second polypeptide comprises a second fluorescent protein domain, and
   wherein the first fluorescent protein domain and the second fluorescent protein domain are different.

The first fluorescent protein domain and the second fluorescent protein domain may be selected from the group consisting of mTagBFP2, mNeonGreen, and mCherry. For example, the first fluorescent protein domain can be mTagBFP2 or mNeonGreen. For example, the second fluorescent protein domain can be mCherry.

For example, the first fluorescent protein domain can be mTagBFP2. Accordingly, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 10. For example, the first coding region can comprise a sequence encoding the sequence set forth in SEQ ID NO: 10. For example, the first coding region can comprise a sequence encoding an amino acid sequence having the same biological function as the sequence set forth in SEQ ID NO: 10. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 11. For example, the first coding region can comprise the sequence set forth in SEQ ID NO: 11. For example, the first coding region can comprise a sequence having the same biological function as the sequence set forth in SEQ ID NO: 11.

For example, the second fluorescent protein domain can be mTagBFP2. Accordingly, the second coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 10. For example, the second coding region can comprise a sequence encoding the sequence set forth in SEQ ID NO: 10. For example, the second coding region can comprise a sequence encoding an amino acid sequence having the same biological function as the sequence set forth in SEQ ID NO: 10. For example, the second coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 11. For example, the second coding region can comprise the sequence set forth in SEQ ID NO: 11. For example, the second coding region can comprise a sequence having the same biological function as the sequence set forth in SEQ ID NO: 11.

For example, the first fluorescent protein domain can be mNeonGreen. Accordingly, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 12. For example, the first coding region can comprise a sequence encoding the sequence set forth in SEQ ID NO: 12. For example, the first coding region can comprise a sequence encoding an amino acid sequence having the same biological function as the sequence set forth in SEQ ID NO: 12. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 13. For example, the first coding region can comprise the sequence set forth in SEQ ID NO: 13. For example, the first coding region can comprise a sequence having the same biological function as the sequence set forth in SEQ ID NO: 13.

For example, the second fluorescent protein domain can be mNeonGreen. Accordingly, the second coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 12. For example, the second coding region can comprise a sequence encoding the sequence set forth in SEQ ID NO: 12. For example, the second coding region can comprise a sequence encoding an amino acid sequence having the same biological function as the sequence set forth in SEQ ID NO: 12. For example, the second coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 13. For example, the second coding region can comprise the sequence set forth in SEQ ID NO: 13. For example, the second coding region can comprise a sequence having the same biological function as the sequence set forth in SEQ ID NO: 13.

For example, the first fluorescent protein domain can be mCherry. Accordingly, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 14. For example, the first coding region can comprise a sequence encoding the sequence set forth in SEQ ID NO: 14. For example, the first coding region can comprise a sequence encoding an amino acid sequence having the same biological function as the sequence set forth in SEQ ID NO: 14. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 15. For example, the first coding region can comprise the sequence set forth in SEQ ID NO: 15. For example, the first coding region can comprise a sequence having the same biological function as the sequence set forth in SEQ ID NO: 15.

For example, the second fluorescent protein domain can be mCherry. Accordingly, the second coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 14. For example, the second coding region can comprise a sequence encoding the sequence set forth in SEQ ID NO: 14. For example, the second coding region can comprise a sequence encoding an amino acid sequence having the same biological function as the sequence set forth in SEQ ID NO: 14. For example, the second coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 15. For example, the second coding region can comprise the sequence set forth in SEQ ID NO: 15. For example, the second coding region can comprise a sequence having the same biological function as the sequence set forth in SEQ ID NO: 15.

### Module 1 - Fe²⁺-sensitive domain

Herein, the term "Fe²⁺-sensitive domain" refers to a domain within a polypeptide that is capable of interacting with cellular Fe²⁺. Said interaction can be physical and/or chemical. For example, the Fe²⁺-sensitive domain may comprise one or more amino acids that are capable of interacting, e.g. binding, to Fe²⁺. Interacting with Fe²⁺ may cause, for example, a conformational change and/or result in a stabilization of the Fe²⁺-sensitive domain. An example of an Fe²⁺-sensitive domain may be a degron sequence and/or a domain comprising a degron sequence. For example, a degron sequence may be comprised in a hemerythrin-like (Hr) domain of FBXL5. Another example of an Fe²⁺-sensitive domain may be a potential iron sulfur cluster (ISC) domain.

Accordingly, in an embodiment of the first aspect, the first Fe²⁺-sensitive domain comprises a degron sequence. For example, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 6. For example, the first coding region can comprise a sequence encoding the sequence set forth in SEQ ID NO: 6. For example, the first coding region can comprise a sequence encoding an amino acid sequence having the same biological function as the sequence set forth in SEQ ID NO: 6. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 7. For example, the first coding region can comprise the sequence set forth in SEQ ID NO: 7. For example, the first coding region can comprise a sequence having the same biological function as the sequence set forth in SEQ ID NO: 7.

For example, a degron sequence can be comprised in a Hr domain of FBXL5. In the absence of cellular Fe²⁺, the Fe²⁺-sensitive degron sequence is exposed to ubiquitination and degradation pathways and thus, the Hr domain can be degraded, along with any protein domain such as a reporter protein domain fused to it. When Fe²⁺ binds to the degron sequence, it is hypothesized that the binding results in a shielding of the degron sequence from ubiquitination. Thus, the Hr domain can be stabilized. Stabilization of the Hr domain (SEQ ID NO: 8) may result from three glutamic acids in positions 58, 61, and 130, as well as 3 histidines in positions 15, 57, and 126, respectively. It is hypothesized that an Fe²⁺ - Oxygen - Fe²⁺ coordination requires the histidine in position 80 in the Hr domain (SEQ ID NO: 8), which is located on the degron sequence. When Fe²⁺ binds to the degron sequence the Hr domain can be stabilized and polypeptide fused thereto (such as mNeonGreen in IronFIST, design 2). Thus, Fe²⁺ binding can prevent degradation, leading to an accumulation of the Hr domain. This is expected to result, e.g. in case of Module 1, in an increase in the signal of the reporter protein domain fused thereto, like a mNeonGreen fluorescent protein domain in case of the herein exemplarily investigated Module 1 - Design 2 construct.

Accordingly, in an embodiment of the first aspect, the first Fe²⁺-sensitive domain comprises or consists of a Hr domain of FBXL5. For example, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 8. For example, the first coding region can comprise a sequence encoding the sequence set forth in SEQ ID NO: 8. For example, the first coding region can comprise a sequence encoding an amino acid sequence having the same biological function as the sequence set forth in SEQ ID NO: 8. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 9. For example, the first coding region can comprise the sequence set forth in SEQ ID NO: 9. For example, the first coding region can comprise a sequence having the same biological function as the sequence set forth in SEQ ID NO: 9.

### Module 1 - Polypeptide separator region

Strategies for co-expressing two or more coding regions encompassed by the present invention can include use of multiple vectors (such as viral vectors and/or plasmids), use of multiple promoter regions within a vector, creation of a fusion protein with or without a proteolytic cleavage site, use of an internal ribosome entry site between coding regions, and/or use of ribosome skipping. Herein, it is preferred that the polypeptide separator region is a ribosome skipping region and/or a region encoding a proteolytic cleavage site. These two options are advantageous as in both cases expression of both the first and the second coding region are under control of the (same) promoter region and thus, comparable amounts of first and second polypeptides can be obtained by transcription and translation, while their subsequent fate may vary.

According to an embodiment of the first aspect, the polypeptide separator region is a ribosome skipping region. Additionally or alternatively, the polypeptide separator region may be a region encoding a proteolytic cleavage site. Proteolytic cleavage sites are known to the person skilled in the art and the same applies to nucleic acids encoding the same that can be used as a polypeptide separator region. Preferably, the polypeptide separator region is a ribosome skipping region.

Herein, the term "ribosome skipping region" refers to a sequence comprised in a nucleic acid that encodes a ribosome skipping peptide. Ribosome skipping offers the advantage of having multiple coding regions under control of a single promoter. Thus, ribosome skipping can advantageously ensure equal transcription (as only one mRNA is transcribed) as well as translation of comparable amounts of proteins encoded by the respective coding regions. Ribosome skipping can have the advantage that comparatively high protein levels can be expected compared to other strategies for co-expressing two or more coding regions. Ribosome skipping can be advantageous in that ribosome skipping peptides are small in size and thus, can bear a lower risk of interfering with the function of co-expressed coding regions compared to other co-expression strategies.

Preferably, ribosome skipping is followed by recommencement of translation; thus, two "cleaved" proteins can be expected, namely one protein fused to a fragment of the ribosome skipping peptide and the other protein fused to a further (and/or remaining) fragment of the ribosome skipping peptide (cf., e.g., Liu et al., Systematic comparison of 2A peptides for cloning multi-genes in a polycistronic vector, Sci Rep 7, 2193 (2017), https://doi.org/10.1038/s41598-017-02460-2). For example, in case of a polypeptide separator region being a ribosome skipping region encoding T2A, the first polypeptide may comprise the first reporter protein and the T2A amino acid sequence except the (final) proline, and the second polypeptide may comprise the (final) proline from the T2A amino acid sequence and the second reporter protein domain. Ribosome skipping followed by recommencement of translation can be advantageous for a genetically encoded Fe²⁺ biosensors, wherein a first fusion polypeptide can be used for measuring cellular Fe²⁺ and wherein a second polypeptide comprises a reporter protein domain that can be used as internal control or as another indicator for cellular Fe²⁺.

Accordingly, in an embodiment of the first aspect, the polypeptide separator region is a ribosome skipping region, wherein the ribosome skipping region encodes a peptide that causes ribosome skipping followed by recommencement of translation. For example, the ribosome skipping region can comprise a sequence encoding an amino acid sequence having more than 95%, more than 96%, more than 97%, more than 98%, more than 99%, more than 99.5%, more than 99.9% sequence identity with the sequence set forth in SEQ ID NO: 2. For example, the ribosome skipping region can comprise a sequence encoding the sequence set forth in SEQ ID NO: 2.

In an embodiment of the first aspect, the polypeptide separator region is a ribosome skipping region, wherein the ribosome skipping region encodes a peptide that causes ribosome skipping followed by recommencement of translation. For example, the ribosome skipping region can comprise a sequence encoding an amino acid sequence having more than 95%, more than 96%, more than 97%, more than 98%, more than 99%, more than 99.5%, more than 99.9% sequence identity with the sequence set forth in SEQ ID NO: 3. For example, the ribosome skipping region can comprise a sequence encoding the sequence set forth in SEQ ID NO: 3.

In an embodiment of the first aspect, the polypeptide separator region is a ribosome skipping region, wherein the ribosome skipping region is a 2A region. Thus, the polypeptide separator region is a 2A region. Herein, the term "2A region" refers to a nucleic acid region encoding a 2A peptide. 2A peptide are examples of peptides capable of causing ribosome skipping. Advantageously, 2A peptide are examples of peptides capable of causing ribosome skipping followed by recommencement of translation. Examples of 2A peptides are F2A (foot-and-mouth disease virus), E2A (equine rhinitis A virus), P2A (porcine teschovirus-1 2A), and T2A (thosea asigna virus 2A). Accordingly, the 2A region preferably encodes a 2A peptide selected from the group consisting of F2A (foot-and-mouth disease virus), E2A (equine rhinitis A virus), P2A (porcine teschovirus-1 2A), and T2A (thosea asigna virus 2A).

In a preferred embodiment of the first aspect, the polypeptide separator region is a ribosome skipping region, wherein the ribosome skipping region encodes T2A. Thus, the polypeptide separator region encodes T2A. For example, the ribosome skipping region can comprise a sequence encoding an amino acid sequence having more than 95%, more than 96%, more than 97%, more than 98%, more than 99%, more than 99.5%, more than 99.9% sequence identity with the sequence set forth in SEQ ID NO: 4. For example, the ribosome skipping region can comprise or consist of a sequence encoding the sequence set forth in SEQ ID NO: 4. For example, the ribosome skipping region can comprise or consist of a sequence encoding an amino acid sequence having the same biological function as the sequence set forth in SEQ ID NO: 4. For example, the ribosome skipping region can comprise a sequence having more than 95%, more than 96%, more than 97%, more than 98%, more than 99%, more than 99.5%, more than 99.9% sequence identity with the sequence set forth in SEQ ID NO: 5. For example, the ribosome skipping region can comprise or consist of the sequence set forth in SEQ ID NO: 5. For example, the ribosome skipping region can comprise or consist of a sequence having the same biological function as the sequence set forth in SEQ ID NO: 5.

### Module 1 - Options for the second coding region

Regarding Module 1, a reporter protein domain is comprised in a polypeptide encoded in a coding region of the nucleic acid of the first aspect. The first polypeptide comprises a first reporter protein domain and a first Fe²⁺-sensitive domain. Thus, said first polypeptide may be understood as a first fusion polypeptide comprising two domains, namely a first reporter protein domain and a first Fe²⁺-sensitive domain. Within the first polypeptide said two domains may be fused together directly or indirectly (e.g. via a linker, e.g. via a linker consisting of 3 repeats of Gly and/or being encoded, e.g., by the sequence GGCGGCGGC). The second polypeptide comprises a second reporter protein domain. For example, the second polypeptide can consist of the second reporter protein domain. Alternatively, the second polypeptide can comprise a second reporter protein domain. Additionally or alternatively, the second polypeptide can comprise a second reporter protein domain and a further domain. In this case, the second polypeptide can be understood as a second fusion polypeptide comprising two domains, namely a second reporter protein domain and a further domain such as, e.g., a second Fe²⁺-sensitive domain. Within the second polypeptide said two domains can be fused together directly or indirectly (e.g. via a linker, e.g. via a linker consisting of 3 repeats of Gly and/or being encoded, e.g., by the sequence GGCGGCGGC).

In an embodiment of the first aspect, the second polypeptide is Fe²⁺-insensitive. Additionally or alternatively, the second polypeptide does not comprise an Fe²⁺-sensitive domain. Additionally or alternatively, the second polypeptide consists of the second reporter protein domain. Any of these three options and combinations thereof can be advantageous as a signal obtained from the second reporter protein domain can be used as an internal control when assessing cellular Fe²⁺ based on a signal obtained from the first reporter protein domain. In case the first and the second reporter proteins are fluorescent proteins, intensity of fluorescence of the second fluorescent protein domain can be used as internal control when assessing cellular Fe²⁺ based on an intensity of fluorescence of the first fluorescent protein domain.

Herein, the term "fluorescence of', e.g., a fluorescent protein domain, refers to fluorescence emitted by, e.g., said fluorescent protein domain, preferably upon excitation. Excitation can be done by providing an excitation spectrum of wavelengths or a specific excitation wavelength. Thus, energy is added to, e.g., said fluorescent protein domain, causing it to emit a wavelength of light, e.g. an emission spectrum or a specific emission wavelength, that can be measured and the intensity of which can be analyzed. For example, in case of a reporter protein domain comprising or being mTagBFP2 (may also be referred to as BFP), a cell comprising said protein domain can be excited at (about) 405 nm and emitted fluorescence measured at (about) 450 nm. For example, in case of a reporter protein domain comprising or being mNeonGreen, a cell comprising said protein domain can be excited at (about) 470-490 nm and emitted fluorescence measured at (about) 500-520 nm. For example, in case of a reporter protein domain comprising or being mCherry, a cell comprising said protein domain can be excited at (about) 61 nm and/or at about 540-590 nm and emitted fluorescence measured at (about) 565-650 nm.

Herein, the term "about", when used in conjunction with a numerical range, modifies said range by extending the boundaries above and below the numerical values set forth. In general, the term "about" is used herein to modify a numerical value above and below the stated value by a variance of 20 percent, preferably 10 percent, more preferably 5 percent up or down (higher or lower).

In an embodiment of the first aspect, the second polypeptide is Fe²⁺-sensitive. Additionally or alternatively, the second polypeptide comprises a second Fe²⁺-sensitive domain. For example, the second Fe²⁺-sensitive domain can comprise or consist of a (potential) iron sulfur cluster (ISC) binding domain. For example, such a domain can be comprised in NCOA. NCOA is assumed to be responsible for targeting ferritin to lysosomes (ferritinophagy). Ferritinophagy may be a response to iron deficiency to increase LIP and eventually ISC. Thus, NCOA may be stable in the absence of cellular Fe²⁺.

Accordingly, in an embodiment of the first aspect, the second polypeptide comprises a second Fe²⁺-sensitive domain, wherein the second Fe²⁺-sensitive domain comprises or consists of NCOA. For example, the second Fe²⁺-sensitive domain can comprise a sequence encoding an amino acid sequence having more than 95%, more than 96%, more than 97%, more than 98%, more than 99%, more than 99.5%, more than 99.9% sequence identity with the sequence set forth in SEQ ID NO: 16. For example, the second Fe²⁺-sensitive domain can comprise or consist of a sequence encoding the sequence set forth in SEQ ID NO: 16. For example, the second Fe²⁺-sensitive domain can comprise or consist of a sequence encoding an amino acid sequence having the same biological function as the sequence set forth in SEQ ID NO: 16. For example, the second Fe²⁺-sensitive domain can comprise a sequence having more than 95%, more than 96%, more than 97%, more than 98%, more than 99%, more than 99.5%, more than 99.9% sequence identity with the sequence set forth in SEQ ID NO: 17. For example, the second Fe²⁺-sensitive domain can comprise or consist of the sequence set forth in SEQ ID NO: 17. For example, the second Fe²⁺-sensitive domain can comprise or consist of a sequence having the same biological function as the sequence set forth in SEQ ID NO: 17.

It is readily understood by the skilled person that the first Fe²⁺-sensitive domain may be Fe²⁺-sensitive domain comprised or consisting of NCOA and optionally a second Fe²⁺-sensitive domain may comprise a degron sequence and/or comprise or consist of an Hr domain of FBXL5. As regards NCOA, degron sequence and an Hr domain of FBXL5, and/or its respective features, the same applies as has been described herein in connection with the nucleic acid according to the first aspect.

### Module 1 - Expression cassette

In an embodiment of the first aspect, the nucleic acid is an expression cassette.

Herein, the term "expression cassette" refers to a nucleic acid that comprises a promoter region, a coding region and optionally one or more regulatory sequences, wherein the coding region is to be expressed. For example, when the expression cassette is part of a DNA plasmid, the coding region is to be expressed by the cell into which the plasmid is introduced. An expression cassette can be a recombinant expression cassette. Herein, the term "recombinant" with respect to a nucleic acid refers to a nucleic acid produced by a recombinant DNA technique. Recombinant nucleic acids may also comprise sequences, which as such does not exist in nature but are modified, changed, mutated or otherwise manipulated by man. Preferably, a "recombinant nucleic acid" is a non-naturally occurring nucleic acid that differs in sequence from a naturally occurring nucleic acid by at least one nucleic acid. A "recombinant nucleic acid" may also comprise a "recombinant construct" which comprises, preferably operably linked, a nucleic acid not naturally occurring in that order. Preferred methods for producing said recombinant nucleic acid may comprise cloning techniques, directed or non-directed mutagenesis, synthesis or recombination techniques. Concerning an expression cassette, a recombinant expression cassette may be understood as an expression cassette that is not naturally occurring but has been engineered and may comprise different elements that do not occur together in nature or additional synthetic sequences. For example, a recombinant expression cassette may further comprise an enhancer region, a polyA sequence encoding region, a linker encoding region and/or further functional regions. The recombinant expression cassette may also comprise recognition or binding motifs, such as miRNA binding sites. Herein, an expression cassette can be a naturally occurring or a recombinant expression cassette.

### Module 1 - Exemplarily designs

In an embodiment of the first aspect, the first reporter protein domain is a first fluorescent protein domain, the second reporter protein domain is a second fluorescent protein domain, and the nucleic acid comprises
a. A promoter region comprising or consisting of a hPGK promoter;
b. A first coding region encoding a first polypeptide,
   wherein the first polypeptide comprises a first fluorescent protein domain and a first Fe²⁺-sensitive domain wherein first Fe²⁺-sensitive domain comprises or consists of a Hr domain of FBXL5;
c. A polypeptide separator region encoding T2A; and
d. A second coding region encoding a second polypeptide,
   wherein the second polypeptide comprises a second fluorescent protein domain, and
   wherein the first fluorescent protein domain and the second fluorescent protein domain are different.

Module 1 has been exemplified by assessing two constructs, herein referred to as Design 1 and Design 2.

Design 1 may be understood as a proof-of-principle construct. In particular, Design 1 comprises an Fe²⁺-sensitive degron sequence comprised in a Hr domain of FBXL5 fused to mTagBFP2 as first fluorescent protein domain, wherein expression of the first coding region is under control of hPGK as a moderate promoter. The plasmid sequence investigated in case of Design 1 is set forth in SEQ ID NO: 18.

Design 2 may be understood as a construct for a pseudo-ratiometric approach. Design 2 comprises an Fe²⁺-sensitive degron sequence comprised in a Hr domain of FBXL5 fused to mNeonGreen as first fluorescent protein domain and mCherry as second fluorescent protein (thus serving, e.g., as control and/or internal reference), wherein the first and the second coding regions are separated by a T2A ribosome skipping region and wherein expression of the two coding regions is under control of hPGK as a moderate promoter. The respective nucleic acid comprised the sequences set forth in SEQ ID NO: 1 and SEQ ID NO: 30. The plasmid sequence exemplarily investigated in case of Design 2 is set forth in SEQ ID NO: 19.

Accordingly, in an embodiment of the first aspect, the nucleic acid comprises the sequence set forth in SEQ ID NO: 30.

### Module 1 - Vectors and cell

In a second aspect, the present invention relates to a vector comprising the nucleic acid according to the first aspect. As regards the nucleic acid comprised in the vector and/or its respective features, the same applies as has been described herein in connection with the nucleic acid according to the first aspect.

Herein, the term "vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. For example, a vector may be a genomic integrated vector, which can become integrated into the chromosomal DNA of the host cell. Another type of vector may be an episomal vector, i.e., a nucleic acid molecule capable of extra-chromosomal replication. As a further example, a vector comprising a nucleic acid of the invention may also be an expression vector comprising sequences recognized by any RNA polymerase, including mitochondrial RNA polymerase, RNA pol I, RNA pol II, and RNA pol III. The term "vector" should also be construed to include non-plasmid and non-viral compounds which facilitate transfer of nucleic acid into cells, such as polylysine compounds, liposomes, and the like.

In an embodiment of the second aspect, the vector is a viral vector. For example, the vector can be a lentivirus vector, an adenovirus, an adeno-associated virus (AAV), and/or a baculovirus.

In an embodiment of the second aspect, the vector is a plasmid. For example, the plasmid can be a VB240227-1627une plasmid or a PcDNA3.1- plasmid.

In an embodiment of the second aspect, the vector comprises or consists of the sequence set forth in SEQ ID NO: 19.

In a third aspect, the present invention relates to a cell comprising the nucleic acid according to the first aspect and/or the vector according to the second aspect. As regards the nucleic acid comprised in the cell and/or its respective features, the same applies as has been described herein above in connection with the nucleic acid according to the first aspect.

In an embodiment of the third aspect, the first polypeptide and the second polypeptide are stably or transiently expressed in the cell.

In an embodiment of the third aspect, the cell is a HeLa cell, a macrophage, an enterocyte, a hepatocyte, a cancer cell, an immune cell and/or a neuron. For example, the cel can be a HeLa cell. For example, the cell can be a cell exhibiting distinctive iron homeostasis characteristics, such as macrophages and enterocytes or hepatocytes. Accordingly, the cell can be for example a macrophage, an enterocyte or a hepatocyte. For example, the cell can be a cell with potential sensitivity to iron depletion. For example, the cell can be a cell with potential sensitivity to iron overload, such as various cancer cells, immune cells and neurons. Accordingly, the cell can be for example a cancer cell, an immune cell and/or a neuron.

Herein, a condition of iron depletion may also be referred to as a low (intracellular) Fe²⁺ condition. Furthermore, a condition of high intracellular Fe²⁺ may also be referred to herein as a high (cellular) Fe²⁺ condition. It is hypothesized that in a high Fe²⁺ condition, the GSH pool (that normally complexes with Fe²⁺) becomes saturated and thus, cellular Fe²⁺ can bind to an Fe²⁺-sensitive domain such as a degron sequence comprised in a Hr domain before Fe²⁺ is oxidized to Fe³⁺ (Fenton reaction, generating cytotoxic ROS). When Fe²⁺ is bound, e.g., to the degron sequence comprised in the Hr domain, ferritin translation can be induced. Further, it is hypothesized that in a low Fe²⁺ condition, substantially no free Fe²⁺ is available to bind to an Fe²⁺-sensitive domain such as a degron sequence comprised in a Hr domain. However, the Hr domain may only be stable when Fe²⁺ is bound by/to the Fe²⁺-sensitive domain being a degron sequence within the Hr domain. Any cytosolic Fe²⁺ may remain complexed with GSH or oxidized and optionally stored within ferritin.

### Module 1 - Methods

In the following, methods using, *inter alia,* the cell according to the third aspect are described. As regards the cell and its respective features, the same applies as has been described herein above in connection with the nucleic acid according to the first aspect, the vector according to the second aspect and/or the cell according to the third aspect.

Herein, the terms "measurement", "to measure" and variations thereof encompass i) determining an amount, level, intensity and/or change thereof and/or ii) quantifying the same. Means and methods for measuring a signal of a reporter protein domain are well known to the person skilled in the art and encompass known standard techniques depending on the type or reporter protein domain being used.

In a fourth aspect, the present invention relates to an *in vitro* method for measuring cellular Fe²⁺, comprising:
a. Providing the cell according to the third aspect, and
b. Measuring a signal of the first reporter protein domain and a signal of the second reporter protein domain,
wherein at least intensity of the signal the first reporter protein domain is indicative of an amount of cellular Fe²⁺.

In an embodiment of the fourth aspect, the first reporter protein domain is a first fluorescent protein domain, the second reporter protein domain is a second fluorescent protein domain, the signal is fluorescence and the method comprises:
a. Providing the cell according to the third aspect and exciting the cell,
b. Measuring fluorescence of the first fluorescent protein domain and fluorescence of the second fluorescent protein domain,
   wherein at least intensity of fluorescence of the first fluorescent protein domain is indicative of an amount of cellular Fe²⁺.

In an embodiment of the fourth aspect, the method comprises prior or after, preferably after, b): c. Amending an amount of extracellular and/or cellular Fe²⁺.

For example, a high Fe²⁺ condition can be induced by adding Fe²⁺ salts to a cell, optionally in combination with Vitamin C, which facilitates Fe²⁺ uptake via plasma membrane ion channels, particularly DMT1. Additionally or alternatively, iron carbohydrate nanoparticles can be used to increase cellular iron content. Also other experimental methods can be suitable to elevate cytosolic Fe²⁺ as known by the person skilled in the art.

For example, a low Fe²⁺ condition can be induced by adding an iron chelator such as deferoxamine (DFO) and/or ELT. DFO may be advantageous to bind free extracellular iron, e.g. in buffers. ELT may be advantageous to bind intracellular iron.

In an embodiment of the fourth aspect, the method comprises prior or after, preferably after, b):
c. Amending an amount of extracellular and/or cellular Fe²⁺,
wherein the amount of Fe²⁺ is amended by adding to the cell a composition comprising Fe²⁺. For example, the composition can comprise or consist of FeSO₄. For example, the composition can be FeSO₄ Vitamin C.

In an embodiment of the fourth aspect, the method comprises prior or after, preferably after, b):
c. Amending an amount of extracellular and/or cellular Fe²⁺,
wherein the amount of Fe²⁺ is amended by adding to the cell a Fe²⁺ chelator, preferably a cell membrane permeable Fe²⁺ chelator. For example, the Fe²⁺ chelator can be eltrombopag (ELT).

In an embodiment of the fourth aspect, the method comprises prior or after, preferably after, b):
c. Amending an amount of extracellular and/or cellular Fe²⁺,
wherein the amount of Fe²⁺ is amended by adding to the cell a compound affecting or being suspected of affecting cellular Fe²⁺. For example, the compound is an active ingredient, a drug candidate and/or a drug.

In an embodiment of the fourth aspect, the method comprises prior or after, preferably after, b)
c. Amending an amount of extracellular and/or cellular Fe²⁺,
and after step c) another step b) referred to as step d).

Preferably, the method further comprises
e. Comparing measurements obtained from step b) with measurements obtained from step d).

In an embodiment of the fourth aspect, the method comprises
e. Comparing measurements obtained from step b) with measurements obtained from a control.

In a fifth aspect, the present invention relates to an *in vitro* screening method for a compound that affects cellular Fe²⁺, the method comprising the steps of:
a. Providing the cell according to the third aspect,
b. Optionally measuring a signal of the first reporter protein domain and a signal of the second reporter protein domain,
c. Adding a compound affecting or being suspected of affecting cellular Fe²⁺,
d. Measuring a signal of the first reporter protein domain and a signal of the second reporter protein domain, and
e. Comparing measurements obtained from step d) with measurements obtained from step b) and/or a control,
   wherein at least intensity of the signal the first reporter protein domain is indicative of an amount of cellular Fe²⁺, and
   wherein a significant change in at least the intensity of the signal of the first reporter protein domain is indicative of the compound affecting cellular Fe²⁺.

Herein, a "significant change" of a signal intensity encompasses a significant increase and a significant decrease of a signal intensity. Herein, a change is considered significant if the change is larger than the margin of error inherent in the measurement technique. For example, a change in a signal intensity can refer to an increase or decrease by about 2-fold or greater of a control signal intensity. In case the signal is fluorescence and the intensity of the signal is an intensity of a fluorescence, a significant change in fluorescence is preferably defined as
**i)** a signal-to-noise ratio (SNR) of ≥ 3:1,
**ii)** a fold change of about ≥ 2 (-fold),
**iii)** a change of about ≥ 30% difference from a control and/or baseline fluorescence,
iv) a statistical significance with a p-value < 0.05, and/or
v) a statistical significance with a p-value < 0.05 in combination with a change of at least about 10%, preferably of at least about 20%.

In case of fluorescence-activated cell analysis (FACS), e.g., a significant change can be defined as i) a ≥ 10%, preferably a ≥ 20%, shift in fluorescence intensity and/or ii) an increase in the percentage of positive cells, preferably of more than 5%.

In an embodiment of the fifth aspect, the first reporter protein domain is a first fluorescent protein domain, the second reporter protein domain is a second fluorescent protein domain, the signal is fluorescence and the method comprises
a. Providing the cell according to the third aspect and exciting the cell,
b. Optionally measuring fluorescence of the first fluorescent protein domain and fluorescence of the second fluorescent protein domain,
c. Adding a compound affecting or being suspected of affecting cellular Fe²⁺,
d. Measuring fluorescence of the first fluorescent protein domain and fluorescence of the second fluorescent protein domain, and
e. Comparing measurements obtained from step d) with measurements obtained from step b) and/or a control,
   wherein at least an intensity of fluorescence of the first fluorescent protein domain is indicative for an amount of cellular Fe²⁺, and
   wherein a significant change in at least the intensity of fluorescence of the first fluorescent protein domain is indicative of the compound affecting cellular Fe²⁺.

In a sixth aspect, the present invention relates to an *in vitro* method for identifying a dosage and/or formulation of a compound that affects cellular Fe²⁺, the method comprising the steps of
a. Providing the cell according to the third aspect,
b. Optionally measuring a signal of the first reporter protein domain and a signal of the second reporter protein domain,
c. Adding a compound affecting or being suspected of affecting cellular Fe²⁺ in in a dosage and/or formulation,
d. Measuring a signal of the first reporter protein domain and a signal of the second reporter protein domain, and
e. Comparing measurements obtained from step d) with measurements obtained from step b) and/or a control,
   wherein at least intensity of the signal the first reporter protein domain is indicative of an amount of cellular Fe²⁺, and
wherein a significant change in at least the intensity of the signal of the first reporter protein domain is indicative of the dosage and/or formulation affecting cellular Fe²⁺.

In an embodiment of the sixth aspect, the first reporter protein domain is a first fluorescent protein domain, the second reporter protein domain is a second fluorescent protein domain, the signal is fluorescence and the method comprises
a. Providing the cell according to the third aspect and exciting the cell,
b. Optionally measuring fluorescence of the first fluorescent protein domain and fluorescence of the second fluorescent protein domain,
c. Adding a compound affecting or being suspected of affecting cellular Fe²⁺ in in a dosage and/or formulation,
d. Measuring fluorescence of the first fluorescent protein domain and fluorescence of the second fluorescent protein domain, and
e. Comparing measurements obtained from step d) with measurements obtained from step b) and/or a control,

wherein at least an intensity of fluorescence of the first fluorescent protein domain is indicative for an amount of cellular Fe²⁺, and
wherein a significant change in at least the intensity of fluorescence of the first fluorescent protein domain is indicative of the dosage and/or formulation affecting cellular Fe²⁺.

In an embodiment of the fourth, fifth and/or sixth aspect, the method further comprises prior step a) the step of contacting the cell according to the third aspect with the vector according to the second aspect.

In an embodiment of the fourth, fifth and/or sixth aspect, binding of cellular Fe²⁺ to the first Fe²⁺-sensitive domain results in degradation of the first reporter protein domain and/or of the first polypeptide. For example, the first reporter protein domain and/or the first polypeptide may be degraded by a proteasome. If the first protein domain is a first fluorescent protein domain, binding of cellular Fe²⁺ to the first Fe²⁺-sensitive domain can result in degradation of the first fluorescent protein domain and/or of the first polypeptide, preferably, the first fluorescent protein domain and/or the first polypeptide may be degraded by a proteasome.

In an embodiment of the fourth, fifth and/or sixth aspect, binding of cellular Fe²⁺ to the first Fe²⁺-sensitive domain results in stabilization of the first reporter protein domain and/or of the first polypeptide. For example, the first reporter protein domain and/or the first polypeptide can be stabilized by binding of cellular Fe²⁺ to the first Fe²⁺-sensitive domain. For example, stability of the first reporter protein domain and/or of the first polypeptide can be increased by binding of cellular Fe²⁺ to the first Fe²⁺-sensitive domain compared to an absence of cellular Fe²⁺ bound to the first Fe²⁺-sensitive domain.

If the first reporter protein domain is a first fluorescent protein domain, binding of cellular Fe²⁺ to the first Fe²⁺-sensitive domain results in stabilization of the first fluorescent protein domain and/or of the first polypeptide. For example, the first fluorescent protein domain and/or the first polypeptide can be stabilized by binding of cellular Fe²⁺ to the first Fe²⁺-sensitive domain. For example, stability of the first fluorescent protein domain and/or of the first polypeptide can be increased by binding of cellular Fe²⁺ to the first Fe²⁺-sensitive domain compared to an absence of cellular Fe²⁺ bound to the first Fe²⁺-sensitive domain.

In an embodiment of the fourth, fifth and/or sixth aspect, the second polypeptide is Fe²⁺-insensitive. Additionally or alternatively, the second reporter protein domain, preferably the second fluorescent protein domain, can be an internal control. For example, the method can be a pseudo-ratiometric method.

In an embodiment of the fourth, fifth and/or sixth aspect, the second polypeptide is Fe²⁺-sensitive. Additionally or alternatively, the second polypeptide can comprise a second Fe²⁺-sensitive domain. Additionally or alternatively, an intensity of the signal of the second reporter protein domain can be indicative of an amount of cellular Fe²⁺. For example, the method can be a ratiometric method.

For example, binding of cellular Fe²⁺ to the second Fe²⁺-sensitive domain can result in degradation of the second reporter protein domain and/or of the second polypeptide. For example, the second reporter protein domain and/or the second polypeptide may be degraded by a proteasome. As another example, binding of cellular Fe²⁺ to the second Fe²⁺-sensitive domain can result in stabilization of the second reporter protein domain and/or of the second polypeptide. For example, the second reporter protein domain and/or the second polypeptide can be stabilized by binding of cellular Fe²⁺ to the second Fe²⁺-sensitive domain. For example, stability of the second reporter protein domain and/or of the second polypeptide can be increased by binding of cellular Fe²⁺ to the second Fe²⁺-sensitive domain compared to an absence of cellular Fe²⁺ bound to the second Fe²⁺-sensitive domain. In a preferred embodiment, of the fourth, fifth and/or sixth aspect, the first reporter protein domain and/or the first polypeptide is stabilized by binding of cellular Fe²⁺ to the first Fe²⁺-sensitive domain and the second reporter protein domain and/or the second polypeptide is degraded by binding of cellular Fe²⁺ to the second Fe²⁺-sensitive domain. Alternatively, the first reporter protein domain and/or the first polypeptide is degraded by binding of cellular Fe²⁺ to the first Fe²⁺-sensitive domain and the second reporter protein domain and/or the second polypeptide is stabilized by binding of cellular Fe²⁺ to the second Fe²⁺-sensitive domain.

Preferably, the first reporter protein domain is a first fluorescent protein domain, the second reporter protein domain is a second fluorescent protein domain and the signal is fluorescence. Thus, an intensity of fluorescence of the second fluorescent protein domain can be indicative of an amount of cellular Fe²⁺. For example, binding of cellular Fe²⁺ to the second Fe²⁺-sensitive domain can result in degradation of the second fluorescent protein domain and/or of the second polypeptide. For example, the second fluorescent protein domain and/or the second polypeptide may be degraded by a proteasome. As another example, binding of cellular Fe²⁺ to the second Fe²⁺-sensitive domain can result in stabilization of the second fluorescent protein domain and/or of the second polypeptide. For example, the second fluorescent protein domain and/or the second polypeptide can be stabilized by binding of cellular Fe²⁺ to the second Fe²⁺-sensitive domain. For example, stability of the second fluorescent protein domain and/or of the second polypeptide can be increased by binding of cellular Fe²⁺ to the second Fe²⁺-sensitive domain compared to an absence of cellular Fe²⁺ bound to the second Fe²⁺-sensitive domain. In a preferred embodiment, of the fourth, fifth and/or sixth aspect, the first fluorescent protein domain and/or the first polypeptide is stabilized by binding of cellular Fe²⁺ to the first Fe²⁺-sensitive domain and the second fluorescent protein domain and/or the second polypeptide is degraded by binding of cellular Fe²⁺ to the second Fe²⁺-sensitive domain. Alternatively, the first fluorescent protein domain and/or the first polypeptide is degraded by binding of cellular Fe²⁺ to the first Fe²⁺-sensitive domain and the second fluorescent protein domain and/or the second polypeptide is stabilized by binding of cellular Fe²⁺ to the second Fe²⁺-sensitive domain.

In an embodiment of the fourth, fifth and/or sixth aspect, the first reporter protein domain is a first fluorescent protein domain, the second reporter protein domain is a second fluorescent protein domain, the signal is fluorescence, and the cell is excited at 405 nm, 470-490 nm, 561 nm, and/or 540-590 nm. Additionally or alternatively, fluorescence is measured at 450 nm, 500-520 nm, and/or 565-650 nm. Preferably, fluorescence of the first fluorescent protein domain is excited at 405 nm and/or 470-490 nm and measured at 450 nm and/or 500-520 nm. For example, fluorescence of the second fluorescent protein domain can be excited at 561 nm and/or 540-590 nm and measured at 565-650 nm. For example, fluorescence of the first fluorescent protein domain and fluorescence of the second fluorescent protein domain can be measured by dynamic time-lapse imaging. For example, fluorescence of the first fluorescent protein domain and fluorescence of the second fluorescent protein domain can be measured using a high-resolution microscopy. For example, fluorescence of the first fluorescent protein domain and fluorescence of the second fluorescent protein domain can be measured using a confocal technique.

In an embodiment of the fourth, fifth and/or sixth aspect, step b) is performed repeatedly and/or continuously. This can be advantageous for monitoring cellular iron and changes thereof over time.

### Module 1 - Uses

In the following, uses of the nucleic acid according to the first aspect, the vector according to the second aspect and/or the cell according to the third aspect are described. As regards the nucleic acid, the vector, the cell, and their respective features, the same applies as has been described herein above in connection with the nucleic acid according to the first aspect, the vector according to the second aspect and/or the cell according to the third aspect.

In a seventh aspect, the present invention relates to the use of the nucleic acid according to the first aspect to obtain the vector according to the second aspect.

In an eight aspect, the present invention relates to the use of the vector according to the second aspect to express the nucleic acid according to the first aspect.

In a ninth aspect, the present invention relates to the use of the vector according to the second aspect to obtain the cell according to the third aspect.

In a tenth aspect, the present invention relates to the use of the cell according to the third aspect for *in vitro* measuring and/or monitoring ferrous iron (Fe²⁺) in a cell, preferably according to the method according to the fourth aspect.

In an eleventh aspect, the present invention relates to the use of the cell according to the third aspect for *in vitro* screening for a compound that affects cellular Fe²⁺, preferably according to the method according to the fifth aspect.

In a twelfth aspect, the present invention relates to the use of the cell according to the third aspect *in vitro* identifying a dosage and/or formulation of a compound that affects cellular Fe²⁺, preferably according to the method according to the sixth aspect.

### Module 2

Module 2 has been developed to measure, and optionally monitor, changes in cellular Fe²⁺ and/or iron sulfur cluster (ISC; also: Fe/S) amounts and/or changes thereof. Module 2 represents a genetically encoded cellular Fe²⁺ and/or cellular ISC biosensor. Module 2 provides two reporter protein domain signals, wherein one signal can be indicative for elevated cellular Fe²⁺ presence, and wherein the other signal can be indicative for reduced cellular Fe²⁺ presence. Module 2 can indicate changes in cellular iron status by opposing expression of two different reporter protein domains. Module 2 can offer a ratiometric approach based on a ratio of obtained reporter protein domain signals. Thus, Module 2 can reflect changes in cellular iron status with improved comparability as it allows comparison between signal ratios obtained from different cells, cell types and/or conditions. Module 2 can enable dynamic real-time monitoring of cellular iron status. Module2 can be suitable for long-term studies and live cell imaging *in vitro.* Module 2 can be advantageous for screening approaches of iron-based therapeutics such as nanoparticle-based approaches

In a thirteenth aspect, the present invention relates to a nucleic acid, comprising in 5' to 3' direction:
a. A first promoter region,
b. A first coding region encoding
   i. an iron responsible element (IRE), and
   ii. a first reporter protein domain,
c. A second promoter region comprising an Fe²⁺ and/or ISC sensitive region,
d. A second coding region encoding
   i. a second reporter protein domain, wherein the first reporter protein domain and the second reporter protein domain are different, and
   ii. an IRE.
As regards the first reporter protein domain, the second reporter protein domain, the first promoter region, and/or its respective features, the same applies as has been described herein above in connection with the nucleic acid according to the first aspect.

In an embodiment of the thirteenth aspect, the nucleic acid is an expression cassette.

In an embodiment of the thirteenth aspect, b) is under control of a). Additionally or alternatively, d) is under control of c).

### Module 2 - First promoter region

In an embodiment of the thirteenth aspect, the first promoter region results in a moderate expression of the first coding region operably linked thereto. Additionally or alternatively, the first promoter region comprises an hPGK. As regards a moderate promoter and hPGK, and/or its respective features, the same applies as has been described herein above in connection with the nucleic acid according to the first aspect. For example, the first promoter region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 1. For example, the first promoter region can comprise or consist of the sequence set forth in SEQ ID NO: 1. For example, the first promoter region can comprise or consist of a sequence having the same biological function as the sequence set forth in SEQ ID NO: 1.

### Module 2 - Second promoter region

In an embodiment of the thirteenth aspect, the second promoter region results in a moderate expression of the second coding region operably linked thereto.

In an embodiment of the thirteenth aspect, in c) the Fe²⁺ and/or ISC sensitive region is a hypoxia-responsive element (HRE). This can be advantageous as HRE can result in sensitivity to low iron levels.

For example, the HRE can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 20. For example, the HRE can comprise or consist of the sequence set forth in SEQ ID NO: 20. For example, the HRE can comprise or consist of a sequence having the same biological function as the sequence set forth in SEQ ID NO: 20.

Preferably, the second promoter region comprises or consists of five, six, seven, eight, nine or ten HRE. For example, the second promoter region can comprise or consist of eight HRE. For example, the second promoter region can comprise or consist of at least a part of a TfR promoter region, preferably of at least a part of the TfR promoter region comprising HRE. For example, the second promoter region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 21. For example, the second promoter region can comprise or consist of the sequence set forth in SEQ ID NO: 21. For example, the second promoter region can comprise or consist of a sequence having the same biological function as the sequence set forth in SEQ ID NO: 21.

### Module 2 - IREs

In an embodiment of the thirteenth aspect, the IRE in b) i) comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 22. For example, the IRE in b) i) can comprise or consist of the sequence set forth in SEQ ID NO: 22. For example, the IRE in b) i) can comprise or consist of a sequence having the same biological function as the sequence set forth in SEQ ID NO: 22.

In an embodiment of the thirteenth aspect, the IRE in d) ii) comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 31. For example, the IRE in d) ii) can comprise or consist of the sequence set forth in SEQ ID NO: 31. For example, the IRE in d) ii) can comprise or consist of a sequence having the same biological function as the sequence set forth in SEQ ID NO: 31.

In an embodiment of the thirteenth aspect, the IRE in d) ii) can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 32. For example, the IRE in d) ii) can comprise or consist of the sequence set forth in SEQ ID NO: 32. For example, the IRE in d) ii) can comprise or consist of a sequence having the same biological function as the sequence set forth in SEQ ID NO: 32.

Preferably, the IRE in b) i) comprises or consists of the sequence set forth in SEQ ID NO: 22 and the IRE in d) ii) comprises the sequence set forth in SEQ ID NO: 31 and/or the sequence set forth in SEQ ID NO: 32. For example, the IRE in b) i) comprises or consists of the sequence set forth in SEQ ID NO: 22 and the IRE in d) ii) comprises the sequence set forth in SEQ ID NO: 31 and the sequence set forth in SEQ ID NO: 32. As regards the IREs in b) i) and d) ii) the same applies as stated herein above in more detail. Furthermore, SEQ ID NO: 31 and 32 may be comprised in a sequence encoding a Transferrin Receptor (TfR) 3'UTR, for example in SEQ ID NO: 23.

### Module 2 - Reporter protein domains

Preferably, the reporter protein domain is a fluorescent protein domain. Accordingly, in an embodiment of the thirteenth aspect, the first reporter protein domain is a first fluorescent protein domain and the second reporter protein domain is a second fluorescent protein domain, and the nucleic acid, comprises in 5' to 3' direction
a. A first promoter region,
b. A first coding region encoding
   i. an iron responsible element (IRE), and
   ii. a first fluorescent protein domain,
c. A second promoter region comprising an Fe²⁺ and/or ISC sensitive region,
d. A second coding region encoding
   i. a second fluorescent protein domain, wherein the first fluorescent protein domain and the second fluorescent protein domain are different, and
   ii. an IRE.
As regards the first fluorescent protein domain, the second fluorescent protein domain, and/or its respective features, the same applies as has been described herein above in connection with the nucleic acid according to the first aspect.

For example, the first fluorescent protein domain and the second fluorescent protein domain may be selected from the group consisting of mTagBFP2, mNeonGreen, and mCherry. For example, the first fluorescent protein domain may be mCherry. For example, the second fluorescent protein domain may be mNeonGreen.

For example, the first fluorescent protein domain can be mCherry. Accordingly, the first coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 14. For example, the first coding region can comprise a sequence encoding the sequence set forth in SEQ ID NO: 14. For example, the first coding region can comprise a sequence encoding an amino acid sequence having the same biological function as the sequence set forth in SEQ ID NO: 14. For example, the first coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 15. For example, the first coding region can comprise the sequence set forth in SEQ ID NO: 15. For example, the first coding region can comprise a sequence having the same biological function as the sequence set forth in SEQ ID NO: 15.

For example, the second fluorescent protein domain can be mNeonGreen. Accordingly, the second coding region can comprise a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 12. For example, the second coding region can comprise a sequence encoding the sequence set forth in SEQ ID NO: 12. For example, the second coding region can comprise a sequence encoding an amino acid sequence having the same biological function as the sequence set forth in SEQ ID NO: 12. For example, the second coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 13. For example, the second coding region can comprise the sequence set forth in SEQ ID NO: 13. For example, the second coding region can comprise a sequence having the same biological function as the sequence set forth in SEQ ID NO: 13.

### Module 2 - First coding region, further elements

In an embodiment of the thirteenth aspect, the first coding region comprises a 3' polyA tail region. For example, the polyA tail region can be a BgH polyA tail region. For example, the polyA tail region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 25. For example, the polyA tail region can comprise or consist of the sequence set forth in SEQ ID NO: 25. For example, the polyA tail region can comprise or consist of a sequence having the same biological function as the sequence set forth in SEQ ID NO: 25.

In an embodiment of the thirteenth aspect, the first coding region comprises a 3' terminator region. For example, the terminator region can be 3' of b) ii). For example, the terminator region can be 3' of a polyA tail region, preferably of the polyA tail region as described herein above.

For example, the first coding region can comprise a 3' terminator region, wherein the terminator region comprises a T7 terminator sequence and/or a Rrng terminator sequence.

For example, the terminator region can comprise in 5' to 3'direction a T7 terminator sequence and a Rrng terminator sequence. For example, the T7 terminator sequence can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 26. For example, the T7 terminator sequence can comprise or consist of the sequence set forth in SEQ ID NO: 26. For example, the T7 terminator sequence can comprise or consist of a sequence having the same biological function as the sequence set forth in SEQ ID NO: 26.

For example, the Rrng terminator sequence can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 27. For example, the Rrng terminator sequence can comprise or consist of the sequence set forth in SEQ ID NO: 27. For example, the Rrng terminator sequence can comprise or consist of a sequence having the same biological function as the sequence set forth in SEQ ID NO: 27.

### Module 2 - Second coding region

In an embodiment of the thirteenth aspect, d) ii) comprises one, two, three, four, five, six, seven, eight, nine or ten IRE(s). For example, d) ii) can comprise five IREs.

In an embodiment of the thirteenth aspect, an mRNA transcript of the second coding region comprises the IRE of d) ii) in a 3' untranslated region (UTR) of a sequence encoding d) i). For example, the 3'UTR can comprise or consist of a part of a Transferrin Receptor (TfR) 3'UTR. For example, the 3'UTR can comprise or consist of a Transferrin Receptor (TfR) 3'UTR. For example, the second coding region can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 23. For example, the second coding region can comprise or consist of the sequence set forth in SEQ ID NO: 23. For example, second coding region can comprise or consist of a sequence having the same biological function as the sequence set forth in SEQ ID NO: 23.

### Module 2 - Exemplarily designs

In an embodiment of the thirteenth aspect, the reporter protein domain is a fluorescent protein domain and the nucleic acid comprises in 5' to 3' direction
a. A first promoter region comprising or consisting of an hPGK,
b. A first coding region encoding
   i. an iron responsible element (IRE),
   ii. a first fluorescent protein domain,
   iii. a polyA tail region, preferably a BgH polyA tail region, and
   iv. a terminator region comprising a T7 terminator sequence and a Rrng terminator sequence,
c. A second promoter region comprising an Fe²⁺ and/or ISC sensitive region, wherein the Fe²⁺ and/or ISC sensitive region comprises or consists of eight HRE,
d. A second coding region encoding
   i. a second fluorescent protein domain, wherein the first fluorescent protein domain and the second fluorescent protein domain are different, and
   ii. an IRE, wherein the IRE is comprised in a part of a Transferrin Receptor (TfR) 3'UTR.

For example in view of a) and b), the nucleic acid can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 28. For example, the nucleic acid can comprise the sequence set forth in SEQ ID NO: 28.

For example in view of c) and d), the nucleic acid can comprise a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 24. For example, the nucleic acid can comprise the sequence set forth in SEQ ID NO: 24.

### Module 2 - Vectors and cell

In a fourteenth aspect, the present invention relates to a vector comprising the nucleic acid according to the thirteenth aspect. As regards the nucleic acid comprised in the vector and/or its respective features, the same applies as has been described herein above in connection with the nucleic acid according to the thirteenth aspect.

In an embodiment of the fourteenth aspect, the vector is a viral vector. For example, the vector can be a lentivirus vector, an adenovirus, an adeno-associated virus (AAV), and/or a baculovirus.

In an embodiment of the fourteenth aspect, the vector is a plasmid. For example, the plasmid can be a VB240227-1627une plasmid or a PcDNA3.1- plasmid.

In an embodiment of the thirteenth aspect, the vector comprises or consists of the sequence set forth in SEQ ID NO: 29.

In a fifteenth aspect, the present invention relates to a cell comprising i) the nucleic acid according to the thirteenth aspect and/or the vector according to the fourteenth aspect, and ii) an iron responsive protein (IRP). As regards the nucleic acid comprised in the cell and/or its respective features, the same applies as has been described herein above in connection with the nucleic acid according to the thirteenth aspect.

In an embodiment of the fifteenth aspect, the first polypeptide and the second polypeptide are stably or transiently expressed in the cell.

In an embodiment of the fifteenth aspect, the cell is a HeLa cell, a macrophage, an enterocyte, a hepatocyte, a cancer cell, an immune cell and/or a neuron. For example, the cel can be a HeLa cell. For example, the cell can be a cell exhibiting distinctive iron homeostasis characteristics, such as macrophages and enterocytes or hepatocytes. Accordingly, the cell can be for example a macrophage, an enterocyte or a hepatocyte. For example, the cell can be a cell with potential sensitivity to iron depletion. For example, the cell can be a cell with potential sensitivity to iron overload, such as various cancer cells, immune cells and neurons. Accordingly, the cell can be for example a cancer cell, an immune cell and/or a neuron.

### Module 2 - Methods

In the following, methods using, *inter alia,* the cell according to the fifteenth aspect are described. As regards the cell and its respective features, the same applies as has been described herein above in connection with the nucleic acid according to the thirteenth aspect, the vector according to the fourteenth aspect and/or the cell according to the fifteenth aspect.

In a sixteenth aspect, the present invention relates to an *in vitro* method for measuring cellular Fe²⁺ and/or cellular ISC, comprising:
a. Providing the cell according to the fifteenth aspect, and
b. Measuring a signal of the first reporter protein domain and a signal of the second reporter protein domain,
   wherein intensity of the signal of the first reporter protein domain is indicative of an amount of cellular ISC, and/or
   wherein intensity of the signal of the second reporter protein domain is indicative of an amount of cellular Fe²⁺ and/or cellular ISC.

In an embodiment of the sixteenth aspect, the first reporter protein domain is a first fluorescent protein domain, the second reporter protein domain is a second fluorescent protein domain, the respective signals are fluorescence, and the method comprises:
a. Providing the cell according to the fifteenth aspect and exciting the cell, and
b. Measuring fluorescence of the first fluorescent protein domain and fluorescence of the second fluorescent protein domain,
   wherein intensity of fluorescence of the first fluorescent protein domain is indicative of an amount of cellular ISC, and/or
   wherein intensity of fluorescence of the second fluorescent protein domain is indicative of an amount of cellular Fe²⁺ and/or cellular ISC.

In an embodiment of the sixteenth aspect, step b) further comprises obtaining a ratio from the measured intensity of the signal the first reporter protein domain and the measured intensity of the signal of the second reporter protein domain. Preferably, the first reporter protein domain is a first fluorescent protein domain, the second reporter protein domain is a second fluorescent protein domain, the respective signals are fluorescence, and step b) further comprises obtaining a ratio from the measured intensity of fluorescence of the first fluorescent protein domain and the measured intensity of fluorescence of the second fluorescent protein domain.

In an embodiment of the sixteenth aspect, the method further comprises prior or after step b): c. Amending an amount of extracellular and/or cellular Fe²⁺.

For example, the amount of Fe²⁺ and/or cellular ISC can be amended by adding to the cell a composition comprising Fe²⁺. For example, the composition can comprise FeSO₄. For example, the composition can be FeSO₄ Vitamin C.

Additionally or alternatively, the amount of Fe²⁺ and/or cellular ISC can be amended by adding to the cell an Fe²⁺ chelator, preferably a cell membrane permeable Fe²⁺ chelator. For example, the Fe²⁺ chelator can be eltrombopag (ELT).

Additionally or alternatively, the amount of Fe²⁺ and/or cellular ISC can be amended by adding a compound affecting or being suspected of affecting cellular Fe²⁺ and/or cellular ISC. For example, the compound can be an active ingredient, a drug candidate and/or a drug.

Additionally or alternatively, the method comprises after step c) another step b) referred to as step d).

Additionally or alternatively, the method further comprises
e. Comparing measurements obtained from step b) and a control and/or step d).

Preferably, in step e) ratios are compared.

In a seventeenth aspect, the present invention relates to an *in vitro* screening method for a compound that affects cellular Fe²⁺ and/or cellular ISC the method comprising the steps of
a. Providing the cell according to the fifteenth aspect,
b. Optionally measuring a signal of the first reporter protein domain and a signal of the second reporter protein domain and obtaining a ratio from the measured intensity of the signal of the first reporter protein domain and the measured intensity of the signal of the second reporter protein domain,
c. Adding a compound affecting or being suspected of affecting cellular Fe²⁺ and/or cellular ISC,
d. Measuring a signal of the first reporter protein domain and a signal of the second reporter protein domain and obtaining a ratio from the measured intensity of the signal of the first reporter protein domain and the measured intensity of the signal of the second reporter protein domain, and
e. Comparing the ratio obtained from step d) with the ratio obtained from step b) and/or a control,
   wherein intensity of the signal of the first reporter protein domain is indicative of an amount of cellular ISC, and/or wherein intensity of the signal of the second reporter protein domain is indicative of an amount of cellular Fe²⁺ and/or cellular ISC, and
   wherein a significant change in the ratio obtained from step d) and the ratio obtained from step b) and/or a control is indicative of the compound affecting cellular Fe²⁺ and/or cellular ISC.

Preferably, the first reporter protein domain is a first fluorescent protein domain, the second reporter protein domain is a second fluorescent protein domain, and the respective signals are fluorescence. Accordingly, in an embodiment of the seventeenth aspect, method comprises the steps of
a. Providing the cell according to the fifteenth aspect and exciting the cell,
b. Optionally measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain,
c. Adding a compound affecting or being suspected of affecting cellular Fe²⁺ and/or cellular ISC,
d. Measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain, and
e. Comparing the ratio obtained from step d) with the ratio obtained from step b) and/or a control,
   wherein intensity of fluorescence of the first fluorescent protein domain is indicative of an amount of cellular ISC, and/or wherein intensity of fluorescence of the second fluorescent protein domain is indicative of an amount of cellular Fe²⁺ and/or cellular ISC, and
   wherein a significant change in the ratio obtained from step d) and the ratio obtained from step b) and/or a control is indicative of the compound affecting cellular Fe²⁺ and/or cellular ISC.

In an eighteenth aspect, the present invention relates to an *in vitro* method for identifying a dosage and/or formulation of a compound that affects cellular Fe²⁺ and/or cellular ISC, the method comprising the steps of
a. Providing the cell according to the fifteenth aspect,
b. Optionally measuring a signal of the first reporter protein domain and a signal of the second reporter protein domain and obtaining a ratio from the measured intensity of the signal of the first reporter protein domain and the measured intensity of the signal of the second reporter protein domain,
c. Adding a compound affecting or being suspected of affecting cellular Fe²⁺ and/or cellular ISC,
d. Measuring a signal of the first reporter protein domain and a signal of the second reporter protein domain and obtaining a ratio from the measured intensity of the signal of the first reporter protein domain and the measured intensity of the signal of the second reporter protein domain, and
e. Comparing the ratio obtained from step d) with the ratio obtained from step b) and/or a control,
   wherein intensity of the signal of the first reporter protein domain is indicative of an amount of cellular ISC, and/or wherein intensity of the signal of the second reporter protein domain is indicative of an amount of cellular Fe²⁺ and/or cellular ISC, and
   wherein a significant change in the ratio obtained from step d) and the ratio obtained from step b) and/or a control is indicative of the dosage and/or formulation affecting cellular Fe²⁺ and/or cellular ISC.

Preferably, the first reporter protein domain is a first fluorescent protein domain, the second reporter protein domain is a second fluorescent protein domain, and the respective signals are fluorescence. Accordingly, in an embodiment of the eighteenth aspect, method comprises the steps of
a. Providing the cell according to the fifteenth aspect and exciting the cell,
b. Optionally measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain,
c. Adding a compound affecting or being suspected of affecting cellular Fe²⁺ and/or cellular ISC,
d. Measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain, and
e. Comparing the ratio obtained from step d) with the ratio obtained from step b) and/or a control,
   wherein intensity of fluorescence of the first fluorescent protein domain is indicative of an amount of cellular ISC, and/or wherein intensity of fluorescence of the second fluorescent protein domain is indicative of an amount of cellular Fe²⁺ and/or cellular ISC, and
   wherein a significant change in the ratio obtained from step d) and the ratio obtained from step b) and/or a control is indicative of the dosage and/or formulation affecting cellular Fe²⁺ and/or cellular ISC.

In an embodiment of the sixteenth, seventeenth and/or eighteenth aspect, the method further comprises prior step a) the step of contacting the cell according to the fifteenth aspect with the vector according to the fourteenth aspect.

In an embodiment of the sixteenth, seventeenth and/or eighteenth aspect, ISC is synthesized in presence of cellular Fe²⁺.

In an embodiment of the sixteenth, seventeenth and/or eighteenth aspect, an ISC-IRP complex is formed by binding of ISC to IRP.

In an embodiment of the sixteenth, seventeenth and/or eighteenth aspect, presence of ISC regulates translation of the first fluorescent protein domain. For example, translation of the first fluorescent protein domain can be positively regulated in a high cellular Fe²⁺ condition, preferably by binding of the ISC-IRP complex to the IRE in the first coding region. Alternatively, translation of the first fluorescent protein domain can be negatively regulated in a low cellular Fe²⁺ condition.

In an embodiment of the sixteenth, seventeenth and/or eighteenth aspect, presence of cellular Fe²⁺ and/or ISC regulates transcription and/or stability of an mRNA encoding the sequence encoding the second fluorescent protein domain.

For example, transcription of the second fluorescent protein domain can be positively regulated in a low cellular Fe²⁺ condition, preferably by binding of cellular Fe²⁺ and/or ISC to the Fe²⁺ and/or ISC sensitive region in the second promoter region. Alternatively, transcription of the second fluorescent protein domain can be negatively regulated in a high cellular Fe²⁺ condition.

Additionally or alternatively, stability of an mRNA encoding the sequence encoding the second fluorescent protein domain can be negatively regulated in a high cellular Fe²⁺ condition, preferably by binding of the ISC-IRP complex to the IRE 3' of the sequence encoding the second fluorescent protein domain. Alternatively, stability of the mRNA encoding the sequence encoding the second fluorescent protein domain can be positively regulated in a low cellular Fe²⁺ condition.

In an embodiment of the sixteenth, seventeenth and/or eighteenth aspect, the method is a ratiometric method.

In an embodiment of sixteenth, seventeenth and/or eighteenth aspect, the cell is excited at 405 nm, 470-490 nm, 561 nm, and/or 540-590 nm. Additionally or alternatively, fluorescence is measured at 450 nm, 500-520 nm, and/or 565-650 nm. Preferably, fluorescence of the first fluorescent protein domain is excited at 405 nm and/or 470-490 nm and measured at 450 nm and/or 500-520 nm. For example, fluorescence of the second fluorescent protein domain can be excited at 561 nm and/or 540-590 nm and measured at 565-650 nm. For example, fluorescence of the first fluorescent protein domain and fluorescence of the second fluorescent protein domain can be measured by dynamic time-lapse imaging. For example, fluorescence of the first fluorescent protein domain and fluorescence of the second fluorescent protein domain can be measured using a high-resolution microscopy. For example, fluorescence of the first fluorescent protein domain and fluorescence of the second fluorescent protein domain can be measured using a confocal technique.

In an embodiment of the sixteenth, seventeenth and/or eighteenth aspect, step b) is performed repeatedly and/or continuously. This can be advantageous for monitoring cellular iron amounts and changes thereof over time.

### Module 2 - Uses

In the following, uses of the nucleic acid according to the thirteenth aspect, the vector according to the fourteenth aspect and/or the cell according to the fifteenth aspect are described. As regards the nucleic acid, the vector, the cell, and their respective features, the same applies as has been described herein above in connection with the nucleic acid according to the thirteenth aspect, the vector according to the fourteenth aspect and/or the cell according to the fifteenth aspect.

In a nineteenth aspect, the present invention relates to the use of the nucleic acid according to the thirteenth aspect to obtain the vector according to the fourteenth aspect.

In a twentieth aspect, the present invention relates to the use of the vector according to the fourteenth aspect to express the nucleic acid according to the thirteenth aspect.

In a twenty-first aspect, the present invention relates to the use of the vector according to the fourteenth aspect to obtain the cell according to the fifteenth aspect.

In a twenty-second aspect, the present invention relates to the use of the cell according to the fifteenth aspect for *in vitro* measuring and/or monitoring ferrous iron (Fe²⁺) in a cell, preferably according to the method according to the sixteenth aspect.

In a twenty-third aspect, the present invention relates to the use of the cell according to the fifteenth aspect for *in vitro* screening for a compound that affects cellular Fe²⁺, preferably according to the method according to the seventeenth aspect.

In a twenty-fourth aspect, the present invention relates to the use of the cell according to the fifteenth aspect *in vitro* identifying a dosage and/or formulation of a compound that affects cellular Fe²⁺, preferably according to the method according to the eighteenth aspect.

### Genetically encoded iron biosensor system

The genetically encoded cellular Fe²⁺ biosensor system of the present invention, LlMS, comprises preferably Module 1 and Module 2 as described herein above. Thus, as regards Module 1, Module 2, and/or their respective features, the same applies as has been described herein above in connection with the respective Module (cf. Module 1: aspects one to twelve, Module 2: aspects thirteen to twenty-four). Module 1 provides at least one reporter protein domain signal and Module 2 provides at least two different reporter protein domain signals. Thus, LIMS comprising both Modules 1 and 2 can provide at least three different reporter protein domain signals as read-out. The at least three signals can be measured and/or investigated independently as described herein above in detail (cf. Methods of Module 1: aspects four to six, Methods of Module 2: aspects sixteen to eighteen, respectively). Additionally or alternatively, the at least three signals of LIMS comprising both Modules 1 and 2 can be obtained for a cell and investigated. A combined investigation of signals of Modules 1 and 2 can provide a more comprehensive insights into the dynamic cellular Fe²⁺ status, preferably encompassing LIP, ISC synthesis, Fe²⁺ uptake, release, export, and/or storage. Thus, analysing signals obtained from both Modules 1 and 2 can enable a more comprehensive assessment of whether a cell is, e.g., in a state of Fe²⁺ deficiency, iron overload, or balanced iron supply.

As regards the nucleic acids, vectors, cells, methods, uses, and/or its respective features, the same applies as has been described herein above in connection with the nucleic acids according to the first and thirteenth aspects, the vectors according to the second and fourteenth aspects, the cells according to the third and fifteenth aspects, the methods according to the fourth to sixth and sixteenth to eighteenth aspects, the uses according to the seventh to twelfth and nineteenth to twenty-fourth aspects. Moreover, also the other features of such nucleic acids, vectors, cells, methods, and/or uses can be as described herein, e.g. herein above.

In a twenty-fifth aspect, the present invention relates to a kit of nucleic acids comprising the nucleic acid according to the first aspect and the nucleic acid according to the thirteenth aspect.

In a twenty-sixth aspect, the present invention relates to a kit of vectors comprising the vector according to the second aspect and the vector according to the fourteenth aspect.

In a twenty-seventh aspect, the present invention relates to a vector comprising the nucleic acid according to the first aspect and the nucleic acid according to the thirteenth aspect.

In a twenty-eighth aspect, the present invention relates to a cell comprising i) the nucleic acid according to the first aspect and the nucleic acid according to the thirteenth aspect, ii) the vector according to the second aspect and the vector according to the fourteenth aspect, and/or iii) the vector according to the twenty-seventh aspect.

### Diseases and active ingredient and/or drug discovery

Module 1 and Module 2, either alone or in combination, are suitable for studying a disease, in which iron dysregulation may play a, potentially significant, role. In the following, diseases are exemplarily listed in which iron imbalance is hypothesized to play a critical role:
- Cancer: Iron overload and dysregulation are assumed to be closely linked to tumorigenesis, cancer progression, and/or resistance to treatment. Experimental models, such as cancer cell lines and xenografts, can be investigated using Module 1 and/or 2 as described herein, e.g. to measure, and optionally monitor, iron homeostasis and/or LIP changes that can impact cell survival.
- Neurodegenerative diseases: Diseases like Alzheimer's, Parkinson's, and Huntington's diseases are assumed to be associated with altered iron metabolism, which can lead to toxic iron accumulation, e.g. in neurons. Experimental models, such as cultured neuronal cells, can be investigated using Module 1 and/or 2 as described herein, e.g. to measure, and optionally monitor, iron homeostasis and/or deviation thereof that can contribute to neurodegeneration.
- Anemia and iron deficiency: Iron-deficiency anemia and related conditions are assumed to be directly influenced by cellular Fe²⁺ availability. Module 1 and/or 2 can be applied to study in a model, where iron deficiency is induced, how cells adapt their iron regulation in response to changing iron levels.
- Ferroptosis-related diseases: Ferroptosis, a form of regulated cell death triggered by iron accumulation, is assumed to be relevant in diseases such as acute kidney injury and ischemia-reperfusion injury. Module 1 and/or Module 2 can be advantageous for identifying when and how iron dysregulation can lead to ferroptosis and thus, for gaining a better understanding of these diseases and/or conditions.
- Hemochromatosis: Hemochromatosis refers to a genetic disorder characterized by iron overload and organ damage. Using Module 1 and/or Module 2, e.g. in patient-derived cell models, can be advantageous for assessing how excess iron and/or iron regulation can impact disease progression.

Module 1 and Module 2, either alone or in combination, can be advantageous for identifying active ingredients, for drug discovery, for optimization of formulation and/or for optimization of dosage regimes. Performing methods in accordance with any one of the fourth to sixth and sixteenth to eighteen aspects can be advantageous for identifying active ingredients, drugs, respective formulations and/or dosages that modulate and/or are capable of modulating cellular iron levels, e.g. by affecting LIP and/or ISC synthesis. By visualizing and quantifying these processes, compounds can be identified that can help correcting iron imbalances or preventing toxic iron accumulation, e.g. in diseases where iron metabolism is disrupted.

Module 1 and/or Module 2, either alone or in combination, can be advantageous as an/or within a companion diagnostic. Module 1 and/or Module 2, either alone or in combination, can help measuring *in vitro* a patient's iron status during treatment, e.g., with an iron-modulating therapy, a therapy suspected of modulating cellular iron, and/or a drug targeting iron metabolism.

It is to be noted that Module 1 and/or Module 2 can also be advantageous for identify differences in iron homeostasis and/or regulation thereof between different cell cultures, cell types, tissues, organoids, organs and/or organisms. Accordingly, any of the methods described herein, e.g. in accordance with the fourth to sixth and sixteenth to eighteen aspects of the present invention, can also encompass comparing signals between cells, e.g. between different cells within a tissue, wherein a significant change in the signal between cells can be indicative for a compound added thereto affecting cellular Fe²⁺ and/or ISC in a respective specific subgroup of the cells under study. For example, a significant change in such a comparison can be based on a significant change in signal intensities like in fluorescence intensities as described herein above. Additionally, in such a comparison the number of cells exhibiting a measurable and/or detectable signal can be considered and optionally added to one or more of the criteria of a significant change in signal intensity as indicated herein above. For example, in addition to any one of the criteria of a significant change in a signal, the number of cells with a detectable and/or measurable signal may (have to) change by at least about 10%, 25%, 50%, 75% or 100% compared to a control and/or compared to the cells before a compound under study has been added.

### Further remarks and definitions

The present invention as illustratively described herein may suitably be practiced in the absence of any element or elements, limitation or limitations, not specifically disclosed herein. Hence, it is to be understood that the invention is not limited to the particular methodology or protocols. It is also to be understood that the terminology used herein is for the purpose of describing particular embodiments only and is not intended to limit the scope of the present invention which will be limited only by the appended claims. Hence, the present invention is described herein with respect to particular embodiments and with reference to certain figures, but the invention is not limited thereto but only by the claims.

It must be noted that as used herein and in the appended claims, the singular forms "a," "and", and "the" include plural reference unless the context clearly dictates otherwise. Thus, for example, reference to "a vector" is a reference to one or more vectors and includes equivalents thereof known to those skilled in the art, and so forth.

As used herein, the word "or" means any one member of a particular list and also includes any combination of members of that list.

The words "comprise," "comprising," "include," "including," and "includes" when used in this specification and in the following claims are intended to specify the presence of one or more stated features, integers, components, or steps, but they do not preclude the presence or addition of one or more other features, integers, components, steps, or groups thereof.

Technical terms are used by their common sense. If a specific meaning is conveyed to certain terms, definitions of terms will be given in the following in the context of which the terms are used.

### Brief description of the drawings

**Figure 1****:** Potential key mechanisms controlling cellular Fe²⁺ status. Schematic representation of the assumed role of FBXL5 in maintaining cellular Fe²⁺ homeostasis and its potential interaction with the IRE-IRP system. Iron uptake via transferrin through the TfR pathway may lead to an increase in the labile iron pool (LIP). Lysosomal reduction and release of Fe²⁺ are not shown. An increase in LIP may stabilize FBXL5, thus preventing its degradation. Stabilized FBXL5 may bind to Aco2, thus leading to its degradation (right side of **Figure 1****).** As a result, ferritin (Fr) expression may increase to store excess Fe²⁺, while transferrin receptor (TfR) expression may be reduced, thereby limiting further iron uptake.

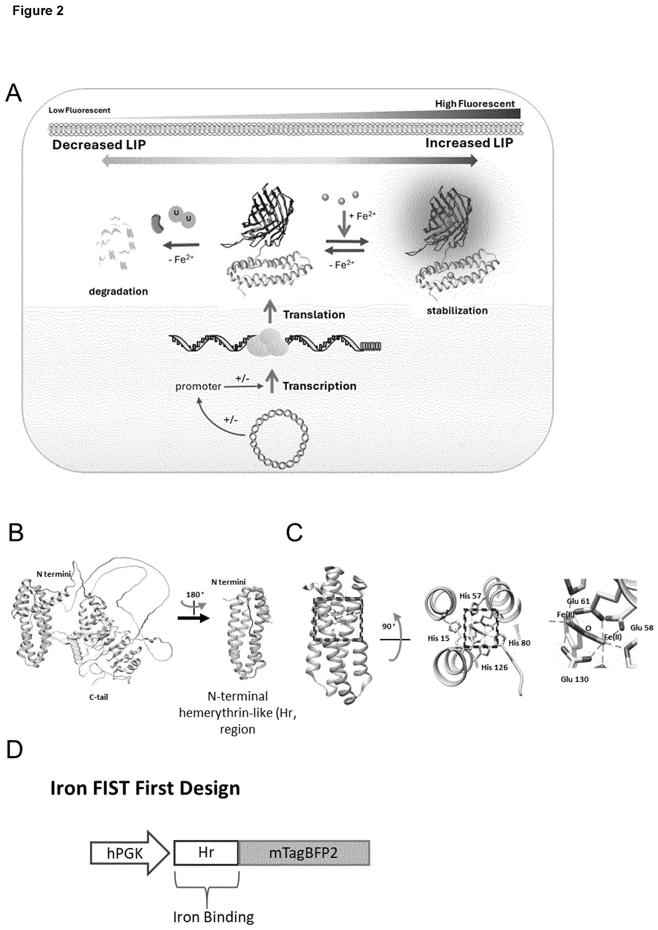

**Figure 2****:** Mechanism and structural basis of Module 1 - Design 1. A) Schematic representation of the mechanism of action underlying Module 1. As exemplarily shown, Module 1 may comprise a fluorescent protein domain such as a blue fluorescent protein domain, e.g. mTagBFP2. A nucleic acid, for example comprised in a vector, may comprise a coding region encoding a polypeptide comprising a fluorescent protein domain like mTagBFP2. Within a cell, the nucleic acid is transcribed into a respective mRNA, which is then translated into a polypeptide comprising a fluorescent protein domain and a Fe²⁺-sensitive domain such as a hemerythrin-like (Hr) domain. The polypeptide may thus be seen as a fusion protein. In the absence of cellular Fe²⁺, the fusion protein may be unstable due to ubiquitination and subsequent degradation. However, in the presence of cellular Fe²⁺, Fe²⁺ may bind to the Hr domain, thus stabilizing the polypeptide and preventing degradation. Accordingly, accumulation of blue fluorescence may be observed that is indicative for an increased labile iron pool (LIP). B) and C) Detailed structural representations of the Hr domain from a FBXL5 protein comprising an iron-dependent degron sequence. Zoom-in views in C) indicate a critical interaction between His 80 and Fe²⁺-O- Fe²⁺, which may facilitate protein stabilization in the presence of cellular Fe²⁺. D) Schematic representation of a DNA sequence encoding a cellular Fe²⁺ biosensor according to Module 1 - Design 1. The arrow indicates a promoter region, e.g. a promoter region encoding hPGK.

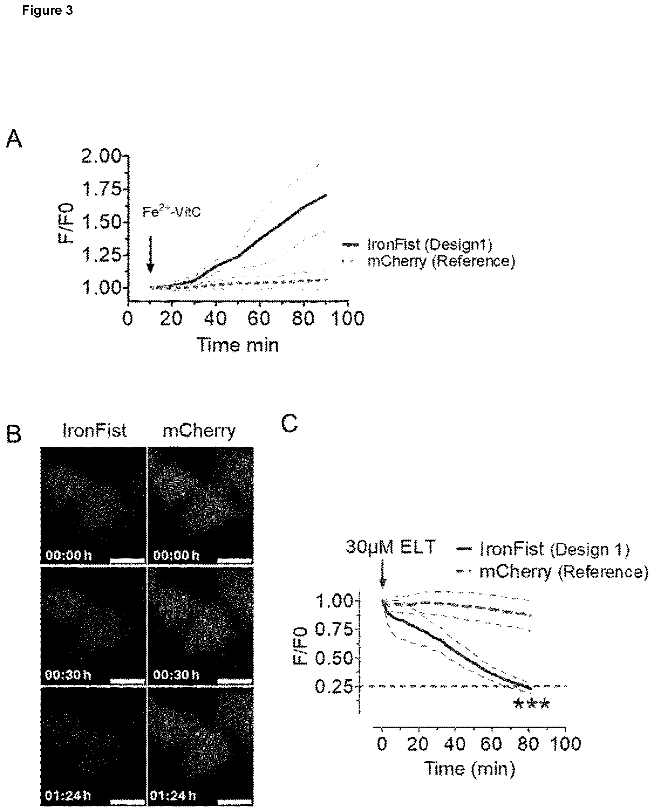

**Figure 3****:** Functional evaluation and dynamic response of Module 1 - Design 1 in HeLa Cells A) Curve graph showing average intensity ± SD (n=11) of blue fluorescence from mTagBFP (Module 1 - Design 1; dark solid line) and mCherry (control; dark dashed line) overtime in response to Fe²⁺ addition. HeLa cells were co-transfected with constructs encoding Module 1 - Design 1 and mCherry; cells expressing both constructs were selected. B) Representative images of HeLa cells pre-treated with FeSO₄/Vit C for 2 hours and expressing the Module 1 - Design 1 construct together with mCherry as internal control before and during treatment with the Fe²⁺ chelator ELT. Images show a decrease in blue fluorescence (Module 1 - Design 1) over time, indicating the response of the encoded polypeptide to cellular iron depletion. C) Corresponding intensity curves for blue fluorescence (Module 1 - Design 1; dark solid line) and red fluorescence (mCherry, iron-insensitive reference; dark dashed line) during the treatment shown in Panel B. *** indicate significant differences between mCherry and mTagBFP2 fluorescence intensity at given time points. p<0.0001.

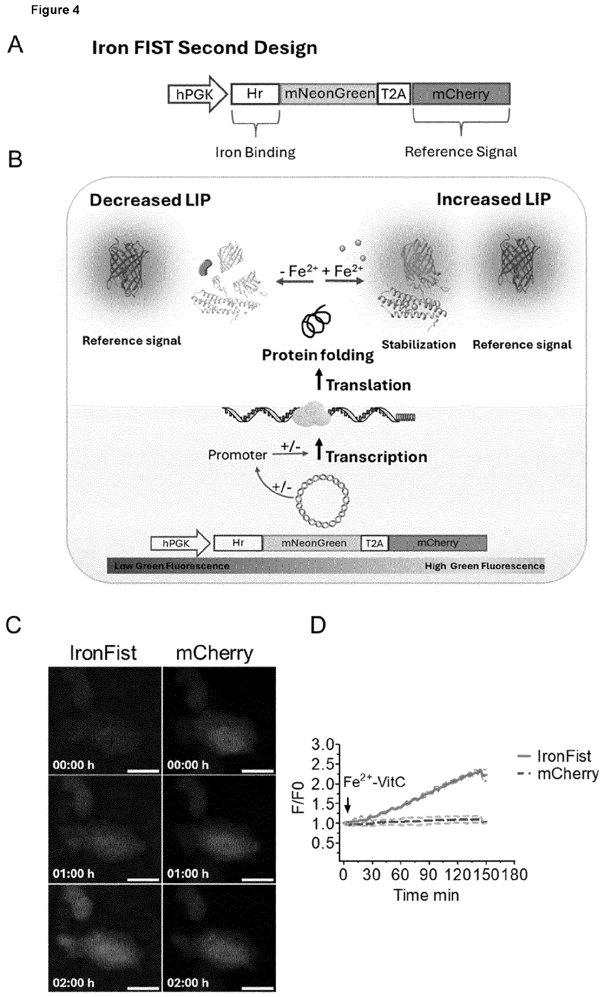

**Figure 4****:** Functional Evaluation of Module 1 - Design 2, a pseudo-ratiometric tool for monitoring the labile iron pool (LIP). A) Schematic representation of a DNA sequence encoding a cellular Fe²⁺ biosensor according to Module 1 - Design 2. The arrow indicates a promoter region, e.g. a promoter region encoding hPGK. Under the control of the promoter region are a Hr domain fused to mNeonGreen (a green fluorescent protein domain) as first fluorescent protein domain. The nucleic acid comprised further a sequence encoding a second fluorescent protein domain such as mCherry, a red fluorescent protein domain. Between the sequence regions encoding the first and the second fluorescent protein domains a ribosome skipping sequence was positioned such as a sequence encoding T2A. Using this design, the second fluorescent protein domain can serve as an iron-insensitive reference fluorescent signal and thus, as a control, while the Hr-mNeonGreen fusion polypeptide can respond to changes in cellular Fe²⁺ levels. Thus, Design 2 can represent a pseudo-ratiometric approach to measure and/or monitor cellular LIP. B) Cartoon illustrating the principle of Module 1 - Design 2. Upon binding of Fe²⁺ to the Hr domain of the fusion polypeptide, green fluorescence from mNeonGreen increases, while red fluorescence from mCherry remains unaffected, providing a pseudo-ratiometric readout indicative of the cellular status of the LIP. C) Representative time-lapse images of HeLa cells expressing Module 1 - Design 2 before (first row) and after treatment with FeSO₄ and Vitamin C (middle and last, third row). Green fluorescence from mNeonGreen increased following iron treatment, while red fluorescence from mCherry remained constant, thus indicating sensitivity to cellular Fe²⁺ fluctuations and changes in LIP. D) Time-lapse analysis of the green/red fluorescence ratio in HeLa cells expressing Module 1 - Design 2. The upper, dark solid curve indicates an increase in green fluorescence from mNeonGreen overtime, reflecting an increase in LIP, while the lower, dark dashed curve indicated the stable red fluorescence from mCherry serving as an Fe²⁺ insensitive internal control, thus confirming the pseudo-ratiometric functionality of Design 2.

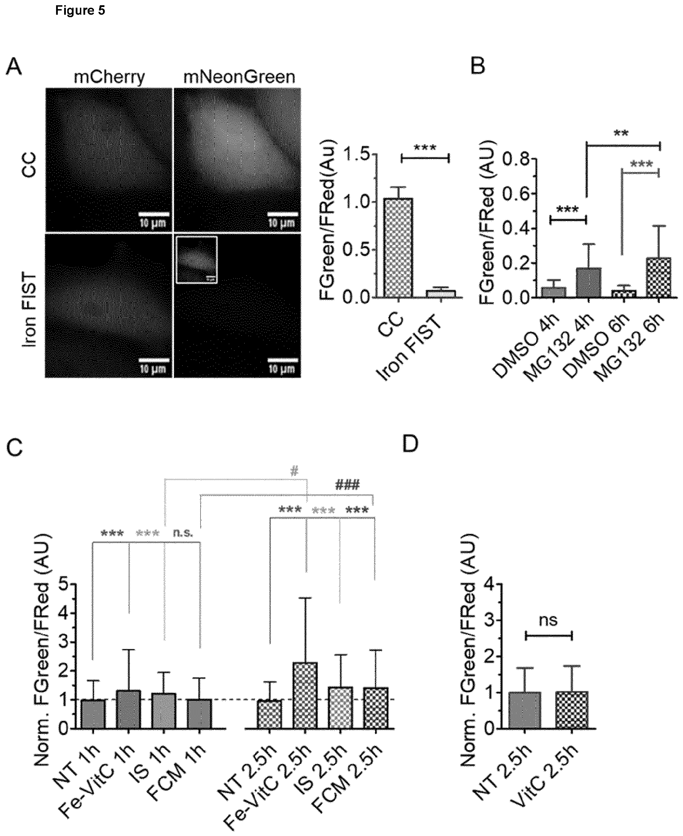

**Figure 5****:** Differential response of the exemplarily Module 1 - Design 2 construct, Vitamin C, and proteasome inhibition in HeLa Cells. A) Representative images of HeLa cells expressing the exemplarily Module 1 - Design 2 construct and the control construct, expression of mNeonGreen in Module 1 - Design 2 construct treated cell shown in smaller brightness contrast setting in inset image, with corresponding mNeonGreen/mCherry ratio plots. In the ratio plot (right panel), the left column represents the control construct (CC), and the right column represents the Module 1 - Design 2 construct under non-iron-treated conditions. B) Normalized mNeonGreen/mCherry ratio plots of HeLa cells expressing the Module 1 - Design 2 construct following treatment with 10 µM MG132 for 4 and 6 hours. Treatment for 4 hours (left) and 6 hours (right). n of DMSO 4h =3wells/114 cells n of DMSO 6 hours=3wells/118 cells no f MG132 4h=3wells/90 cells. N of MG132 6 h=3wells/129cells.C) Normalized mNeonGreen/mCherry ratio plots of HeLa cells expressing the Module 1 - Design 2 construct untreated (NT) or treated with either 150 µM FeSO₄ and 250 µM Vitamin C (Fe-VitC), 500 µg/mi IS, or 500 µg/ml FCM for 1 hour (left) and 2.5 hours (right), respectively. n of 1h NT= 9wells/240 cells, n of 1h Fe-VitC= 8wells/185 cells, n of 1h IS= 7wells/179 cells, n of 1h FCM= 9 wells/269 cells 2,5h NT= 9wells/240 cells, n of 2,5h Fe-VitC= 9wells/248 cells, n of 2,5h IS= 9wells/313 cells, n of 2,5h FCM= 9wells/256. D) Normalized mNeonGreen/mCherry ratio of HeLa cells expressing the Module 1 - Design 2 construct treated with 250 µM Vitamin C (VitC) compared to untreated cells (NT). n of NT= 9wells/244 cells, n of VitC= 7wells/160 cells. DATA=mean ±SD, statistical analysis non-parametric t test, *P<0.05, **P<0.001 ***P<0.0001 statistically significant.

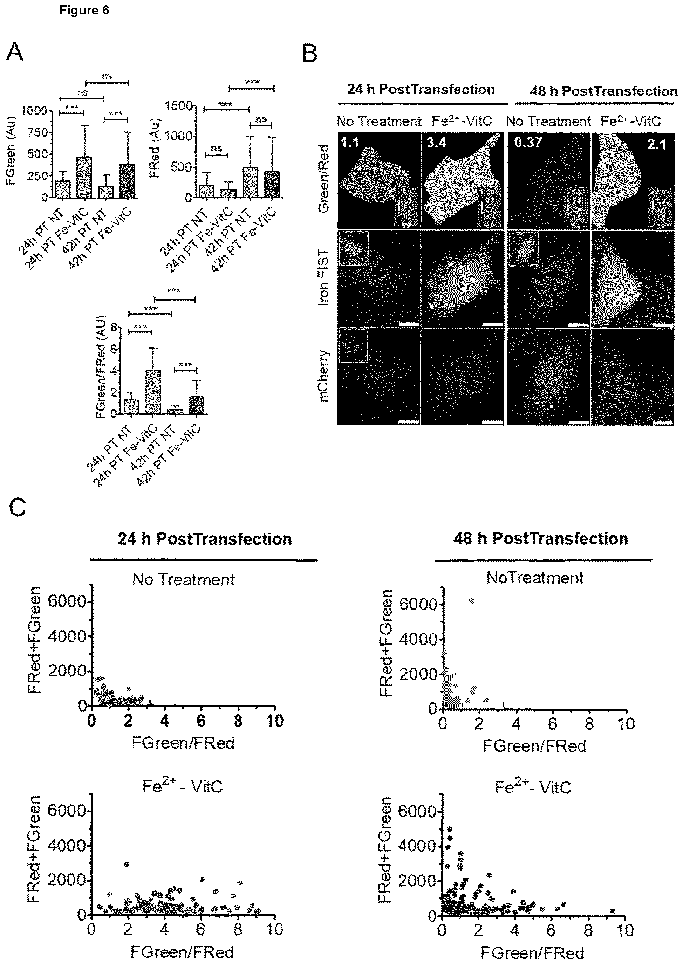

**Figure 6****:** Module 1 - Design 2 functionality over time post-transfection. A) Bar graphs showing fluorescence intensities and mNeonGreen/mCherry ratios at 24 hours (n=80 for untreated (NT); n=94 for Fe/VitC treated (Fe-VitC)) and for 42 hours (n=80 for NT; n=132 for Fe/VitC treated) post-transfection (PT), following treatment with FeSO₄ (150 µM) and Vitamin C (250 µM) for 2.5 h. In the top-left panel, average mNeonGreen intensities are displayed: the first column represents untreated cells (NT, n=80) at 24 hours post-transfection, followed by the second column for treated cells at the same time point (Fe-VitC, n=94). The next two columns show data for 42 hours post-transfection for untreated cells (NT, n=125) and for treated cells (Fe-VitC, n=132), respectively. The top-right panel displays corresponding values for the mCherry reference protein. In the lower panel, mNeonGreen/mCherry ratios for both time points are shown, comparing untreated (NT) and treated cells (Fe-VitC) to illustrate changes in iron sensitivity. ns indicates non-significant and *** significant differences P<0.001. B) Representative images of HeLa cells transfected with IronFist Design 2, with treatment conducted 24 or 42 hours post-transfection. The left set shows untreated and treated cells imaged 24 hours post-transfection, and the right set shows images taken at 42 hours. Each set displays the generated ratio images (top row), mNeonGreen fluorescence (middle row), and mCherry fluorescence (bottom row). C) Scatter plots display the mNeonGreen + mCherry sum (Y) plotted against the mNeonGreen/mCherry ratio values (X) of all the individual cells at both 24 (left) and 42 (right) hours, highlighting the absence of correlation between expression levels and ratio values. Label "Fe-VitC" used, e.g., in A) corresponds to label "Fe²⁺-VitC" used, e.g., in B) and C).

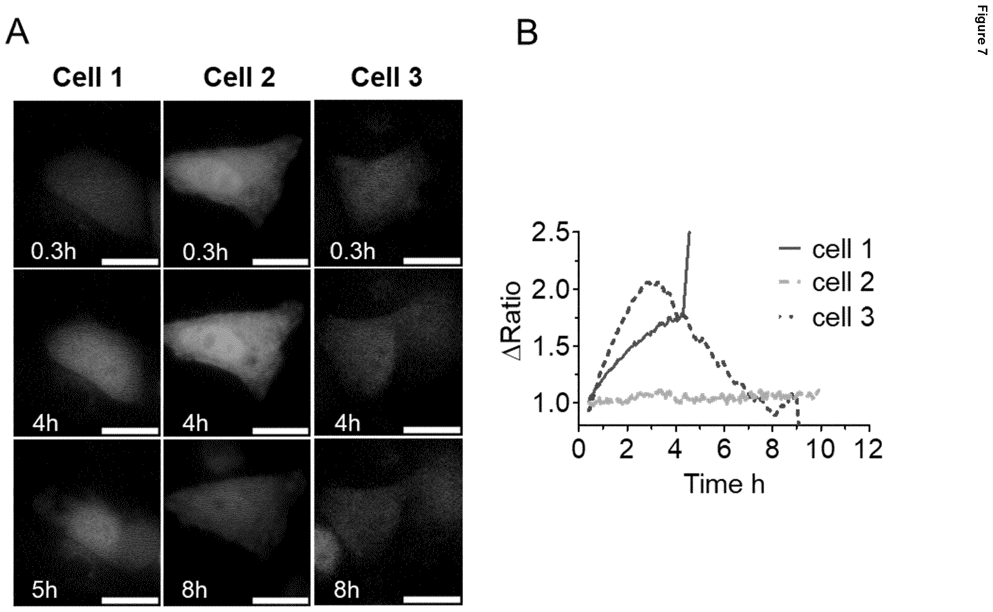

**Figure 7****:** Single-cell analysis of LIP dynamics using Module 1 - Design 2. A) Representative time-lapse images of three HeLa cells, each responding differently to FeSO₄ (150 µM) and Vitamin C (250 µM) treatment, demonstrating variability in labile iron pool (LIP) dynamics. Green (mNeonGreen) fluorescence channels are shown for each cell at three selected time points across the 8-hour monitoring period as indicated. B) Normalized ratio curves showing the green/red fluorescence ratio over time for each cell, highlighting distinct LIP responses. Ratio values were normalized to the initial ratio at the start of the measurement to emphasize changes. Cell 1 (dark solid line) showed a gradual increase in LIP over approximately 4 hours followed by cell death, likely due to iron toxicity. Cell 2 (light dashed line) maintained a stable ratio over the entire period, indicating no significant LIP increase. Cell 3 (dark dashed) showed an initial increase in the ratio, followed by a return to baseline, suggesting an initial LIP rise that the cell then regulated.

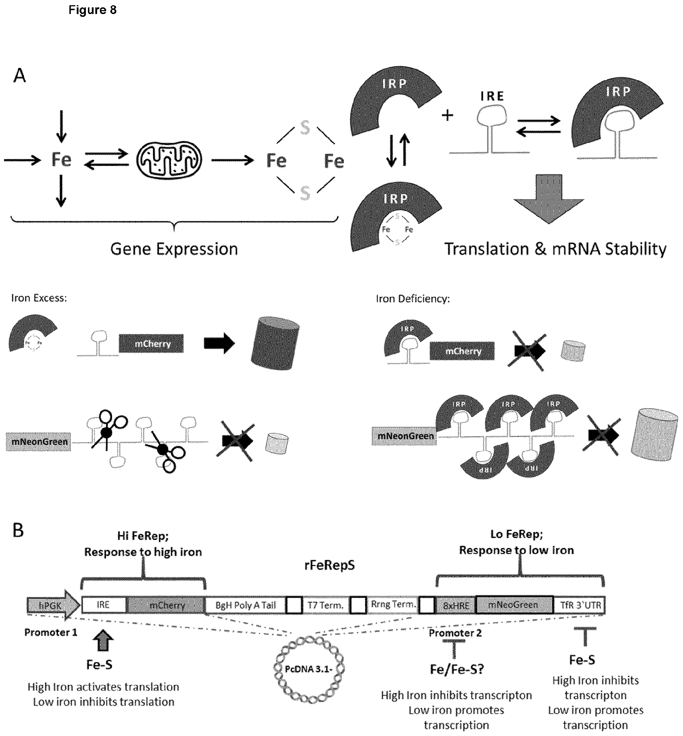

**Figure 8****:** Principle and design of Module 2 as a ratiometric biosensor, referred to in the Figure as rFeRepS. A) Upper panel: Iron within a cell is assumed to be utilized for iron-sulfur cluster (ISC) biosynthesis, a process for which mitochondria are considered key organelles. It is believed that ISC export from mitochondria allows integration into cytosolic proteins, such as iron regulatory proteins (IRPs), which control protein expression by interacting with iron response elements (IREs) on respective mRNAs. Lower panel: A system was exemplarily created that converted changes in cellular iron levels into a detectable red-green ratio metric fluorescent signal using a genetically encoded approach. B) rFeRepS DNA Structure: A plasmid was designed that was derived from the PcDNA3.1- vector, by incorporating iron response elements (IREs) on mRNAs regulated by endogenous iron-responsive proteins (IRPs). The system enabled opposing expression of two distinct fluorescent protein domains, mCherry and mNeonGreen, depending on cellular iron levels. An hPGK promoter controlled expression of the Hi FeRep mRNA, while a BgH Poly A Tail, T7 terminator, and Rrng terminator, all derived from the original PcDNA3.1- vector, were used to terminate Hi FeRep expression. Lo FeRep: The 8xHRE (hypoxia-responsive element) promoter region from the TfR gene controlling mNeonGreen expression under low iron conditions was applied. Additionally, stability of the mNeonGreen mRNA was regulated by five IRE repeats in the 3' UTR, reflecting the sequence of the TfR 3' UTR. The BgH Poly A Tail and terminator sequences were included after the TfR 3' UTR (not shown).

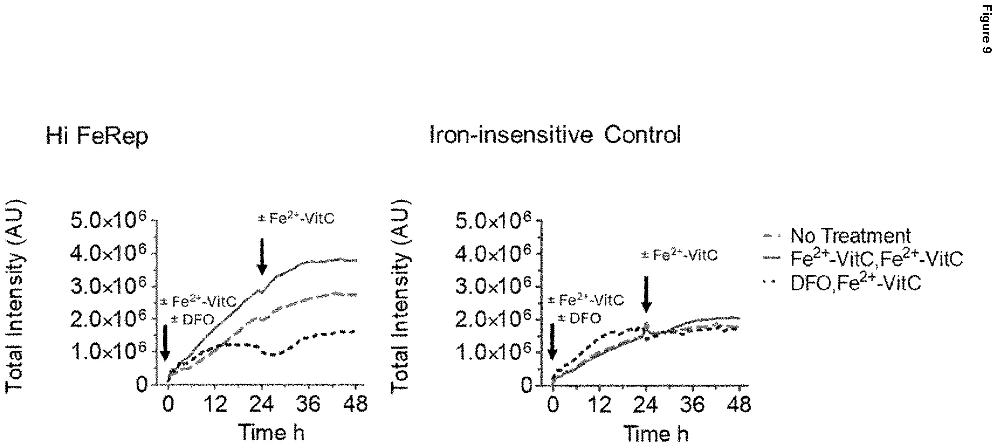

**Figure 9****:** Dynamic response of Hela cells expressing Hi FeRep or iron insensitive control construct without an IRE element (Hi FeRep -IRE). Left: Average curves (n=3 wells) showing total fluorescence intensity of HeLa cells expressing Hi FeRep, monitored overtime. Right: Total fluorescence intensity of HeLa cells expressing the iron-insensitive control construct i.e. Hi FeRep-IRE, monitored over time (average curves, n=3 wells). In both panels, solid curves represent cells treated twice (0 h and 24 h) with Fe²⁺ combined with Vitamin C (150 µM FeSO₄ and 250 µM Vitamin C; Fe²⁺-VitC), to increase intracellular iron levels. Dark dotted curves show cells pre-treated with 100 µM DFO (deferoxamine) at time zero; 24 hours later, the media was replaced with Fe²⁺ combined with Vitamin C to restore iron levels (DFO, Fe²⁺-VitC). Light dashed curves represent untreated control cells (NT), which received no additional iron or chelator treatment.

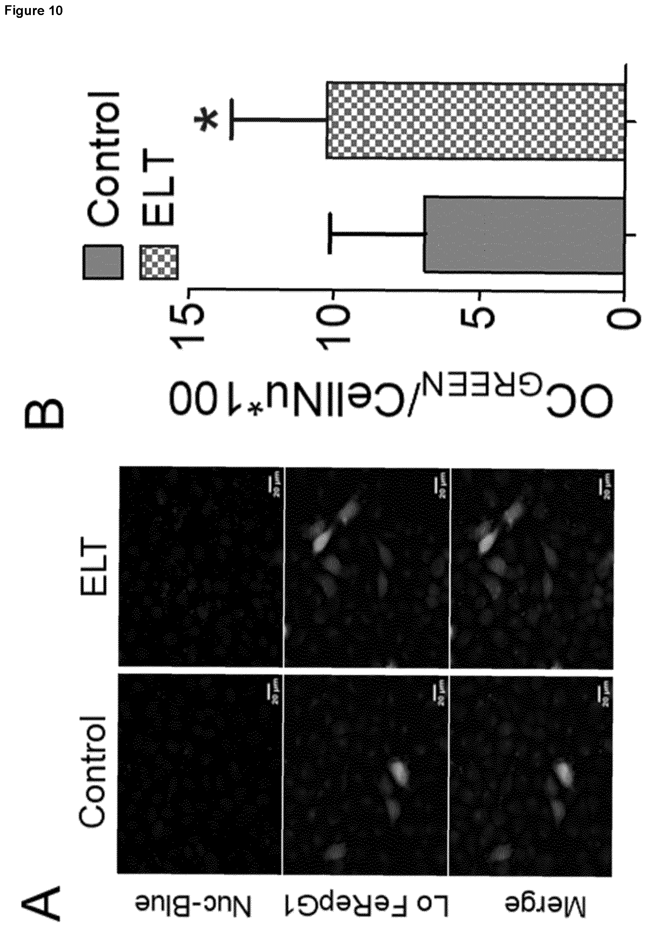

**Figure 10****:** Response of Lo FeRep to ELT treatment in HeLa cells. A) Representative images of HeLa cells expressing Lo FeRep, with nuclei stained in blue to count the number of cells per imaging area (upper row). The green channel shows the Lo FeRep signal (middle row), and the merged image combines both channels (lower row). B) Bar graph showing the average percentage of Lo FeRep-positive cells, with error bars representing ± SD. The left bar (n=3 wells) represents control conditions, while the right bar (n=3 wells) shows cells treated with ELT. * indicates statistically significant differences (p < 0.05) between control and ELT-treated conditions.

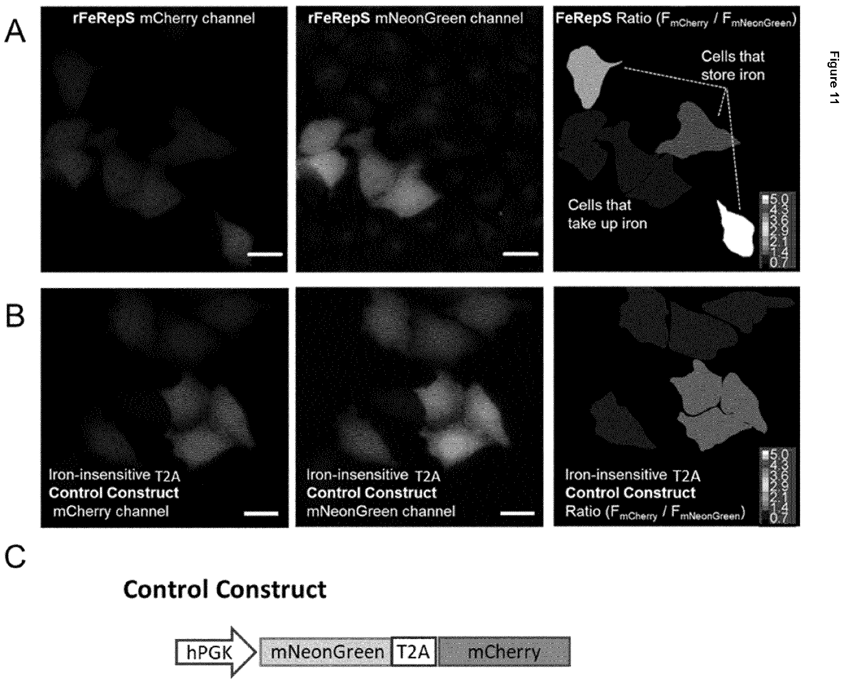

**Figure 11****:** Evaluation of rFeRepS iron-sensing capabilities in HeLa cells. A) Representative images of HeLa cells transiently expressing rFeRepS. The left image shows mCherry fluorescence, the middle image shows mNeonGreen fluorescence, and the right image displays the mCherry/mNeonGreen ratio image, highlighting cells with varying iron levels. B) Representative images of HeLa cells expressing the control construct, designed to produce mCherry and mNeonGreen independently of iron levels. In these control cells, fluorescence intensity ratios remained consistent across the cell population, confirming that variations in Panel A were due to iron-sensing elements within rFeRepS. C) Schematic diagram of the control construct design. Both mCherry and mNeonGreen were expressed under the hPKG promoter with a T2A ribosome skipping sequence, ensuring equal and iron-independent expression of both fluorescent proteins.

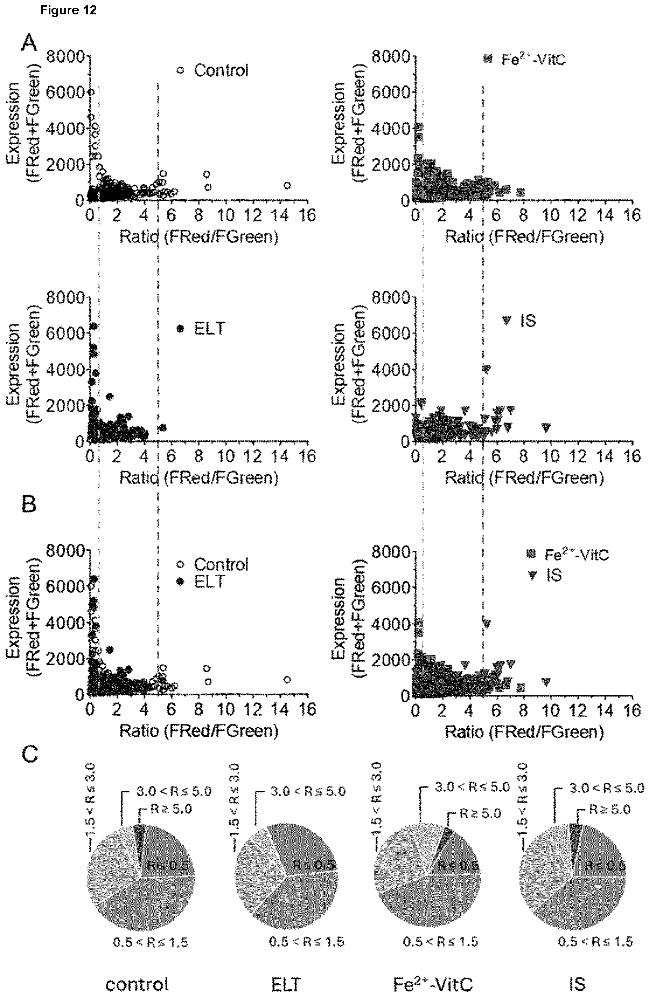

**Figure 12****:** Distribution of mCherry/mNeonGreen ratios in HeLa Cells under different treatment conditions. A) XY plots showing the distribution of mCherry/mNeonGreen ratios versus total fluorescence intensity for HeLa cells expressing rFeRepS. The upper left panel (A) shows control cells (open circles), while the upper right panel (A) displays cells treated with 150 µM FeSO₄ and 250 µM Vitamin C (Fe²⁺-VitC, filled squares). The lower left panel (A) depicts cells treated with 30 µM ELT (dark filled circles), and the lower right panel (A) shows cells treated with 400 µg/mi IS (dark filled triangles). All cells were treated for 8 hours prior to imaging. B) Left: Overlay of control cells (open circles) and ELT-treated cells (dark filled circles), showing the distinct shifts in ratio distribution. Right: Overlay of cells treated with FeSO₄ (Fe²⁺-VitC, dark filled squares) and IS (dark filled triangles), illustrating differences in ratio distributions following these treatments. C) Pie charts illustrating the percentage distribution of ratio values for each treatment condition, as indicated.

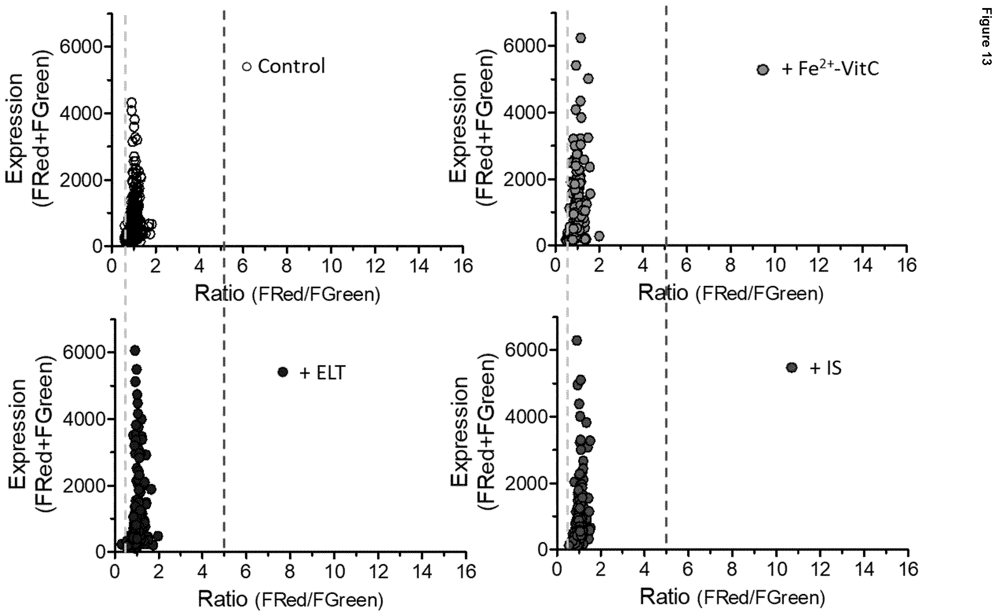

**Figure 13****:** mCherry/mNeonGreen ratio distribution in control construct expressing HeLa cells under different treatments. XY plots showing the distribution of mCherry/mNeonGreen ratios versus total fluorescence intensity for HeLa cells expressing the control construct. Open circles represent untreated control cells, light filled circles indicate cells treated with 150 µM FeSO₄ and 250 µM Vitamin C (Fe²⁺-VitC), dark filled circles show cells treated with 30 µM ELT, and middle dark filled circles represent cells treated with 400 pg/ml IS. All cells were treated for 8 hours before imaging. The ratio values remained consistent across all treatments

### Examples

### Materials and Methods

### Buffers and solutions

Cell culture materials were obtained from Grainer Bio-One (Kremsmünster, Austria). Experimental buffer 1 prepared in house with following ingredients and concentrations (in mM); 2 CaCl2, 135 NaCl, 1 MgCl2,5 KCI, 10 HEPES, 2.6 NaHCO3, 0.44 KH2PO4, 0.34 Na2HPO4, 1x amino acids, 1x vitamins, 10 D-Glucose, 2 L Glutamine (pH=7.45) NucBlue^{™} was purchased from ThermoFisher Scientific #33342.

### Compounds

Eltrombopag (ELT), MG 132, Erastin-2 (Ers-2) were purchased from MedChem Express (#HY-15306, #HY-13259, #HY-139087, respectively) purchased from MedChem, expressed and dissolved in DMSO at 10 mM concentration then aliquoted and kept at -20 °C for further use and used at 10 µM final concentration. IS np, (ferric iron sucrose nanoparticle) and FCM (Ferric carboxy maltose) were obtained from Vifor pharma. Ferrous sulphate heptahydrate was purchased from Sigma Aldrich #215422. Ascorbic acid is obtained from Aponorm. Fe-Vitamin C mix prepared freshly by dissolving both compounds in double distilled water.

### Molecular cloning and design

Module 1: First prototype (Design 1; plasmid sequence given in SEQ ID NO: 18) of iron FIST were designed by fusing a blue fluorescent protein domain, mtagBFP2, to a Hr domain of FBXL5. To generate a pseudo-ratiometric approach (Design 2; sequences given in SEQ ID NOs: 1 and 30 (nucleic acid part a) and b), c) and d), respectively); plasmid sequence given in SEQ ID NO: 19), a plasmid was designed which encoded a fusion of a Hr domain to mNeonGreen followed by a ribosome skipping sequence, T2A, and mCherry as a reference signal. As non-iron dependent control a control plasmid was designed, which expressed both fluorescent protein domains under the same promoter. A moderate strength promoter, hPGK (human phosphoglycerate kinase), was used to not overwhelm proteasomal degradation in the cells. All three plasmids were synthetized by Vector Builder (Neu-Isenburg, Germany).

It is to be noted that the sequence encoding a reporter protein domain like mNeonGreen (cf. nucleic acid sequence SEQ ID NO: 13) can also be used in a construct according to the present invention with omitting the initial start codon when used as a reporter protein domain in a fusion protein, wherein the reporter protein domain is fused C terminal, as, e.g., in case of SEQ ID NOs: 19 and 30. The same holds true in case of respective encoded amino acid sequences. For example in case of a reporter protein domain being mNeonGreen (cf. amino acid sequence SEQ ID NO: 12), the initial M may be omitted when fused C terminal in a fusion protein. Furthermore, stop codons may be used interchangeably. The skilled person is aware that the same rational holds true in case of other reporter protein domains.

Module 2: Plasmids encoding LoFeRepG1, HiFeRepR1, Hi FeRep R1-IRE and, mCh_T2A_mNeonGreen (control) were synthesized and cloned by Vector Builder (Neu-Isenburg, Germany). PcDNA 3.1- encoding bGH polyA tail signal, T7 terminator, and rrnG terminator between EcoRI and BamHl restriction sites (here and on referred as PAT&T in PcDNA3.1-), was purchased from Gene Universal (Delaware, USA). To express both FeReps in one vector HiFeRepR1 was PCR amplified with forward primer carrying Bglll enzyme site in the 5' and EcoRI restriction site at the 3' end then ligated into PAT&T in PcDNA 3.1-. After confirming insertion of HiFeRepR1 into the vector, LoFeRepG1 and HiFeRepR1 carrying PAT&T PcDNA 3.1- plasmids were cut by BamHI and HinDIII enzymes and then ligated. The resulting plasmid carrying both constructs was named Module 2, herein also referred to as rFeRepS (preferred plasmid sequence given in SEQ ID NO: 29; sequence of Hi FeRep given in SEQ ID NO: 28, sequence of Lo FeRep given in SEQ ID NO: 24).

It is to be noted that the promoter region having a sequence as given in SEQ ID NO: 1 can also be used in a construct according to the present invention omitting the initial three Gs, as, e.g., in case of SEQ ID NOs: 28 and 29.

### Cell culture and transfection

Hela S3 cells were cultured in Dulbecco's modified Eagle medium (DMEM D5523, Sigma Aldrich) supplemented with 10% FCS, 1% penicillin-streptomycin, 1.25mg/mL amphotericin B and 25 mM HEPES (pH=7.45). Cells were routinely passaged every 2-3 days and kept in a humidified cell culture incubator (37°C 5%CO2). Before any experiment, cells were seeded on 30mm glass coverslips (Co. KG, Lauda-K6nigshofen) in 6 well plates (Paul Marienfeld GmbH, Germany). One day after seeding cells were transiently transfected with respective plasmids with Polyjet (Signagen Laboratories, Rockville, USA) according to the manufacturer's manual. For transfection of a single plasmid 2 µL, and for co-transfection of two plasmids 3 µL of the reagent was used. Transfected cells imaged after 36-42 hours.

### End point live cell imaging

Transfected Hela S3 cells were imaged with Nikon Eclipse Ti2 (Nikon, Austria) equipped with CoolLED pE800 light source (CoolLED, UK, Andover), LED light source with 365, 400, 435, 470, 500, 550, 580, 635, and 740 nm (LEDs is introduced at the back focal plane), Apo 40X/1.15NA water immersion objective, two back-illuminated Kinetix Scientific CMOS cameras (TELEDYNE PHOTOMETRICS, USA, Tucson) mounted to the Nipkow based Crest optics X Light V3 with 70µm pinhole spinning disc system (Crest optics, Italy, Rome) in EPI fluorescent mode by bypassing the spinning disc. Nikon software (NIS-Elements AR 5.42.06 (Build 1821) LO 64bit, Nikon, Austria) was used for microscope control and image acquisition.

Imaging Hela cells expressing LoFeRep was performed with co staining of nucleus for normalization of positive green cells to cell number. For this, 2 drops of NucBlue^{™} were added on each well before imaging and the plate was kept in a cell culture incubator for 15 minutes before imaging.

For imaging of Hela cells expressing Module 2, cells were either treated with IS (400µg iron/mL/35mm dish), Fe²⁺ -Vitamin C mixture, (150µM, 250µM, respectively) or ELT (30µM) for 8 hours or non-treated (control) before imaging. After treatment, cell media were aspirated and replaced with EHL solution.

Imaging of Hela cells expressing Module 1 were treated with IS and FCM (500µg iron/mL/35mm dish) or Fe²⁺ -Vitamin C mixture (150µM, 250µM, respectively) for 1 and 2,5 hours. After treatment, cell media were aspirated and replaced with EHL solution before imaging.

### Kinetic measurement of Module 1 using fluorescence microscopy

Dynamic measurements of Module 1 - Design 1 (First prototype) cells were either pre-treated with Fe²⁺ -Vitamin C mixture for 2 hours or non-pre-treated, then imaging started with Fe²⁺ -Vitamin C mixture treatment (150µM, 250µM, respectively) or ELT (30µM). Imaging was done in 3 minutes intervals for nearly 2 hours.

### Time-lapse imaging in the cell culture incubator

For Module 2, kinetic measurements of Hi FeRep R1, and Hi FeRepR1-IRE (iron insensitive control construct) were performed in 12 well plates imaged in 1 hour intervals for 2 days in a device that was placed in a cell culture incubator, CellCyteX^{™} (Cytena, Freiburg Germany) with 10X dry objective. First, cells were seeded on a plastic clear bottom 12 well plate (Greiner Bio-one, #665180), the next day cells were transfected with Hi FeRep R1, and Hi FeRep R1-IRE carrying plasmids. After overnight incubation, media was aspirated and changed with 100 µMD DFO (Deferoxamine) or Fe²⁺-VitC (150, 250 µM, respectively), containing fresh medium, then plates were transferred to the imaging device and imaging started with bright field and red channel.

### imaging analysis

Images were processed using Fiji software. For two channel intensity calculations, a macro script was used. First, background subtracted by using a rolling ball function. ROls (regions of interest) were manually drawn around cells and thus obtained intensities then transferred to Excel files. Finally, ratio and sum of both channels (*F*_{Red}/*F*_{Green}, *F*_{Green}/*F*_{Red}, and *F*_{Red}+F_{Green}, where applicable) were calculated in Excel. Ratio images were generated by using each cell's ROI and ratio assigned by the math function of Fiji.

### Data analysis

Data obtained from microscope images were transferred and analysed using GraphPad Prism 5 Software (GraphPad Software, Inc., La Jolla, CA, USA)

### Module 1 - Design 1

### Overview of experiments performed with Module 1

A series of experiments was conducted to exemplarily assess the response of two different Module 1 designs, namely Design1 **(****Figure 2** A and D) and Design 2 **(****Figure 11** A and B) in HeLa cells **(Table 1).**

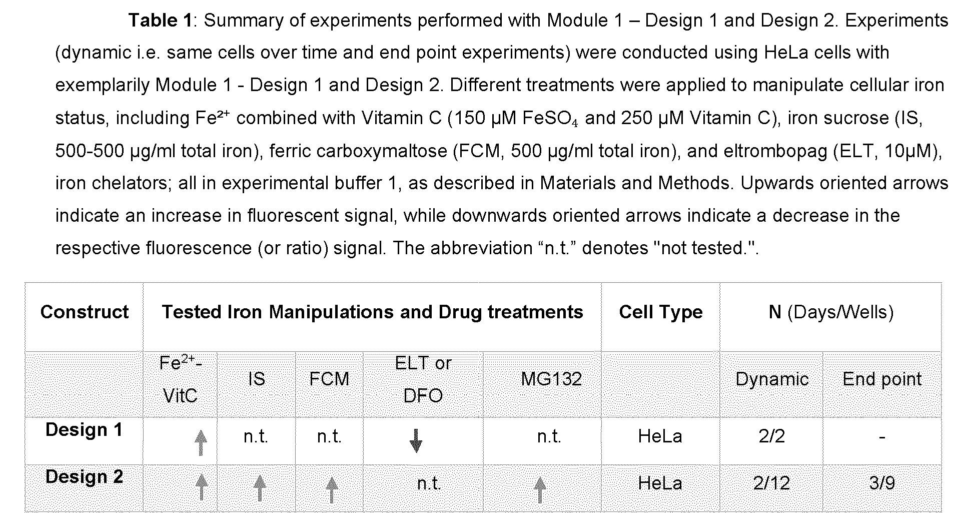

### A Functional Prototype for cellular Fe²⁺ sensing

A first prototype of Module 1 as illustrated in **Figure 2** A) and D) was tested exemplarily. Module 1 - Design 1 used a plasmid that drives the expression of a construct under the control of the moderate hPGK promoter. The construct included an Hr domain derived from a FBXL5 protein (cf. **Figure** 1), which may regulate iron homeostasis. For Module1 - Design 1 the Hr domain was fused with mTagBFP2 as a blue fluorescent protein domain. It was hypothesized that such a construct would undergo degradation via the ubiquitin-proteasome system under conditions of low cellular Fe²⁺ availability. On the other hand, it was assumed that when the labile Fe²⁺ pool rises, cellular Fe²⁺ would bind to the Hr domain, thus stabilizing the fusion polypeptide and preventing its degradation. As a result, the fusion polypeptide including mTagBFP2 would accumulate and its fluorescence signal would be detectable. Module1 - Design 1 can provide a dynamic Fe²+-dependent readout of cellular iron levels.

### Structural basis of Module 1 - Design 1

The core principle underlying Module 1 - Design 1 as exemplarily investigated in this Example may be summarized as being based on the iron-regulated degradation of the fusion polypeptide that is driven by the Hr domain derived from the FBXL5 protein. The predicted structure of FBXL5 and the crystal structure of its Hr domain are shown in Figure 2 B) and C). The Hr domain contains a hemerythrin-like structure that comprises an iron-dependent degron sequence. This sequence, located within amino acids 75-85, appears critical for regulating the stability of the polypeptide in response to cellular iron levels. Zoom-ins at different angles in **Figure 2** B) and C) illustrate assumed key amino acid residues involved in iron coordination. Specifically, His 80, located within the degron region, appears to play a crucial role in the interaction with the Fe²+-O-Fe²+ complex. In the absence of cellular Fe²⁺, the degron is exposed, leading to ubiquitination and degradation of the polypeptide. However, when cellular Fe²⁺ binds to the His 80 residue, it stabilizes the polypeptide by preventing the degron from being recognized by the cellular degradation machinery, thus preventing its ubiquitination and degradation. This structure-function relationship underpins relevance and functioning of Module 1, allowing for a dynamic and Fe²⁺-sensitive response that can be used to monitor cellular iron levels.

### Functional evaluation of Module 1 - Design 1 in HeLa cells

To assess the functionality of the construct, HeLa cells were co-transfected with the construct and mCherry, a red fluorescent protein domain, and cells selected that exhibited both red and faint blue fluorescence. Presence of both fluorescence signals indicated expression of the Module 1 - Design 1 construct. The cells were treated with Fe(ll) sulfate and Vitamin C to elevate iron levels and fluorescence signals were monitored for both channels over time using live cell imaging.

Upon treatment with Fe²⁺, a significant increase in blue fluorescence over time was observed in many cells, while red fluorescence remained constant (**Figure 3** A). This result demonstrated that the Module 1 - Design 1 construct functioned as intended, with an increase in labile iron pool (LIP) triggering stabilization of the encoded fusion polypeptide construct and a corresponding increase in blue fluorescence.

To further assess the dynamic sensitivity of the Module 1 - Design 1 construct to cellular Fe²⁺ levels, HeLa cells were first treated with Fe²⁺ to increase their iron content and then ELT (iron chelator) was added during time-lapse imaging. While the red fluorescence from the iron-sensitive mCherry remained stable, blue fluorescence from the encoded fusion polypeptide gradually decreased over time (Figure 3 B and C). This indicated that the Module 1 - Design 1 construct was capable of dynamically and reversibly reporting changes in cellular Fe²⁺ levels, with a decrease in blue fluorescence corresponding to a reduction of the labile iron pool (LIP) upon iron chelation by ELT treatment. This confirmed responsiveness of the Module 1 - Design 1 system to both iron supplementation and depletion, further validating its potential as a tool for monitoring cellular iron dynamics.

### Functional evaluation of Module 1 - Design 2: a pseudo-ratiometric tool

To further improve ratiometric sensing of the labile iron pool (LIP), Module 1 - Design 2 was investigated exemplarily. The respective construct incorporated a green/red fluorescence ratio system. In this example of Design 2, the Hr domain was fused with mNeonGreen, a bright green fluorescent protein domain variant, while mCherry, an iron-insensitive reference fluorescent protein domain, was expressed via a ribosome skipping sequence **(****Figure 4** A and B). This enabled a pseudo- ratiometric detection of cellular Fe²⁺ by measuring green (mNeonGreen) and red (mCherry) fluorescence intensities.

To test the functionality of the investigated Design 2, its performance was examined in HeLa cells. Upon treatment with FeSO₄/Vit C, a significant increase in the green/red fluorescence ratio was observed, as shown in **Figure 4** C) and D). The data demonstrated that Module 1 - Design 2 was sensitive to changes in cellular Fe²⁺ levels and can be used to effectively monitor the LIP through pseudo-ratiometric fluorescence time-lapse imaging.

### End-point analysis of Module 1 - Design 2 pseudo-ratiometric response in HeLa cells

To evaluate the response of Module 1 - Design 2 compared to a control construct, the mNeonGreen/mCherry fluorescence ratio was measured in HeLa cells under various conditions. HeLa cells were transfected with either IronFist or the iron-insensitive control construct and imaged two days post-transfection. Under untreated conditions, cells expressing the Module 1 - Design 2 construct exhibited a significantly lower mNeonGreen/mCherry ratio (0.07) than those expressing the control construct (1.03), indicating insufficient labile iron pool (LIP) to stabilize the fusion polypeptide of the Module in the absence of supplemental iron **(****Figure 5** A). To confirm that the Hr-mNeonGreen component of the Module undergoes proteasomal degradation, cells expressing the construct were treated with the proteasome inhibitor MG132 for 4 and 6 hours. This treatment increased the mNeonGreen/mCherry ratio by selectively enhancing green fluorescence intensity, validating that Hr-mNeonGreen degradation occurs through the proteasomal pathway **(****Figure 5** B).

Further testing involved treating HeLa cells with FeSO₄/Vitamin C, iron supplementation (IS), or ferric carboxymaltose (FCM) for 1 and 2.5 hours, followed by end-point imaging. FeSO₄/Vitamin C treatment produced a clear increase in the mNeonGreen/mCherry ratio at 1 hour, with a more pronounced effect observed at 2.5 hours **(****Figure 5** C). IS treatment produced only a slight increase in the ratio at 1 hour, but the effect became more evident by 2.5 hours. In contrast, FCM did not affect the ratio at 1 hour but increased it by 2.5 hours, indicating a slower response relative to IS and FeSO₄/Vitamin C. To ascertain whether Vitamin C alone influences the IronFist ratio, cells were treated with Vitamin C without iron supplementation. Vitamin C alone did not alter the mNeonGreen/mCherry ratio significantly at 2.5 hours, suggesting that the observed ratio changes were specific to iron exposure in the FeSO₄/Vitamin C condition **(****Figure 5** D).

### Broad detection window from 24 to 42 hours post-transfection for reliable iron sensing with Module 1 - Design 2

It was hypothesized that following transfection, mRNA and expression levels of the Module 1-Construct 2 construct may progressively increase, reaching a peak due to rising rates in transcription, translation, and protein folding before gradually declining due to cell division and probably other processes. Consequently, the timing post-transfection could potentially influence the ratiometric output of Module - Design 2. However, findings showed that at both 24- and 42-hours post-transfection, FeSO₄/Vitamin C treatment significantly increased mNeonGreen fluorescence without affecting the iron-insensitive mCherry reference fluorescence, leading to a marked increase in the green/red ratio at both time points **(****Figure 6** A and B). As expected, mCherry fluorescence intensity was significantly higher at 42 hours compared to 24 hours **(****Figure 6** A, left upper panel right), reflecting the accumulation of this protein over time in cells expressing the construct. However, mNeonGreen fluorescence remained comparably low at both time points prior to iron treatment, increasing significantly only upon FeSO₄/Vitamin C addition **(****Figure 6** A, left upper panel left).

Additionally, no correlation was found between the mNeonGreen/mCherry ratio and the absolute expression levels, regardless of iron treatment (Figure 13 C, upper panels for untreated; lower panels for FeSO₄/Vitamin C treated). This suggested that the pseudo-ratiometric sensitivity of Module 1 - Design 2 was reliable across a range of expression levels and times post-transfection.

Overall, these experiments demonstrated that transient transfection of the exemplarily investigated Module 1 - Design 2 provides a substantial operational window, from at least 24 to 42 hours post-transfection, within which the system remains responsive to cellular iron changes and effectively visualizes alterations in the labile iron pool (LIP).

### Long-term monitoring of Module 1 - Design 2 revealed heterogeneous LIP responses in single cells

Long-term live-cell imaging was conducted to monitor the green/red ratiometric Module 1 - Design 2 in individual HeLa cells over an 8-hour period, capturing images every 3 minutes for both mNeonGreen and mCherry fluorescence channels. Just before initiating this time-lapse experiment, cells were treated with FeSO₄ and Vitamin C to elevate the labile iron pool (LIP). **Figure 7** presents three distinct response patterns to highlight the heterogeneity of cellular reactions to iron treatment:

In Cell 1, an immediate, continuous increase in the green/red fluorescence ratio was observed following Fe(ll) treatment, suggesting a progressive buildup of the LIP over 4 hours. After this period, the cell likely was affected by iron toxicity and died, indicated by an abrupt cessation of further ratio changes. Cell 2, by contrast, displayed no significant change in the green/red ratio throughout the experiment and remained viable, suggesting that Fe(ll) treatment did not induce a substantial increase in the LIP for this cell. Cell 3 exhibited an initial increase in the green/red ratio upon iron addition, followed by a gradual return to baseline levels after about 3 hours. This cell appeared to respond by first increasing the LIP, then successfully regulating and reducing it back to starting levels.

These results demonstrated the utility of the pseudo-ratiometric Module 1 Design 2 for visualizing dynamic, cell-specific fluctuations in the LIP, enabling real-time insights into individual cellular responses to iron treatment

### Module 2

Module 2 represents a genetically encoded construct that can translate a cellular Fe²⁺ status into a ratiometric reporter protein domain signal, preferably a ratiometric fluorescence signal. For example, using a genetic construct encoding, *inter alia,* two fluorescent protein domains like mCherry and mNeonGreen, a ratio can be obtained from the two fluorescent signals like *F*_{mcherry} and *F*_{mNeonGreen}. For example, a ratio of *F*_{mCherry} and *F*_{mNeonGreen}, denoted as *F*_{mCherry}/*F*_{mNeonGreen}, can reflect cellular Fe²⁺ homeostasis based on endogenously iron-responsive proteins **(****Figure 8** A); a high ratio suggests ferritin-based cellular iron storage, while a low ratio implies transferrin receptor (TfR)-based active cellular iron uptake. To translate the iron status of living cells into a (optical) ratiometric readout, genetically encoded biosensor, also referred to as rFeRepS, constructs were developed. Module 2 can convert alterations in cellular iron status into a detectable ratiometric signal by the opposing expression changes in at least two distinct proteins that represent or generate two distinct signals - such as distinct fluorescent protein variants of different colours **(****Figure 8****).** The rFeRepS displayed the elevation of one signal concomitant with heightened iron supply while simultaneously suppressing another signal **(****Figure 8****).** Conversely, in conditions of cellular iron deficiency, the inverse occurred: the suppressed signal ascended while the initially elevated signal diminishes.

To enable expression of mCherry and mNeonGreen as reporter protein domains based on cellular iron status, a modified PcDNA3.1- vector was exemplary designed and constructed **(****Figure 8** B). The vector allowed for an independent expression of each fluorescent protein domain in response to iron levels within the cell. In this example, mCherry expression mimicked the expression pattern of Fr, increasing when cellular iron levels were high, while mNeonGreen expression reflected increasing levels of TfR when iron levels were low. In this example, the CMV promoter in the standard PcDNA3.1- vector was replaced with the moderate-strength human phosphoglycerate kinase (hPGK) promoter to drive mCherry mRNA expression. The lower expression level (compared to the CMV promoter in the standard vector) helped to maintain endogenous regulation by Aco1 and Aco2 and thus, was advantageous for the mCherry signal to reflect Fr expression. When Aco1 and Aco2 detect high iron levels, they are believed to dissociate from mRNAs, enabling translation, e.g., of the red fluorescent protein domain. This resulted in a signal from a construct referred to as "Hi FeRep" in Figure 8 B). To terminate mCherry expression and enable independent expression of a second mRNA, a polyA tail as well as terminator signals from the original PcDNA3.1 vector were inserted at the end of Hi FeRep, i.e. 5' of the sequence encoding the Hi FeRep construct.

In this example, the signal from a construct referred to as "Lo FeRep" in Figure 8 B) was controlled by an 8xHRE (hypoxia-responsive elements) promoter region obtained from the TfR gene, which is sensitive to low iron levels. The choice of this promoter resulted in mNeonGreen expression when cellular iron was low, reflecting a need for increased TfR expression to enhance iron uptake from the extracellular space. Additionally, stability of the mNeonGreen mRNA was regulated by five IRE repeats in the 3' UTR, which controlled translation under low iron conditions. Together, the iron-sensitive 8xHRE promoter and the IRE repeats in the 3' UTR ensured in this example that mNeonGreen expression was activated when iron levels were falling, thus indicating that the cell required more cellular iron.

### Overview of experiments performed with Module 2

A series of experiments was conducted to assess the response of each of the exemplarily tested constructs, namely Hi FeRep, Lo FeRep, and rFeRepS, in HeLa and EA.hy926 cells (Table 2). For this purpose, vectors specifically coding for each construct were utilized, alongside control constructs that lack iron-responsive elements and that were driven by a hPGK promoter only. This approach allowed for an independent evaluation of the performance of the Hi FeRep, Lo FeRep, and rFeRepS constructs under different cellular iron conditions. To manipulate cellular iron status, treatments were applied that were designed to either increase or decrease intracellular iron levels, including Fe²⁺ combined with Vitamin C, iron sucrose (IS), and ferric carboxymaltose (FCM) nanoparticles to raise iron content, and eltrombopag (ELT), an iron chelator, to lower intracellular iron availability. An overview and summary of the results from these experiments, highlighting findings for each individual FeRep construct, are provided in Table 2.

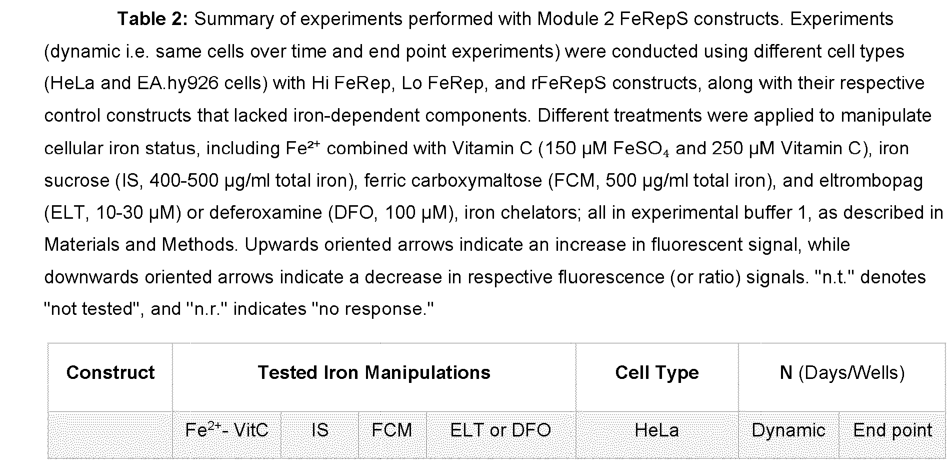

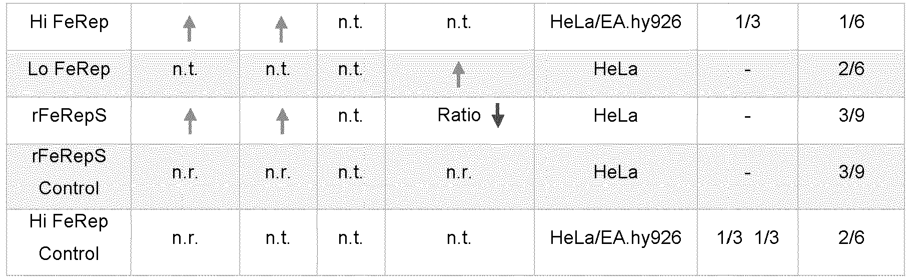

### Module 2 Hi FeRep constructs dynamically reported changes in cellular iron content

To investigate the dynamic response of the Hi FeRep construct to changes in cellular iron levels, a series of experiments was performed in HeLa cells, comparing the Hi FeRep **(****Figure 9****,** left panel) with the iron-insensitive control construct (Hi FeRep-IRE, **Figure 9****,** right panel). Fluorescence intensity in cells expressing each construct was monitored over time under three different treatment conditions to evaluate iron-responsive expression kinetics.

In the first condition, cells were treated with Fe²⁺ combined with Vitamin C to elevate intracellular iron levels and assess the construct's response to iron enrichment. Cells exposed to Fe²⁺/Vitamin C developed Hi FeRep fluorescence intensity at a faster rate than untreated controls and DFO-treated cells **(****Figure** 9, left panel), indicating that Hi FeRep expression was responsive to elevated iron levels. In contrast, cells expressing the iron-insensitive construct (Hi FeRep-IRE) showed no significant changes in fluorescence intensity or expression kinetics across treatments, demonstrating its lack of responsiveness to iron modulation **(****Figure 9****,** right panel).

In the second condition, cells were initially treated with DFO to deplete cellular iron levels; 24 hours later, the media was replaced with Fe²⁺ and Vitamin C, allowing us to observe the construct's response to iron reintroduction following an iron-depleted state. Cells treated with DFO showed a much slower increase in mCherry fluorescence over time compared to control cells and cells treated with the Fe²⁺ salt. Re-addition of iron slightly restored fluorescent intensity **(****Figure 9****,** left panel).

In the third condition, untreated control cells were imaged over time to get a baseline fluorescence intensity for comparison **(****Figure 9****).**

### ELT treatment significantly increases the number of cells expressing Lo FeRep t

To assess the performance of the Lo FeRep construct, the construct was expressed in HeLa cells treated with ELT **(****Figure 10****).** Under control conditions, only 6.8% of cells showed a detectable Lo FeRep signal, indicating that a small proportion of HeLa cells were experiencing iron deficiency **(****Figure 10** B). However, in response to ELT, a significant increase in the number of cells positive for mNeonGreen was observed **(****Figure 10** A and B). The results suggested that the increase in Lo FeRep signal observed at the 24-hour endpoint likely represented an underestimation of iron deficiency. The cytotoxic effect of ELT may have led to the loss of Lo FeRep-positive cells, particularly those suffering from severe iron deficiency due to ELT treatment.

### Assessment of cellular iron status using rFeRepS in HeLa cells

To explore the iron-sensing capability of rFeRepS, a construct designed to express mCherry and mNeonGreen in an iron-dependent manner **(****Figure 8** B), HeLa cells were transiently transfected therewith and fluorescence signals post-transfection were analyzed. Ratiometric images were obtained based on mCherry/mNeonGreen fluorescence intensity **(****Figure 11** A, right image). Across multiple imaging areas, a heterogeneous population of cells was observed: some displayed a low mCherry/mNeonGreen ratio, indicative of low mCherry but high mNeonGreen fluorescence, while others showed a high mCherry/mNeonGreen ratio, with high mCherry and low mNeonGreen fluorescence. According to the rFeRepS functionality, a low red/green fluorescence ratio corresponded to low intracellular iron supply, while a high ratio suggests higher, potentially excessive iron accumulation.

As a control, both fluorescent proteins were expressed in HeLa cells without iron-responsive elements but under the same promoter strength. In this setup, all cells exhibited a consistent red/green ratio **(****Figure 11** B and C), confirming that variability in fluorescence ratios observed with rFeRepS was due to differences in cellular iron levels rather than expression variability.

Based on these findings, it was hypothesized that rFeRepS reliably indicated cellular iron status. For further validation, the transfected cells were treated with Fe(ll) sulfate/Vitamin C, iron sucrose nanoparticles (IS), and eltrombopag (ELT), an iron chelator, to assess whether these manipulations would shift the fluorescence ratios in accordance with altered cellular iron levels.

To examine the distribution of the mCherry/mNeonGreen ratio within the HeLa cell population expressing rFeRepS and investigate any potential correlation with expression levels, the red/green ratio values (x-axis) were plotted against the total fluorescence intensity (summed mCherry and mNeonGreen fluorescence per cell) in an XY scatter plot under control (untreated) conditions **(****Figure 11** A, left upper panel, B and C). This analysis revealed substantial heterogeneity in red/green ratio values across the cell population, with no clear correlation between the ratio values and overall expression levels. Cells with both high and low ratio values were observed, indicative of varying iron states, regardless of expression level. This suggested that cells with either low or high iron supply were present across a range of expression intensities.

Most cells showed a ratio between 0.5 and 5.0, although a subset exhibited ratios below 0.5 or above 5.0, even under control conditions **(****Figure 11** A, left upper panel). It was hypothesized that cells with ratios below 0.5 were iron-deficient, while those with ratios above 5.0 may have experienced iron overload. Within the 0.5 to 5.0 range, considerable heterogeneity was observed, suggesting that some of these cells may have had a balanced iron status, while others might have experienced either iron deficiency or iron excess.

Next, it was explored how individual cells of the cell population responded to iron loading via Fe(ll) sulfate and Vitamin C treatment. As shown in **Figure 12** A (right upper panel, B and C), this treatment did not markedly alter the ratio distribution across the population. While a modest increase in the number of cells with ratios around 5.0 was observed, cells with extremely high ratios were largely absent, possibly due to the cytotoxicity of excess iron, which may trigger ferroptosis in iron-overloaded cells. Interestingly, the population of cells with ratios below 0.5 remained largely unaffected by Fe(ll) treatment, suggesting that certain cells continued to display signs of limited iron uptake despite an increased extracellular iron availability. This observation raised the possibility that the HeLa cells may have had a limited capacity to take up Fe(ll) through the plasma membrane, potentially due to varying levels of DMT1, a key ion channel involved in Fe(ll) uptake across the plasma membrane.

To probe the module's sensitivity to iron depletion, rFeRepS-expressing HeLa cells were treated with ELT and the resulting mCherry/mNeonGreen ratio values were plotted against total fluorescence intensity **(****Figure 12** A and B, lower left panel, and C). ELT treatment led to a marked reduction in the number of cells with ratio values around or above 5.0, while significantly increasing the proportion of cells with low ratios **(****Figure 12** A and B, lower left panel, and C). These findings demonstrated that rFeRepS effectively visualized shifts toward iron deficiency in response to iron chelation by ELT. Furthermore, these experiments suggested that ELT treatment in HeLa cells may have lowered ISC synthesis and likely upregulated TfR expression - both signs of cellular iron "hunger" as cancer cells adjust to iron depletion. In contrast, treatment with carbohydrate-based iron sucrose nanoparticles (IS) effectively increased the proportion of cells with a higher mCherry/mNeonGreen ratio around or above 5.0, while simultaneously reducing the number of cells with a low ratio below 0.5 (**Figure 12** A, right lower panel, B and C). Unlike treatment with Fe(II) salts, which had a limited impact on alleviating iron deficiency, IS nanoparticle treatment appeared to be more effective at increasing intracellular iron levels in HeLa cells. These results suggested that rFeRepS can distinguish the relative efficacy of iron sources *in vitro,* indicating that IS nanoparticles were more readily taken up by HeLa cells and led to a more robust increase in iron availability than Fe(ll) salts.

To further validate that the observed changes in mCherry/mNeonGreen ratios were a result of alterations in cellular iron status, cells expressing the control construct were also treated with the same substances. As shown in **Figure 13****,** the ratio values remained consistent at around 1 across all treatments, suggesting that the changes in the rFeRepS-expressing cells were specifically related to iron modulation, and not due to any non-specific effects on fluorescence or expression levels.

### Sequences

### Sequences referred to herein are listed in the following (Table 3):

**Table 3: Sequences (column 2) listed according to their assigned SEQ ID NO (column 1) with a brief description (columns 3 and 4).**

| **NO** | **Sequence** | **Type** | **Description** |
|---|---|---|---|
| **1** | GGGTTGCGCCTTTTCCAAGGCAGCCCTGGGTTTGCGCAGGGAC GCGGCTGCTCTGGGCGTGGTTCCGGGAAACGCAGCGGCGCCG ACCCTGGGTCTCGCACATTCTTCACGTCCGTTCGCAGCGTCACC CGGATCTTCGCCGCTACCCTTGTGGGCCCCCCGGCGACGCTTC CTGCTCCGCCCCTAAGTCGGGAAGGTTCCTTGCGGTTCGCGGC GTGCCGGACGTGACAAACGGAAGCCGCACGTCTCACTAGTACCC TCGCAGACGGACAGCGCCAGGGAGCAATGGCAGCGCGCCGAC CGCGATGGGCTGTGGCCAATAGCGGCTGCTCAGCAGGGCGCGC CGAGAGCAGCGGCCGGGAAGGGGCGGTGCGGGAGGCGGGGT GTGGGGCGGTAGTGTGGGCCCTGTTCCTGCCCGCGCGGTGTTC CGCATTCTGCAAGCCTCCGGAGCGCACGTCGGCAGTCGGCTCC CTCGTTGACCGAATCACCGACCTCTCTCCCCAGG | nt | hPGK |
| **2** | XDVEXNPGP | aa | ribosome skipping region; conserved core sequence; X = any amino acid or absent |
| **3** | GSGXXXXXXXXLLXXXXDVEXNPGP | aa | ribosome skipping region; conserved sequence; X = any amino acid or absent |
| **4** | GSGEGRGSLLTCGDVEENPGP | aa | ribosome skipping region; full |
| **5** | GGAAGCGGAGAGGGCAGGGGAAGTCTTCTAACATGCGGGGACG TGGAGGAAAATCCCGGCCCC | nt | |
| **6** | TIYNVHSDNK | aa | degron sequence |
| **7** | ACCATTTATAATGTACATTCTGACAATAAA | nt | |
| **8** | MAPFPEEVDVFTAPHWRMKQLVGLYCDKLSKTNFSNNNDFRALLQ SLYATFKEFKMHEQIENEYIIGLLQQRSQTIYNVHSDNKLSEMLSLFE KGLKNVKNEYEQLNYAKQLKERLEAFTRDFLPHMKEEEEVFQPMLM EYFTYEELKDIKKKVIAQHCS | aa | Hr domain |
| **9** | ATGGCGCCCTTTCCTGAAGAAGTGGACGTCTTCACCGCCCCACA CTGGCGGATGAAGCAGCTGGTGGGGCTCTACTGCGACAAGCTTT CTAAAACCAATTTTTCCAACAACAACGATTTCCGTGCTCTTCTGCA GTCTTTGTATGCTACTTTCAAGGAGTTCAAAATGCATGAGCAGATT GAAAATGAATACATTATTGGTTTGCTTCAACAACGCAGCCAGACCA TTTATAATGTACATTCTGACAATAAACTCTCCGAGATGCTTAGCCTC TTTGAAAAGGGACTGAAGAATGTTAAGAATGAATATGAACAGTTAA ATTATGCAAAACAACTGAAAGAGAGATTGGAGGCTTTTACAAGAG ATTTTCTTCCTCACATGAAAGAGGAAGAGGAGGTTTTTCAGCCCA TGTTAATGGAATATTTTACCTATGAAGAGCTTAAGGATATTAAAAAG AAAGTGATTGCACAACACTGCTCT | nt | |
| **10** | MVSKGEELIKENMHMKLYMEGTVDNHHFKCTSEGEGKPYEGTQTM RIKVVEGGPLPFAFDILATSFLYGSKTFINHTQGIPDFFKQSFPEGFT WERVTTYEDGGVLTATQDTSLQDGCLIYNVKIRGVNFTSNGPVMQK KTLGWEAFTETLYPADGGLEGRNDMALKLVGGSHLIANAKTTYRSK KPAKNLKMPGVYYVDYRLERIKEANNETYVEQHEVAVARYCDLPSKL GHKLN | aa | mTagBFP2 |
| **11** | ATGGTGTCTAAGGGCGAAGAGCTGATTAAGGAGAACATGCACATG AAGCTGTACATGGAGGGCACCGTGGACAACCATCACTTCAAGTG CACATCCGAGGGCGAAGGCAAGCCCTACGAGGGCACCCAGACC ATGAGAATCAAGGTGGTCGAGGGCGGCCCTCTCCCCTTCGCCTT CGACATCCTGGCTACTAGCTTCCTCTACGGCAGCAAGACCTTCAT CAACCACACCCAGGGCATCCCCGACTTCTTCAAGCAGTCCTTCC CTGAGGGCTTCACATGGGAGAGAGTCACCACATACGAAGACGGG GGCGTGCTGACCGCTACCCAGGACACCAGCCTCCAGGACGGCT GCCTCATCTACAACGTCAAGATCAGAGGGGTGAACTTCACATCCA ACGGCCCTGTGATGCAGAAGAAAACACTCGGCTGGGAGGCCTT CACCGAGACGCTGTACCCCGCTGACGGCGGCCTGGAAGGCAGA AACGACATGGCCCTGAAGCTCGTGGGCGGGAGCCATCTGATCG CAAACGCCAAGACCACATATAGATCCAAGAAACCCGCTAAGAACC TCAAGATGCCTGGCGTCTACTATGTGGACTACAGACTGGAAAGAA TCAAGGAGGCCAACAACGAGACCTACGTCGAGCAGCACGAGGT GGCAGTGGCCAGATACTGCGACCTCCCTAGCAAACTGGGGCACA AGCTTAATTAA | nt | |
| **12** | MVSKGEEDNMASLPATHELHIFGSINGVDFDMVGQGTGNPNDGYE ELNLKSTKGDLQFSPWILVPHIGYGFHQYLPYPDGMSPFQAAMVDG SGYQVHRTMQFEDGASLTVNYRYTYEGSHIKGEAQVKGTGFPADG PVMTNSLTAADWCRSKKTYPNDKTIISTFKWSYTTGNGKRYRSTAR TTYTFAKPMAANYLKNQPMYVFRKTELKHSKTELNFKEWQKAFTDV MGMDELYK | aa | mNeonGreen |
| **13** | ATGGTGAGTAAGGGGGAGGAAGATAACATGGCCTCACTTCCAGC GACTCACGAACTTCACATTTTTGGGTCCATCAATGGCGTGGATTT CGATATGGTAGGGCAAGGGACTGGCAACCCTAACGACGGTTACG AAGAACTCAATCTGAAATCAACGAAAGGGGATTTGCAATTTTCCC CATGGATACTCGTCCCCCATATAGGCTATGGATTCCACCAATATCT GCCGTATCCAGACGGAATGTCTCCGTTTCAAGCCGCAATGGTAGA CGGTAGCGGTTACCAGGTCCACCGGACGATGCAATTCGAAGATG GGGCCTCCCTGACAGTAAATTATAGATATACCTATGAAGGGAGTCA CATAAAGGGAGAAGCTCAGGTCAAAGGAACGGGTTTCCCGGCTG ACGGACCGGTGATGACCAATTCACTGACCGCCGCCGATTGGTGC CGCTCTAAAAAAACTTACCCAAATGATAAGACTATCATTTCAACTTT TAAATGGAGTTACACTACGGGGAATGGTAAGAGGTACAGGTCAAC | nt | |
| | AGCCAGGACAACTTACACATTTGCCAAGCCAATGGCGGCGAACT ATTTGAAAAATCAGCCAATGTACGTTTTCCGAAAAACCGAATTGAA ACATTCTAAGACCGAGCTTAATTTCAAGGAATGGCAAAAAGCATTT ACGGATGTGATGGGGATGGATGAGCTTTACAAG | | |
| **14** | MVSKGEEDNMAIIKEFMRFKVHMEGSVNGHEFEIEGEGEGRPYEG TQTAKLKVTKGGPLPFAWDILSPQFMYGSKAYVKHPADIPDYLKLSF PEGFKWERVMNFEDGGVVTVTQDSSLQDGEFIYKVKLRGTNFPSD GPVMQKKTMGWEASSERMYPEDGALKGEIKQRLKLKDGGHYDAE VKTTYKAKKPVQLPGAYNVNIKLDITSHNEDYTIVEQYERAEGRHST GGMDELYK | aa | mCherry |
| **15** | ATGGTCTCCAAAGGAGAGGAAGACAATATGGCCATTATCAAAGAG TTTATGCGATTTAAAGTGCACATGGAGGGAAGCGTCAACGGGCAT GAGTTTGAGATTGAGGGGGAAGGAGAGGGAAGACCTTATGAAGG GACCCAGACTGCCAAACTGAAAGTCACCAAGGGCGGACCCCTG CCATTTGCTTGGGACATTCTGAGCCCACAATTCATGTATGGGTCC AAAGCCTATGTTAAACACCCGGCAGACATACCTGACTACCTCAAG CTCTCCTTTCCTGAGGGGTTTAAATGGGAGAGGGTGATGAACTTT GAAGATGGAGGCGTCGTGACAGTAACTCAGGATAGCAGCCTGCA AGACGGGGAGTTTATTTACAAGGTTAAATTGCGCGGAACCAACTT TCCCAGCGATGGACCCGTGATGCAGAAGAAAACAATGGGCTGGG AAGCATCATCCGAAAGAATGTATCCCGAAGATGGCGCGTTGAAGG GGGAAATCAAACAAAGGCTCAAGTTGAAAGATGGAGGGCACTAT GACGCTGAGGTCAAAACCACGTACAAGGCTAAAAAACCGGTACA ACTGCCCGGAGCATATAATGTTAATATTAAGCTGGACATCACCAGC CACAATGAGGATTACACAATAGTGGAACAGTATGAAAGAGCCGAG GGAAGACACTCAACTGGGGGCATGGATGAACTGTATAAATGATAA | nt | |
| **16** | MGAQLTTFALAGAVRRMNTFQDQSGSSSNREPLLRCSDARRDLEL AIGGVLRAEQQIKDNLREVKAQIHSCISRHLECLRSREVWLYEQVDLI YQLKEETLQQQAQQLYSLLGQFNCLTHQLECTQNKDLANQVSVCLE RLGSLTLKPEDSTVLLFEADTITLRQTITTFGSLKTIQIPEHLMAHASS ANIGPFLEKRGCISMPEQKSASGIVAVPFSEWLLGSKPASGYQAPYI PSTDPQDWLTQKQTLENSQTSSRACNFFNNVGGNLKGLENWLLKS EKSSYQKCNSHSTTSSFSIEMEKVGDQELPDQDEMDLSDWLVTPQ ESHKLRKPENGSRETSEKFKLLFQSYNVNDWLVKTDSCTNCQGNQ PKGVEIENLGNLKCLNDHLEAKKPLSTPSMVTEDWLVQNHQDPCKV EEVCRANEPCTSFAECVCDENCEKEALYKWLLKKEGKDKNGMPVE PKPEPEKHKDSLNMWLCPRKEVIEQTKAPKAMTPSRIADSFQVIKNS PLSEWLIRPPYKEGSPKEVPGTEDRAGKQKFKSPMNTSWCSFNTA DWVLPGKKMGNLSQLSSGEDKWLLRKKAQEVLLNSPLQEEHNFPP DHYGLPAVCDLFACMQLKVDKEKWLYRTPLQAYFKMNFQDVTVGN FQIPCGF | aa | NCOA4 |
| **17** | ATGGGGGCCCAGTTGACCACTTTTGCTCTAGCTGGAGCAGTGAG GAGAATGAATACCTTCCAAGACCAGAGTGGCAGCTCCAGTAATAG AGAACCCCTTTTGAGGTGTAGTGATGCACGGAGGGACTTGGAGC TTGCTATTGGTGGAGTTCTCCGGGCTGAACAGCAAATTAAAGATA ACTTGCGAGAGGTCAAAGCTCAGATTCACAGTTGCATAAGCCGTC ACCTGGAATGTCTTAGAAGCCGTGAGGTATGGCTGTATGAACAGG TGGACCTTATTTATCAGCTTAAAGAGGAGACACTTCAACAGCAGG CTCAGCAGCTCTACTCGTTATTGGGCCAGTTCAATTGTCTTACTCA TCAACTGGAGTGTACCCAAAACAAAGATCTAGCCAATCAAGTCTC TGTGTGCCTGGAGAGACTGGGCAGTTTGACCCTTAAGCCTGAAG ATTCAACTGTCCTGCTCTTTGAAGCTGACACAATTACTCTGCGCC AGACCATCACCACATTTGGGTCTCTCAAAACCATTCAAATTCCTGA GCACTTGATGGCTCATGCTAGTTCAGCAAATATTGGGCCCTTCCT GGAGAAGAGAGGCTGTATCTCCATGCCAGAGCAGAAGTCAGCAT CCGGTATTGTAGCTGTCCCTTTCAGCGAATGGCTCCTTGGAAGCA AACCTGCCAGTGGTTATCAAGCTCCTTACATACCCAGCACCGACC CCCAGGACTGGCTTACCCAAAAGCAGACCTTGGAGAACAGTCAG | nt | |
| | ACTTCTTCCAGAGCCTGCAATTTCTTCAATAATGTCGGGGGAAAC CTAAAGGGCTTAGAAAACTGGCTCCTCAAGAGTGAAAAATCAAGT TATCAAAAGTGTAACAGCCATTCCACTACTAGTTCTTTCTCCATTG AAATGGAAAAGGTTGGAGATCAAGAGCTTCCTGATCAAGATGAGA TGGACCTATCAGATTGGCTAGTGACTCCCCAGGAATCCCATAAGC TGCGGAAGCCTGAGAATGGCAGTCGTGAAACCAGTGAGAAGTTT AAGCTCTTATTCCAGTCCTATAATGTGAATGATTGGCTTGTCAAGA CTGACTCCTGTACCAACTGTCAGGGAAACCAGCCCAAAGGTGTG GAGATTGAAAACCTGGGCAATCTGAAGTGCCTGAATGACCACTTG GAGGCCAAGAAACCATTGTCCACCCCCAGCATGGTTACAGAGGA TTGGCTTGTCCAGAACCATCAGGACCCATGTAAGGTAGAGGAGG TGTGCAGAGCCAATGAGCCCTGCACAAGCTTTGCAGAGTGTGTG TGTGATGAGAATTGTGAGAAGGAGGCTCTGTATAAGTGGCTTCTG AAGAAAGAAGGAAAGGATAAAAATGGGATGCCTGTGGAACCCAA ACCTGAGCCTGAGAAGCATAAAGATTCCCTGAATATGTGGCTCTG TCCTAGAAAAGAAGTAATAGAACAAACTAAAGCACCAAAGGCAAT GACTCCTTCTAGAATTGCTGATTCCTTCCAAGTCATAAAGAACAGC CCCTTGTCGGAGTGGCTTATCAGGCCCCCATACAAAGAAGGAAG TCCCAAGGAAGTGCCTGGTACTGAAGACAGAGCTGGCAAACAGA AGTTTAAAAGCCCCATGAATACTTCCTGGTGTTCCTTTAACACAGC TGACTGGGTCCTGCCAGGAAAGAAGATGGGCAACCTCAGCCAGT TATCTTCTGGAGAAGACAAGTGGCTGCTTCGAAAGAAGGCCCAG GAAGTATTACTTAATTCACCTCTACAGGAGGAACATAACTTCCCCC CAGACCATTATGGCCTCCCTGCAGTTTGTGATCTCTTTGCCTGTAT GCAGCTTAAAGTTGATAAAGAGAAGTGGTTATATCGAACTCCTCTA CAGGCATACTTCAAAATGAACTTTCAAGATGTAACCGTTGGGAATT TTCAGATCCCATGTGGATTCTAG | | |
| **18** | caactttgtatagaaaagttggggttgcgccttttccaaggcagccctgggtttgcgcagggacg cggctgctctgggcgtggttccgggaaacgcagcggcgccgaccctgggtctcgcacattcttc acgtccgttcgcagcgtcacccggatcttcgccgctacccttgtgggccccccggcgacgcttcc tgctccgcccctaagtcgggaaggttccttgcggttcgcggcgtgccggacgtgacaaacgga agccgcacgtctcactagtaccctcgcagacggacagcgccagggagcaatggcagcgcgc cgaccgcgatgggctgtggccaatagcggctgctcagcagggcgcgccgagagcagcggc cgggaaggggcggtgcgggaggcggggtgtggggcggtagtgtgggccctgttcctgcccgc gcggtgttccgcattctgcaagcctccggagcgcacgtcggcagtcggctccctcgttgaccga atcaccgacctctctccccagggctagcgccaccatggcgccctttcctgaagaagtggacgtc ttcaccgccccacactggcggatgaagcagctggtggggctctactgcgacaagctttctaaaa ccaatttttccaacaacaacgatttccgtgctcttctgcagtctttgtatgctactttcaaggagttcaa aatgcatgagcagattgaaaatgaatacattattggtttgcttcaacaacgcagccagaccattta taatgtacattctgacaataaactctccgagatgcttagcctctttgaaaagggactgaagaatgtt aagaatgaatatgaacagttaaattatgcaaaacaactgaaagagagattggaggcttttacaa gagattttcttcctcacatgaaagaggaagaggaggtttttcagcccatgttaatggaatattttacc tatgaagagcttaaggatattaaaaagaaagtgattgcacaacactgctctggcggcggctcta gagtgtctaagggcgaagagctgattaaggagaacatgcacatgaagctgtacatggagggc accgtggacaaccatcacttcaagtgcacatccgagggcgaaggcaagccctacgagggca cccagaccatgagaatcaaggtggtcgagggcggccctctccccttcgccttcgacatcctggc tactagcttcctctacggcagcaagaccttcatcaaccacacccagggcatccccgacttcttca agcagtccttccctgagggcttcacatgggagagagtcaccacatacgaagacgggggcgtg ctgaccgctacccaggacaccagcctccaggacggctgcctcatctacaacgtcaagatcag aggggtgaacttcacatccaacggccctgtgatgcagaagaaaacactcggctgggaggcctt caccgagacgctgtaccccgctgacggcggcctggaaggcagaaacgacatggccctgaa gctcgtgggcgggagccatctgatcgcaaacgccaagaccacatatagatccaagaaaccc gctaagaacctcaagatgcctggcgtctactatgtggactacagactggaaagaatcaaggag gccaacaacgagacctacgtcgagcagcacgaggtggcagtggccagatactgcgacctcc ctagcaaactggggcacaagcttaattaagaattcacccagctttcttgtacaaagtggtgatgg ccggccgcttcgagcagacatgataagatacattgatgagtttggacaaaccacaactagaat gcagtgaaaaaaatgctttatttgtgaaatttgtgatgctattgctttatttgtaaccattataagctgc aataaacaagttaacaacaacaattgcattcattttatgtttcaggttcagggggaggtgtgggag gttttttaaagcaagtaaaacctctacaaatgtggtagcggccgcggcgctcttccgcttcctcgct cactgactcgctgcgctcggtcgttcggctgcggcgagcggtatcagctcactcaaaggcggta | nt | Module 1 - Design 1 full construct (exemplary plasmid sequence) |
| | atacggttatccacagaatcaggggataacgcaggaaagaacatgtgagcaaaaggccagc aaaaggccaggaaccgtaaaaaggccgcgttgctggcgtttttccataggctccgcccccctg acgagcatcacaaaaatcgacgctcaagtcagaggtggcgaaacccgacaggactataaa gataccaggcgtttccccctggaagctccctcgtgcgctctcctgttccgaccctgccgcttaccg gatacctgtccgcctttctcccttcgggaagcgtggcgctttctcatagctcacgctgtaggtatctca gttcggtgtaggtcgttcgctccaagctgggctgtgtgcacgaaccccccgttcagcccgaccgc tgcgccttatccggtaactatcgtcttgagtccaacccggtaagacacgacttatcgccactggca gcagccactggtaacaggattagcagagcgaggtatgtaggcggtgctacagagttcttgaagt ggtggcctaactacggctacactagaagaacagtatttggtatctgcgctctgctgaagccagtt accttcggaaaaagagttggtagctcttgatccggcaaacaaaccaccgctggtagcggtggtt tttttgtttgcaagcagcagattacgcgcagaaaaaaaggatctcaagaagatcctttgatcttttct acggggtctgacgctcagtggaacgaaaactcacgttaagggattttggtcatgagattatcaaa aaggatcttcacctagatccttttaaattaaaaatgaagttttaaatcaatctaaagtatatatgagt aaacttggtctgacagttaccaatgcttaatcagtgaggcacctatctcagcgatctgtctatttcgtt catccatagttgcctgactccccgtcgtgtagataactacgatacgggagggcttaccatctggc cccagtgctgcaatgataccgcgagacccacgctcaccggctccagatttatcagcaataaac cagccagccggaagggccgagcgcagaagtggtcctgcaactttatccgcctccatccagtct attaattgttgccgggaagctagagtaagtagttcgccagttaatagtttgcgcaacgttgttgccat tgctacaggcatcgtggtgtcacgctcgtcgtttggtatggcttcattcagctccggttcccaacgat caaggcgagttacatgatcccccatgttgtgcaaaaaagcggttagctccttcggtcctccgatc gttgtcagaagtaagttggccgcagtgttatcactcatggttatggcagcactgcataattctcttac tgtcatgccatccgtaagatgcttttctgtgactggtgagtactcaaccaagtcattctgagaatagt gtatgcggcgaccgagttgctcttgcccggcgtcaatacgggataataccgcgccacatagca gaactttaaaagtgctcatcattggaaaacgttcttcggggcgaaaactctcaaggatcttaccg ctgttgagatccagttcgatgtaacccactcgtgcacccaactgatcttcagcatcttttactttcacc agcgtttctgggtgagcaaaaacaggaaggcaaaatgccgcaaaaaagggaataagggcg acacggaaatgttgaatactcatactcttcctttttcaatattattgaagcatttatcagggttattgtct catgagcggatacatatttgaatgtatttagaaaaataaacaaataggggttccgcgcacatttcc ccgaaaagtgccacctgacgtctaagaaaccattattatcatgacattaacctataaaaatagg cgtatcacgaggccctttcgtcggcgcgccgcggccgc | | |
| **19** | caactttgtatagaaaagttgggatccgggttgcgccttttccaaggcagccctgggtttgcgcag ggacgcggctgctctgggcgtggttccgggaaacgcagcggcgccgaccctgggtctcgcac attcttcacgtccgttcgcagcgtcacccggatcttcgccgctacccttgtgggccccccggcgac gcttcctgctccgcccctaagtcgggaaggttccttgcggttcgcggcgtgccggacgtgacaaa cggaagccgcacgtctcactagtaccctcgcagacggacagcgccagggagcaatggcagc gcgccgaccgcgatgggctgtggccaatagcggctgctcagcagggcgcgccgagagcag cggccgggaaggggcggtgcgggaggcggggtgtggggcggtagtgtgggccctgttcctgc ccgcgcggtgttccgcattctgcaagcctccggagcgcacgtcggcagtcggctccctcgttga ccgaatcaccgacctctctccccagggccaccatggcgccctttcctgaagaagtggacgtcttc accgccccacactggcggatgaagcagctggtggggctctactgcgacaagctttctaaaacc aatttttccaacaacaacgatttccgtgctcttctgcagtctttgtatgctactttcaaggagttcaaaa tgcatgagcagattgaaaatgaatacattattggtttgcttcaacaacgcagccagaccatttata atgtacattctgacaataaactctccgagatgcttagcctctttgaaaagggactgaagaatgtta agaatgaatatgaacagttaaattatgcaaaacaactgaaagagagattggaggcttttacaag agattttcttcctcacatgaaagaggaagaggaggtttttcagcccatgttaatggaatattttacct atgaagagcttaaggatattaaaaagaaagtgattgcacaacactgctctggcggcggcgtga gtaagggggaggaagataacatggcctcacttccagcgactcacgaacttcacatttttgggtcc atcaatggcgtggatttcgatatggtagggcaagggactggcaaccctaacgacggttacgaa gaactcaatctgaaatcaacgaaaggggatttgcaattttccccatggatactcgtcccccatata ggctatggattccaccaatatctgccgtatccagacggaatgtctccgtttcaagccgcaatggta gacggtagcggttaccaggtccaccggacgatgcaattcgaagatggggcctccctgacagta aattatagatatacctatgaagggagtcacataaagggagaagctcaggtcaaaggaacggg tttcccggctgacggaccggtgatgaccaattcactgaccgccgccgattggtgccgctctaaaa aaacttacccaaatgataagactatcatttcaacttttaaatggagttacactacggggaatggta agaggtacaggtcaacagccaggacaacttacacatttgccaagccaatggcggcgaactatt tgaaaaatcagccaatgtacgttttccgaaaaaccgaattgaaacattctaagaccgagcttaat ttcaaggaatggcaaaaagcatttacggatgtgatggggatggatgagctttacaagggtaccg gaagcggagagggcaggggaagtcttctaacatgcggggacgtggaggaaaatcccggcc ccatggtctccaaaggagaggaagacaatatggccattatcaaagagtttatgcgatttaaagt gcacatggagggaagcgtcaacgggcatgagtttgagattgagggggaaggagagggaag | nt | Module 1 - Design 2 full construct; pseudo-ratiometric (exemplary plasmid sequence) |
| | accttatgaagggacccagactgccaaactgaaagtcaccaagggcggacccctgccatttg cttgggacattctgagcccacaattcatgtatgggtccaaagcctatgttaaacacccggcagac atacctgactacctcaagctctcctttcctgaggggtttaaatgggagagggtgatgaactttgaa gatggaggcgtcgtgacagtaactcaggatagcagcctgcaagacggggagtttatttacaag gttaaattgcgcggaaccaactttcccagcgatggacccgtgatgcagaagaaaacaatggg ctgggaagcatcatccgaaagaatgtatcccgaagatggcgcgttgaagggggaaatcaaac aaaggctcaagttgaaagatggagggcactatgacgctgaggtcaaaaccacgtacaaggct aaaaaaccggtacaactgcccggagcatataatgttaatattaagctggacatcaccagccac aatgaggattacacaatagtggaacagtatgaaagagccgagggaagacactcaactgggg gcatggatgaactgtataaatgataaacccagctttcttgtacaaagtggtgatggccggccgctt cgagcagacatgataagatacattgatgagtttggacaaaccacaactagaatgcagtgaaa aaaatgctttatttgtgaaatttgtgatgctattgctttatttgtaaccattataagctgcaataaacaa gttaacaacaacaattgcattcattttatgtttcaggttcagggggaggtgtgggaggttttttaaag caagtaaaacctctacaaatgtggtagcggccgcggcgctcttccgcttcctcgctcactgactc gctgcgctcggtcgttcggctgcggcgagcggtatcagctcactcaaaggcggtaatacggttat ccacagaatcaggggataacgcaggaaagaacatgtgagcaaaaggccagcaaaaggcc aggaaccgtaaaaaggccgcgttgctggcgtttttccataggctccgcccccctgacgagcatc acaaaaatcgacgctcaagtcagaggtggcgaaacccgacaggactataaagataccagg cgtttccccctggaagctccctcgtgcgctctcctgttccgaccctgccgcttaccggatacctgtc cgcctttctcccttcgggaagcgtggcgctttctcatagctcacgctgtaggtatctcagttcggtgta ggtcgttcgctccaagctgggctgtgtgcacgaaccccccgttcagcccgaccgctgcgccttat ccggtaactatcgtcttgagtccaacccggtaagacacgacttatcgccactggcagcagccac tggtaacaggattagcagagcgaggtatgtaggcggtgctacagagttcttgaagtggtggcct aactacggctacactagaagaacagtatttggtatctgcgctctgctgaagccagttaccttcgga aaaagagttggtagctcttgatccggcaaacaaaccaccgctggtagcggtggtttttttgtttgca agcagcagattacgcgcagaaaaaaaggatctcaagaagatcctttgatcttttctacggggtct gacgctcagtggaacgaaaactcacgttaagggattttggtcatgagattatcaaaaaggatctt cacctagatccttttaaattaaaaatgaagttttaaatcaatctaaagtatatatgagtaaacttggt ctgacagttaccaatgcttaatcagtgaggcacctatctcagcgatctgtctatttcgttcatccata gttgcctgactccccgtcgtgtagataactacgatacgggagggcttaccatctggccccagtgc tgcaatgataccgcgagacccacgctcaccggctccagatttatcagcaataaaccagccagc cggaagggccgagcgcagaagtggtcctgcaactttatccgcctccatccagtctattaattgttg ccgggaagctagagtaagtagttcgccagttaatagtttgcgcaacgttgttgccattgctacagg catcgtggtgtcacgctcgtcgtttggtatggcttcattcagctccggttcccaacgatcaaggcga gttacatgatcccccatgttgtgcaaaaaagcggttagctccttcggtcctccgatcgttgtcagaa gtaagttggccgcagtgttatcactcatggttatggcagcactgcataattctcttactgtcatgccat ccgtaagatgcttttctgtgactggtgagtactcaaccaagtcattctgagaatagtgtatgcggcg accgagttgctcttgcccggcgtcaatacgggataataccgcgccacatagcagaactttaaaa gtgctcatcattggaaaacgttcttcggggcgaaaactctcaaggatcttaccgctgttgagatcc agttcgatgtaacccactcgtgcacccaactgatcttcagcatcttttactttcaccagcgtttctggg tgagcaaaaacaggaaggcaaaatgccgcaaaaaagggaataagggcgacacggaaat gttgaatactcatactcttcctttttcaatattattgaagcatttatcagggttattgtctcatgagcggat acatatttgaatgtatttagaaaaataaacaaataggggttccgcgcacatttccccgaaaagtg ccacctgacgtctaagaaaccattattatcatgacattaacctataaaaataggcgtatcacgag gccctttcgtcggcgcgccgcggccgc | | |
| **20** | GATCGCCCTACGTGCTGTCTCA | nt | HRE |
| **21** | GATCGCCCTACGTGCTGTCTCAGATCGCCCTACGTGCTGTCTCA GATCGCCCTACGTGCTGTCTCAGATCGCCCTACGTGCTGTCTCA GATCGCCCTACGTGCTGTCTCAGATCGCCCTACGTGCTGTCTCA GATCGCCCTACGTGCTGTCTCAGATCGCCCTACGTGCTGTCTCA | nt | Module 2 second promoter region |
| **22** | TGTCTCTTGCTTCAACAGTGTTTGGACGGAACA | nt | IRE |
| **23** | ATGTGATACCCATAGCTTCCATGAGAACAGCAGGGTAGTCTGGTT TCTAGACTTGTGCTGATCGTGCTAAATTTTCAGTAGGGCTACAAAA CCTGATGTTAAAATTCCATCCCATCATCTTGGTACTACTAGATGTCT TTAGGCAGCAGCTTTTAATACAGGGTAGATAACCTGTACTTCAAGT TAAAGTGAATAACCACTTAAAAAATGTCCATGATGGAATATTCCCCT ATCTCTAGAATTTTAAGTGCTTTGTAATGGGAACTGCCTCTTTCCT GTTGTTGTTAATGAAAATGTCAGAAACCAGTTATGTGAATGATCTC | nt | 3'UTR of TfR including IREs |
| | TCTGAATCCTAAGGGCTGGTCTCTGCTGAAGGTTGTAAGTGGTC GCTTACTTTGAGTGATCCTCCAACTTCATTTGATGCTAAATAGGAG ATACCAGGTTGAAAGACCTTCTCCAAATGAGATCTAAGCCTTTCCA TAAGGAATGTAGCTGGTTTCCTCATTCCTGAAAGAAACAGTTAACT TTCAGAAGAGATGGGCTTGTTTTCTTGCCAATGAGGTCTGAAATG GAGGTCCTTCTGCTGGATAAAATGAGGTTCAACTGTTGATTGCAG GAATAAGGCCTTAATATGTTAACCTCAGTGTCATTTATGAAAAGAG GGGACCAGAAGCCAAAGACTTAGTATATTTTCTTTTCCTCTGTCCC TTCCCCCATAAGCCTCCATTTAGTTCTTTGTTATTTTTGTTTCTTCC AAAGCACATTGAAAGAGAACCAGTTTCAGGTGTTTAGTTGCAGAC TCAGTTTGTCAGACTTTAAAGAATAATATGCTGCCAAATTTTGGCC AAAGTGTTAATCTTAGGGGAGAGCTTTCTGTCCTTTTGGCACTGA GATATTTATTGTTTATTTATCAGTGACAGAGTTCACTATAAATGGTG TTTTTTTAATAGAATATAATTATCGGAAGCAGTGCCTTCCATAATTAT GACAGTTATACTGTCGGTTTTTTTTAAATAAAAGCAGCATCTGCTAA TAAAACCCAACAGATACTGGAAGTTTTGCATTTATGGTCAACACTT AAGGGTTTTAGAAAACAGCCGTCAGCCAAATGTAATTGAATAAAGT TGAAGCTAAGATTTAGAGATGAATTAAATTTAATTAGGGGTTGCTAA GAAGCGAGCACTGACCAGATAAGAATGCTGGTTTTCCTAAATGCA GTGAATTGTGACCAAGTTATAAATCAATGTCACTTAAAGGCTGTGG TAGTACTCCTGCAAAATTTTATAGCTCAGTTTATCCAAGGTGTAACT CTAATTCCCATTTTGCAAAATTTCCAGTACCTTTGTCACAATCCTAA CACATTATCGGGAGCAGTGTCTTCCATAATGTATAAAGAACAAGGT AGTTTTTACCTACCACAGTGTCTGTATCGGAGACAGTGATCTCCAT ATGTTACACTAAGGGTGTAAGTAATTATCGGGAACAGTGTTTCCCA TAATTTTCTTCATGCAATGACA | | |
| **24** | GATCGCCCTACGTGCTGTCTCAGATCGCCCTACGTGCTGTCTCA GATCGCCCTACGTGCTGTCTCAGATCGCCCTACGTGCTGTCTCA GATCGCCCTACGTGCTGTCTCAGATCGCCCTACGTGCTGTCTCA GATCGCCCTACGTGCTGTCTCAGATCGCCCTACGTGCTGTCTCA CAAGTTTGTACAAAAAAGCAGGCTGCCACCgtcgacATGGTGAGTA AGGGGGAGGAAGATAACATGGCCTCACTTCCAGCGACTCACGAA CTTCACATTTTTGGGTCCATCAATGGCGTGGATTTCGATATGGTAG GGCAAGGGACTGGCAACCCTAACGACGGTTACGAAGAACTCAAT CTGAAATCAACGAAAGGGGATTTGCAATTTTCCCCATGGATACTC GTCCCCCATATAGGCTATGGATTCCACCAATATCTGCCGTATCCAG ACGGAATGTCTCCGTTTCAAGCCGCAATGGTAGACGGTAGCGGT TACCAGGTCCACCGGACGATGCAATTCGAAGATGGGGCCTCCCT GACAGTAAATTATAGATATACCTATGAAGGGAGTCACATAAAGGGA GAAGCTCAGGTCAAAGGAACGGGTTTCCCGGCTGACGGACCGG TGATGACCAATTCACTGACCGCCGCCGATTGGTGCCGCTCTAAAA AAACTTACCCAAATGATAAGACTATCATTTCAACTTTTAAATGGAGT TACACTACGGGGAATGGTAAGAGGTACAGGTCAACAGCCAGGAC AACTTACACATTTGCCAAGCCAATGGCGGCGAACTATTTGAAAAAT CAGCCAATGTACGTTTTCCGAAAAACCGAATTGAAACATTCTAAGA CCGAGCTTAATTTCAAGGAATGGCAAAAAGCATTTACGGATGTGA TGGGGATGGATGAGCTTTACAAGTAATAAggtaccATGTGATACCCAT AGCTTCCATGAGAACAGCAGGGTAGTCTGGTTTCTAGACTTGTGC TGATCGTGCTAAATTTTCAGTAGGGCTACAAAACCTGATGTTAAAA TTCCATCCCATCATCTTGGTACTACTAGATGTCTTTAGGCAGCAGC TTTTAATACAGGGTAGATAACCTGTACTTCAAGTTAAAGTGAATAAC CACTTAAAAAATGTCCATGATGGAATATTCCCCTATCTCTAGAATTT TAAGTGCTTTGTAATGGGAACTGCCTCTTTCCTGTTGTTGTTAATG AAAATGTCAGAAACCAGTTATGTGAATGATCTCTCTGAATCCTAAG GGCTGGTCTCTGCTGAAGGTTGTAAGTGGTCGCTTACTTTGAGT GATCCTCCAACTTCATTTGATGCTAAATAGGAGATACCAGGTTGAA AGACCTTCTCCAAATGAGATCTAAGCCTTTCCATAAGGAATGTAGC TGGTTTCCTCATTCCTGAAAGAAACAGTTAACTTTCAGAAGAGAT GGGCTTGTTTTCTTGCCAATGAGGTCTGAAATGGAGGTCCTTCTG CTGGATAAAATGAGGTTCAACTGTTGATTGCAGGAATAAGGCCTT | nt | Module 2 c) and d), incl. 8 HRE, mNeonGreen and 3'UTR of TfR |
| | AATATGTTAACCTCAGTGTCATTTATGAAAAGAGGGGACCAGAAG CCAAAGACTTAGTATATTTTCTTTTCCTCTGTCCCTTCCCCCATAA GCCTCCATTTAGTTCTTTGTTATTTTTGTTTCTTCCAAAGCACATTG AAAGAGAACCAGTTTCAGGTGTTTAGTTGCAGACTCAGTTTGTCA GACTTTAAAGAATAATATGCTGCCAAATTTTGGCCAAAGTGTTAAT CTTAGGGGAGAGCTTTCTGTCCTTTTGGCACTGAGATATTTATTGT TTATTTATCAGTGACAGAGTTCACTATAAATGGTGTTTTTTTAATAG AATATAATTATCGGAAGCAGTGCCTTCCATAATTATGACAGTTATAC TGTCGGTTTTTTTTAAATAAAAGCAGCATCTGCTAATAAAACCCAA CAGATACTGGAAGTTTTGCATTTATGGTCAACACTTAAGGGTTTTA GAAAACAGCCGTCAGCCAAATGTAATTGAATAAAGTTGAAGCTAA GATTTAGAGATGAATTAAATTTAATTAGGGGTTGCTAAGAAGCGAG CACTGACCAGATAAGAATGCTGGTTTTCCTAAATGCAGTGAATTGT GACCAAGTTATAAATCAATGTCACTTAAAGGCTGTGGTAGTACTCC TGCAAAATTTTATAGCTCAGTTTATCCAAGGTGTAACTCTAATTCCC ATTTTGCAAAATTTCCAGTACCTTTGTCACAATCCTAACACATTATC GGGAGCAGTGTCTTCCATAATGTATAAAGAACAAGGTAGTTTTTAC CTACCACAGTGTCTGTATCGGAGACAGTGATCTCCATATGTTACAC TAAGGGTGTAAGTAATTATCGGGAACAGTGTTTCCCATAATTTTCT TCATGCAATGACA | | |
| **25** | CTGTGCCTTCTAGTTGCCAGCCATCTGTTGTTTGCCCCTCCCCCG TGCCTTCCTTGACCCTGGAAGGTGCCACTCCCACTGTCCTTTCC TAATAAAATGAGGAAATTGCATCGCATTGTCTGAGTAGGTGTCATT CTATTCTGGGGGGTGGGGTGGGGCAGGACAGCAAGGGGGAGG ATTGGGAAGACAATAGCAGGCATGCTGGGGATGCGGTGGGCTCT ATGG | nt | BgH polyAtail |
| **26** | GTAGCATAACCCCTTGGGGCCTCTAAACGGGTCTTGAGGGGTTT TTTG | nt | T7 terminator sequence |
| **27** | GCATTGGCGCAGAAAAAAATGCCTGATGCGACGCTGCGCGTCTT ATACTCCCACATATGCCAGATTCAGCAACGGATACGGCTTCCCCA ACTTGCCCACTTCCATACGTGTCCTCCTTACCAGAAATTTATCCTT AA | nt | Rrng terminator sequence |
| **28** | TTGCGCCTTTTCCAAGGCAGCCCTGGGTTTGCGCAGGGACGCG GCTGCTCTGGGCGTGGTTCCGGGAAACGCAGCGGCGCCGACCC TGGGTCTCGCACATTCTTCACGTCCGTTCGCAGCGTCACCCGGA TCTTCGCCGCTACCCTTGTGGGCCCCCCGGCGACGCTTCCTGCT CCGCCCCTAAGTCGGGAAGGTTCCTTGCGGTTCGCGGCGTGCC GGACGTGACAAACGGAAGCCGCACGTCTCACTAGTACCCTCGCA GACGGACAGCGCCAGGGAGCAATGGCAGCGCGCCGACCGCGAT GGGCTGTGGCCAATAGCGGCTGCTCAGCAGGGCGCGCCGAGAG CAGCGGCCGGGAAGGGGCGGTGCGGGAGGCGGGGTGTGGGG CGGTAGTGTGGGCCCTGTTCCTGCCCGCGCGGTGTTCCGCATTC TGCAAGCCTCCGGAGCGCACGTCGGCAGTCGGCTCCCTCGTTG ACCGAATCACCGACCTCTCTCCCCAGGCAAGTTTGTACAAAAAAG CAGGCTGCCACCTGTCTCTTGCTTCAACAGTGTTTGGACGGAAC AgctagcATGGTCTCCAAAGGAGAGGAAGACAATATGGCCATTATCA AAGAGTTTATGCGATTTAAAGTGCACATGGAGGGAAGCGTCAACG GGCATGAGTTTGAGATTGAGGGGGAAGGAGAGGGAAGACCTTAT GAAGGGACCCAGACTGCCAAACTGAAAGTCACCAAGGGCGGAC CCCTGCCATTTGCTTGGGACATTCTGAGCCCACAATTCATGTATG GGTCCAAAGCCTATGTTAAACACCCGGCAGACATACCTGACTACC TCAAGCTCTCCTTTCCTGAGGGGTTTAAATGGGAGAGGGTGATG AACTTTGAAGATGGAGGCGTCGTGACAGTAACTCAGGATAGCAG CCTGCAAGACGGGGAGTTTATTTACAAGGTTAAATTGCGCGGAAC CAACTTTCCCAGCGATGGACCCGTGATGCAGAAGAAAACAATGG GCTGGGAAGCATCATCCGAAAGAATGTATCCCGAAGATGGCGCG TTGAAGGGGGAAATCAAACAAAGGCTCAAGTTGAAAGATGGAGG GCACTATGACGCTGAGGTCAAAACCACGTACAAGGCTAAAAAACC | nt | Module 2 a) and b); incl. hPGK, IRE, mCherry, polyAtail, T7 and Rrng terminator sequences |
| | GGTACAACTGCCCGGAGCATATAATGTTAATATTAAGCTGGACATC ACCAGCCACAATGAGGATTACACAATAGTGGAACAGTATGAAAGA GCCGAGGGAAGACACTCAACTGGGGGCATGGATGAACTGTATAA ATGAgaattcCTGTGCCTTCTAGTTGCCAGCCATCTGTTGTTTGCCC CTCCCCCGTGCCTTCCTTGACCCTGGAAGGTGCCACTCCCACTG TCCTTTCCTAATAAAATGAGGAAATTGCATCGCATTGTCTGAGTAG GTGTCATTCTATTCTGGGGGGTGGGGTGGGGCAGGACAGCAAG GGGGAGGATTGGGAAGACAATAGCAGGCATGCTGGGGATGCGG TGGGCTCTATGGGCATTGGCGCAGAAAAAAACAACAACAATTGCA TTCATTTTATGTTTCAGGTTCAGGGGGAGGTGTGGGAGGTTTTTT AAAGCAAGTAAAACCTCTACAAATGTGGTATTGGCCCATCTCTATC GGTATCGTAGCATAACCCCTTGGGGCCTCTAAACGGGTCTTGAG GGGTTTTTTGTGCCCCTCGGGCCGGATTGCTATCTACCGGCATTG GCGCAGAAAAAAATGCCTGATGCGACGCTGCGCGTCTTATACTCC CACATATGCCAGATTCAGCAACGGATACGGCTTCCCCAACTTGCC CACTTCCATACGTGTCCTCCTTACCAGAAATTTATCCTTAAGGTCG TCAGCTATCCTGCAGGATAGTAATCAATTACGGGGTCATTAGTTCA TAGCCCATATATGGAGTTCCGggatcc | | |
| **29** | GACGGATCGGGAGATCTTTGCGCCTTTTCCAAGGCAGCCCTGGG TTTGCGCAGGGACGCGGCTGCTCTGGGCGTGGTTCCGGGAAAC GCAGCGGCGCCGACCCTGGGTCTCGCACATTCTTCACGTCCGTT CGCAGCGTCACCCGGATCTTCGCCGCTACCCTTGTGGGCCCCC CGGCGACGCTTCCTGCTCCGCCCCTAAGTCGGGAAGGTTCCTTG CGGTTCGCGGCGTGCCGGACGTGACAAACGGAAGCCGCACGTC TCACTAGTACCCTCGCAGACGGACAGCGCCAGGGAGCAATGGCA GCGCGCCGACCGCGATGGGCTGTGGCCAATAGCGGCTGCTCAG CAGGGCGCGCCGAGAGCAGCGGCCGGGAAGGGGCGGTGCGG GAGGCGGGGTGTGGGGCGGTAGTGTGGGCCCTGTTCCTGCCC GCGCGGTGTTCCGCATTCTGCAAGCCTCCGGAGCGCACGTCGG CAGTCGGCTCCCTCGTTGACCGAATCACCGACCTCTCTCCCCAG GCAAGTTTGTACAAAAAAGCAGGCTGCCACCTGTCTCTTGCTTCA ACAGTGTTTGGACGGAACAgctagcATGGTCTCCAAAGGAGAGGAA GACAATATGGCCATTATCAAAGAGTTTATGCGATTTAAAGTGCACA TGGAGGGAAGCGTCAACGGGCATGAGTTTGAGATTGAGGGGGA AGGAGAGGGAAGACCTTATGAAGGGACCCAGACTGCCAAACTGA AAGTCACCAAGGGCGGACCCCTGCCATTTGCTTGGGACATTCTG AGCCCACAATTCATGTATGGGTCCAAAGCCTATGTTAAACACCCG GCAGACATACCTGACTACCTCAAGCTCTCCTTTCCTGAGGGGTTT AAATGGGAGAGGGTGATGAACTTTGAAGATGGAGGCGTCGTGAC AGTAACTCAGGATAGCAGCCTGCAAGACGGGGAGTTTATTTACAA GGTTAAATTGCGCGGAACCAACTTTCCCAGCGATGGACCCGTGA TGCAGAAGAAAACAATGGGCTGGGAAGCATCATCCGAAAGAATG TATCCCGAAGATGGCGCGTTGAAGGGGGAAATCAAACAAAGGCT CAAGTTGAAAGATGGAGGGCACTATGACGCTGAGGTCAAAACCA CGTACAAGGCTAAAAAACCGGTACAACTGCCCGGAGCATATAATG TTAATATTAAGCTGGACATCACCAGCCACAATGAGGATTACACAAT AGTGGAACAGTATGAAAGAGCCGAGGGAAGACACTCAACTGGGG GCATGGATGAACTGTATAAATGAgaattcCTGTGCCTTCTAGTTGCCA GCCATCTGTTGTTTGCCCCTCCCCCGTGCCTTCCTTGACCCTGG AAGGTGCCACTCCCACTGTCCTTTCCTAATAAAATGAGGAAATTG CATCGCATTGTCTGAGTAGGTGTCATTCTATTCTGGGGGGTGGGG TGGGGCAGGACAGCAAGGGGGAGGATTGGGAAGACAATAGCAG GCATGCTGGGGATGCGGTGGGCTCTATGGGCATTGGCGCAGAAA AAAACAACAACAATTGCATTCATTTTATGTTTCAGGTTCAGGGGGA GGTGTGGGAGGTTTTTTAAAGCAAGTAAAACCTCTACAAATGTGG TATTGGCCCATCTCTATCGGTATCGTAGCATAACCCCTTGGGGCCT CTAAACGGGTCTTGAGGGGTTTTTTGTGCCCCTCGGGCCGGATT GCTATCTACCGGCATTGGCGCAGAAAAAAATGCCTGATGCGACG CTGCGCGTCTTATACTCCCACATATGCCAGATTCAGCAACGGATA CGGCTTCCCCAACTTGCCCACTTCCATACGTGTCCTCCTTACCAG | nt | Module 2 full construct |
| | AAATTTATCCTTAAGGTCGTCAGCTATCCTGCAGGATAGTAATCAAT TACGGGGTCATTAGTTCATAGCCCATATATGGAGTTCCGggatccGAT CGCCCTACGTGCTGTCTCAGATCGCCCTACGTGCTGTCTCAGAT CGCCCTACGTGCTGTCTCAGATCGCCCTACGTGCTGTCTCAGAT CGCCCTACGTGCTGTCTCAGATCGCCCTACGTGCTGTCTCAGAT CGCCCTACGTGCTGTCTCAGATCGCCCTACGTGCTGTCTCACAA GTTTGTACAAAAAAGCAGGCTGCCACCgtcgacATGGTGAGTAAGG GGGAGGAAGATAACATGGCCTCACTTCCAGCGACTCACGAACTT CACATTTTTGGGTCCATCAATGGCGTGGATTTCGATATGGTAGGG CAAGGGACTGGCAACCCTAACGACGGTTACGAAGAACTCAATCT GAAATCAACGAAAGGGGATTTGCAATTTTCCCCATGGATACTCGT CCCCCATATAGGCTATGGATTCCACCAATATCTGCCGTATCCAGAC GGAATGTCTCCGTTTCAAGCCGCAATGGTAGACGGTAGCGGTTA CCAGGTCCACCGGACGATGCAATTCGAAGATGGGGCCTCCCTGA CAGTAAATTATAGATATACCTATGAAGGGAGTCACATAAAGGGAGA AGCTCAGGTCAAAGGAACGGGTTTCCCGGCTGACGGACCGGTG ATGACCAATTCACTGACCGCCGCCGATTGGTGCCGCTCTAAAAAA ACTTACCCAAATGATAAGACTATCATTTCAACTTTTAAATGGAGTTA CACTACGGGGAATGGTAAGAGGTACAGGTCAACAGCCAGGACAA CTTACACATTTGCCAAGCCAATGGCGGCGAACTATTTGAAAAATC AGCCAATGTACGTTTTCCGAAAAACCGAATTGAAACATTCTAAGAC CGAGCTTAATTTCAAGGAATGGCAAAAAGCATTTACGGATGTGAT GGGGATGGATGAGCTTTACAAGTAATAAggtaccATGTGATACCCATA GCTTCCATGAGAACAGCAGGGTAGTCTGGTTTCTAGACTTGTGCT GATCGTGCTAAATTTTCAGTAGGGCTACAAAACCTGATGTTAAAAT TCCATCCCATCATCTTGGTACTACTAGATGTCTTTAGGCAGCAGCT TTTAATACAGGGTAGATAACCTGTACTTCAAGTTAAAGTGAATAAC CACTTAAAAAATGTCCATGATGGAATATTCCCCTATCTCTAGAATTT TAAGTGCTTTGTAATGGGAACTGCCTCTTTCCTGTTGTTGTTAATG AAAATGTCAGAAACCAGTTATGTGAATGATCTCTCTGAATCCTAAG GGCTGGTCTCTGCTGAAGGTTGTAAGTGGTCGCTTACTTTGAGT GATCCTCCAACTTCATTTGATGCTAAATAGGAGATACCAGGTTGAA AGACCTTCTCCAAATGAGATCTAAGCCTTTCCATAAGGAATGTAGC TGGTTTCCTCATTCCTGAAAGAAACAGTTAACTTTCAGAAGAGAT GGGCTTGTTTTCTTGCCAATGAGGTCTGAAATGGAGGTCCTTCTG CTGGATAAAATGAGGTTCAACTGTTGATTGCAGGAATAAGGCCTT AATATGTTAACCTCAGTGTCATTTATGAAAAGAGGGGACCAGAAG CCAAAGACTTAGTATATTTTCTTTTCCTCTGTCCCTTCCCCCATAA GCCTCCATTTAGTTCTTTGTTATTTTTGTTTCTTCCAAAGCACATTG AAAGAGAACCAGTTTCAGGTGTTTAGTTGCAGACTCAGTTTGTCA GACTTTAAAGAATAATATGCTGCCAAATTTTGGCCAAAGTGTTAAT CTTAGGGGAGAGCTTTCTGTCCTTTTGGCACTGAGATATTTATTGT TTATTTATCAGTGACAGAGTTCACTATAAATGGTGTTTTTTTAATAG AATATAATTATCGGAAGCAGTGCCTTCCATAATTATGACAGTTATAC TGTCGGTTTTTTTTAAATAAAAGCAGCATCTGCTAATAAAACCCAA CAGATACTGGAAGTTTTGCATTTATGGTCAACACTTAAGGGTTTTA GAAAACAGCCGTCAGCCAAATGTAATTGAATAAAGTTGAAGCTAA GATTTAGAGATGAATTAAATTTAATTAGGGGTTGCTAAGAAGCGAG CACTGACCAGATAAGAATGCTGGTTTTCCTAAATGCAGTGAATTGT GACCAAGTTATAAATCAATGTCACTTAAAGGCTGTGGTAGTACTCC TGCAAAATTTTATAGCTCAGTTTATCCAAGGTGTAACTCTAATTCCC ATTTTGCAAAATTTCCAGTACCTTTGTCACAATCCTAACACATTATC GGGAGCAGTGTCTTCCATAATGTATAAAGAACAAGGTAGTTTTTAC CTACCACAGTGTCTGTATCGGAGACAGTGATCTCCATATGTTACAC TAAGGGTGTAAGTAATTATCGGGAACAGTGTTTCCCATAATTTTCT TCATGCAATGACAaagcttAAGTTTAAACCGCTGATCAGCCTCGACT GTGCCTTCTAGTTGCCAGCCATCTGTTGTTTGCCCCTCCCCCGTG CCTTCCTTGACCCTGGAAGGTGCCACTCCCACTGTCCTTTCCTAA TAAAATGAGGAAATTGCATCGCATTGTCTGAGTAGGTGTCATTCTA TTCTGGGGGGTGGGGTGGGGCAGGACAGCAAGGGGGAGGATT GGGAAGACAATAGCAGGCATGCTGGGGATGCGGTGGGCTCTATG | | |
| | GCTTCTGAGGCGGAAAGAACCAGCTGGGGCTCTAGGGGGTATC CCCACGCGCCCTGTAGCGGCGCATTAAGCGCGGCGGGTGTGGT GGTTACGCGCAGCGTGACCGCTACACTTGCCAGCGCCCTAGCG CCCGCTCCTTTCGCTTTCTTCCCTTCCTTTCTCGCCACGTTCGCC GGCTTTCCCCGTCAAGCTCTAAATCGGGGGCTCCCTTTAGGGTT CCGATTTAGTGCTTTACGGCACCTCGACCCCAAAAAACTTGATTA GGGTGATGGTTCACGTAGTGGGCCATCGCCCTGATAGACGGTTT TTCGCCCTTTGACGTTGGAGTCCACGTTCTTTAATAGTGGACTCT TGTTCCAAACTGGAACAACACTCAACCCTATCTCGGTCTATTCTTT TGATTTATAAGGGATTTTGCCGATTTCGGCCTATTGGTTAAAAAAT GAGCTGATTTAACAAAAATTTAACGCGAATTAATTCTGTGGAATGT GTGTCAGTTAGGGTGTGGAAAGTCCCCAGGCTCCCCAGCAGGC AGAAGTATGCAAAGCATGCATCTCAATTAGTCAGCAACCAGGTGT GGAAAGTCCCCAGGCTCCCCAGCAGGCAGAAGTATGCAAAGCAT GCATCTCAATTAGTCAGCAACCATAGTCCCGCCCCTAACTCCGCC CATCCCGCCCCTAACTCCGCCCAGTTCCGCCCATTCTCCGCCCC ATGGCTGACTAATTTTTTTTATTTATGCAGAGGCCGAGGCCGCCTC TGCCTCTGAGCTATTCCAGAAGTAGTGAGGAGGCTTTTTTGGAGG CCTAGGCTTTTGCAAAAAGCTCCCGGGAGCTTGTATATCCATTTTC GGATCTGATCAAGAGACAGGATGAGGATCGTTTCGCATGATTGAA CAAGATGGATTGCACGCAGGTTCTCCGGCCGCTTGGGTGGAGA GGCTATTCGGCTATGACTGGGCACAACAGACAATCGGCTGCTCT GATGCCGCCGTGTTCCGGCTGTCAGCGCAGGGGCGCCCGGTTC TTTTTGTCAAGACCGACCTGTCCGGTGCCCTGAATGAACTGCAG GACGAGGCAGCGCGGCTATCGTGGCTGGCCACGACGGGCGTTC CTTGCGCAGCTGTGCTCGACGTTGTCACTGAAGCGGGAAGGGA CTGGCTGCTATTGGGCGAAGTGCCGGGGCAGGATCTCCTGTCAT CTCACCTTGCTCCTGCCGAGAAAGTATCCATCATGGCTGATGCAA TGCGGCGGCTGCATACGCTTGATCCGGCTACCTGCCCATTCGAC CACCAAGCGAAACATCGCATCGAGCGAGCACGTACTCGGATGGA AGCCGGTCTTGTCGATCAGGATGATCTGGACGAAGAGCATCAGG GGCTCGCGCCAGCCGAACTGTTCGCCAGGCTCAAGGCGCGCAT GCCCGACGGCGAGGATCTCGTCGTGACCCATGGCGATGCCTGC TTGCCGAATATCATGGTGGAAAATGGCCGCTTTTCTGGATTCATC GACTGTGGCCGGCTGGGTGTGGCGGACCGCTATCAGGACATAG CGTTGGCTACCCGTGATATTGCTGAAGAGCTTGGCGGCGAATGG GCTGACCGCTTCCTCGTGCTTTACGGTATCGCCGCTCCCGATTC GCAGCGCATCGCCTTCTATCGCCTTCTTGACGAGTTCTTCTGAGC GGGACTCTGGGGTTCGAAATGACCGACCAAGCGACGCCCAACC TGCCATCACGAGATTTCGATTCCACCGCCGCCTTCTATGAAAGGT TGGGCTTCGGAATCGTTTTCCGGGACGCCGGCTGGATGATCCTC CAGCGCGGGGATCTCATGCTGGAGTTCTTCGCCCACCCCAACTT GTTTATTGCAGCTTATAATGGTTACAAATAAAGCAATAGCATCACAA ATTTCACAAATAAAGCATTTTTTTCACTGCATTCTAGTTGTGGTTTG TCCAAACTCATCAATGTATCTTATCATGTCTGTATACCGTCGACCTC TAGCTAGAGCTTGGCGTAATCATGGTCATAGCTGTTTCCTGTGTG AAATTGTTATCCGCTCACAATTCCACACAACATACGAGCCGGAAG CATAAAGTGTAAAGCCTGGGGTGCCTAATGAGTGAGCTAACTCAC ATTAATTGCGTTGCGCTCACTGCCCGCTTTCCAGTCGGGAAACCT GTCGTGCCAGCTGCATTAATGAATCGGCCAACGCGCGGGGAGAG GCGGTTTGCGTATTGGGCGCTCTTCCGCTTCCTCGCTCACTGAC TCGCTGCGCTCGGTCGTTCGGCTGCGGCGAGCGGTATCAGCTC ACTCAAAGGCGGTAATACGGTTATCCACAGAATCAGGGGATAACG CAGGAAAGAACATGTGAGCAAAAGGCCAGCAAAAGGCCAGGAA CCGTAAAAAGGCCGCGTTGCTGGCGTTTTTCCATAGGCTCCGCC CCCCTGACGAGCATCACAAAAATCGACGCTCAAGTCAGAGGTGG CGAAACCCGACAGGACTATAAAGATACCAGGCGTTTCCCCCTGG AAGCTCCCTCGTGCGCTCTCCTGTTCCGACCCTGCCGCTTACCG GATACCTGTCCGCCTTTCTCCCTTCGGGAAGCGTGGCGCTTTCT CATAGCTCACGCTGTAGGTATCTCAGTTCGGTGTAGGTCGTTCGC TCCAAGCTGGGCTGTGTGCACGAACCCCCCGTTCAGCCCGACC | | |
| | GCTGCGCCTTATCCGGTAACTATCGTCTTGAGTCCAACCCGGTAA GACACGACTTATCGCCACTGGCAGCAGCCACTGGTAACAGGATT AGCAGAGCGAGGTATGTAGGCGGTGCTACAGAGTTCTTGAAGTG GTGGCCTAACTACGGCTACACTAGAAGAACAGTATTTGGTATCTG CGCTCTGCTGAAGCCAGTTACCTTCGGAAAAAGAGTTGGTAGCT CTTGATCCGGCAAACAAACCACCGCTGGTAGCGGTTTTTTTGTTT GCAAGCAGCAGATTACGCGCAGAAAAAAAGGATCTCAAGAAGAT CCTTTGATCTTTTCTACGGGGTCTGACGCTCAGTGGAACGAAAAC TCACGTTAAGGGATTTTGGTCATGAGATTATCAAAAAGGATCTTCA CCTAGATCCTTTTAAATTAAAAATGAAGTTTTAAATCAATCTAAAGTA TATATGAGTAAACTTGGTCTGACAGTTACCAATGCTTAATCAGTGA GGCACCTATCTCAGCGATCTGTCTATTTCGTTCATCCATAGTTGCC TGACTCCCCGTCGTGTAGATAACTACGATACGGGAGGGCTTACCA TCTGGCCCCAGTGCTGCAATGATACCGCGAGACCCACGCTCACC GGCTCCAGATTTATCAGCAATAAACCAGCCAGCCGGAAGGGCCG AGCGCAGAAGTGGTCCTGCAACTTTATCCGCCTCCATCCAGTCTA TTAATTGTTGCCGGGAAGCTAGAGTAAGTAGTTCGCCAGTTAATAG TTTGCGCAACGTTGTTGCCATTGCTACAGGCATCGTGGTGTCACG CTCGTCGTTTGGTATGGCTTCATTCAGCTCCGGTTCCCAACGATC AAGGCGAGTTACATGATCCCCCATGTTGTGCAAAAAAGCGGTTAG CTCCTTCGGTCCTCCGATCGTTGTCAGAAGTAAGTTGGCCGCAG TGTTATCACTCATGGTTATGGCAGCACTGCATAATTCTCTTACTGT CATGCCATCCGTAAGATGCTTTTCTGTGACTGGTGAGTACTCAAC CAAGTCATTCTGAGAATAGTGTATGCGGCGACCGAGTTGCTCTTG CCCGGCGTCAATACGGGATAATACCGCGCCACATAGCAGAACTTT AAAAGTGCTCATCATTGGAAAACGTTCTTCGGGGCGAAAACTCTC AAGGATCTTACCGCTGTTGAGATCCAGTTCGATGTAACCCACTCG TGCACCCAACTGATCTTCAGCATCTTTTACTTTCACCAGCGTTTCT GGGTGAGCAAAAACAGGAAGGCAAAATGCCGCAAAAAAGGGAAT AAGGGCGACACGGAAATGTTGAATACTCATACTCTTCCTTTTTCAA TATTATTGAAGCATTTATCAGGGTTATTGTCTCATGAGCGGATACAT ATTTGAATGTATTTAGAAAAATAAACAAATAGGGGTTCCGCGCACA TTTCCCCGAAAAGTGCCACCTGACGTC | | |
| **30** | ATGGCGCCCTTTCCTGAAGAAGTGGACGTCTTCACCGCCCCACA CTGGCGGATGAAGCAGCTGGTGGGGCTCTACTGCGACAAGCTTT CTAAAACCAATTTTTCCAACAACAACGATTTCCGTGCTCTTCTGCA GTCTTTGTATGCTACTTTCAAGGAGTTCAAAATGCATGAGCAGATT GAAAATGAATACATTATTGGTTTGCTTCAACAACGCAGCCAGACCA TTTATAATGTACATTCTGACAATAAACTCTCCGAGATGCTTAGCCTC TTTGAAAAGGGACTGAAGAATGTTAAGAATGAATATGAACAGTTAA ATTATGCAAAACAACTGAAAGAGAGATTGGAGGCTTTTACAAGAG ATTTTCTTCCTCACATGAAAGAGGAAGAGGAGGTTTTTCAGCCCA TGTTAATGGAATATTTTACCTATGAAGAGCTTAAGGATATTAAAAAG AAAGTGATTGCACAACACTGCTCTGGCGGCGGCGTGAGTAAGGG GGAGGAAGATAACATGGCCTCACTTCCAGCGACTCACGAACTTC ACATTTTTGGGTCCATCAATGGCGTGGATTTCGATATGGTAGGGC AAGGGACTGGCAACCCTAACGACGGTTACGAAGAACTCAATCTG AAATCAACGAAAGGGGATTTGCAATTTTCCCCATGGATACTCGTC CCCCATATAGGCTATGGATTCCACCAATATCTGCCGTATCCAGACG GAATGTCTCCGTTTCAAGCCGCAATGGTAGACGGTAGCGGTTACC AGGTCCACCGGACGATGCAATTCGAAGATGGGGCCTCCCTGACA GTAAATTATAGATATACCTATGAAGGGAGTCACATAAAGGGAGAAG CTCAGGTCAAAGGAACGGGTTTCCCGGCTGACGGACCGGTGAT GACCAATTCACTGACCGCCGCCGATTGGTGCCGCTCTAAAAAAA CTTACCCAAATGATAAGACTATCATTTCAACTTTTAAATGGAGTTAC ACTACGGGGAATGGTAAGAGGTACAGGTCAACAGCCAGGACAAC TTACACATTTGCCAAGCCAATGGCGGCGAACTATTTGAAAAATCA GCCAATGTACGTTTTCCGAAAAACCGAATTGAAACATTCTAAGACC GAGCTTAATTTCAAGGAATGGCAAAAAGCATTTACGGATGTGATG GGGATGGATGAGCTTTACAAGggtaccGGAAGCGGAGAGGGCAGG | nt | Module 1 - Design 2: b), c) and d) (comprising Hr domain, Gly linker, mNeonGreen, T2A, and mCherry) |
| | GGAAGTCTTCTAACATGCGGGGACGTGGAGGAAAATCCCGGCCC CATGGTCTCCAAAGGAGAGGAAGACAATATGGCCATTATCAAAGA GTTTATGCGATTTAAAGTGCACATGGAGGGAAGCGTCAACGGGC ATGAGTTTGAGATTGAGGGGGAAGGAGAGGGAAGACCTTATGAA GGGACCCAGACTGCCAAACTGAAAGTCACCAAGGGCGGACCCC TGCCATTTGCTTGGGACATTCTGAGCCCACAATTCATGTATGGGT CCAAAGCCTATGTTAAACACCCGGCAGACATACCTGACTACCTCA AGCTCTCCTTTCCTGAGGGGTTTAAATGGGAGAGGGTGATGAAC TTTGAAGATGGAGGCGTCGTGACAGTAACTCAGGATAGCAGCCT GCAAGACGGGGAGTTTATTTACAAGGTTAAATTGCGCGGAACCAA CTTTCCCAGCGATGGACCCGTGATGCAGAAGAAAACAATGGGCT GGGAAGCATCATCCGAAAGAATGTATCCCGAAGATGGCGCGTTG AAGGGGGAAATCAAACAAAGGCTCAAGTTGAAAGATGGAGGGCA CTATGACGCTGAGGTCAAAACCACGTACAAGGCTAAAAAACCGGT ACAACTGCCCGGAGCATATAATGTTAATATTAAGCTGGACATCACC AGCCACAATGAGGATTACACAATAGTGGAACAGTATGAAAGAGCC GAGGGAAGACACTCAACTGGGGGCATGGATGAACTGTATAAATG ATAA | | |
| **31** | TTGTCACAATCCTAACACATTATCGGGAGCAGTGTCTTCCATAATG TATAAAGAAC | nt | IRE ("C") comprised in 3'UTR of TfR |
| **32** | ACCACAGTGTCTGTATCGGAGACAGTGATCTCCATATGTTACACTA AGGGTGTAAGTAATTATCGGGAACAGTGTTTCCCATAATTTTCTTC A | nt | IREs ("D" and "E") comprised in 3'UTR of TfR |

### Embodiment list

A list of potential embodiments reads as follows:
1. A nucleic acid, comprising
   a. A promoter region;
   b. A first coding region encoding a first polypeptide,
      wherein the first polypeptide comprises a first reporter protein domain and a first Fe²⁺-sensitive domain;
   c. A polypeptide separator region; and
   d. A second coding region encoding a second polypeptide,
      wherein the second polypeptide comprises a second reporter protein domain, and
      wherein the first reporter protein domain and the second reporter protein domain are different.
2. The nucleic acid of embodiment 1, wherein the nucleic acid is an expression cassette.
3. The nucleic acid of embodiment 1 or 2, comprising a) to d) in 5' to 3' direction and/or wherein b), c) and d) are under control of a).
4. The nucleic acid of any one of embodiments 1 to 3, wherein the promoter region results in a moderate expression of b), c) and d), operably linked thereto.
5. The nucleic acid of any one of embodiments 1 to 4, wherein the promoter region comprises or consist of a human phosphoglycerate kinase (hPGK) promoter.
6. The nucleic acid of any one of embodiments 1 to 5, wherein the promoter region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 1.
7. The nucleic acid of any one of embodiments 1 to 6, wherein the promoter region comprises or consists of the sequence set forth in SEQ ID NO: 1.
8. The nucleic acid of any one of embodiments 1 to 7, wherein the promoter region comprises or consists of a sequence having the same biological function as the sequence set forth in SEQ ID NO: 1.
9. The nucleic acid of any one of embodiments 1 to 3, wherein the promoter region results in a strong expression of b), c) and d), operably linked thereto.
10. The nucleic acid of any one of embodiments 1 to 3 and 9, wherein the promoter region comprises a cytomegalovirus (CMV) promoter.
11. The nucleic acid of any one of embodiments 1 to 10, wherein the polypeptide separator region is a region encoding a proteolytic cleavage site.
12. The nucleic acid of any one of embodiments 1 to 10, wherein the polypeptide separator region is a ribosome skipping region.
13. The nucleic acid of embodiment 12, wherein the ribosome skipping region encodes a peptide that causes ribosome skipping followed by recommencement of translation.
14. The nucleic acid of embodiment 12 or 13, wherein the ribosome skipping region comprises a sequence encoding an amino acid sequence having more than 95%, more than 96%, more than 97%, more than 98%, more than 99%, more than 99.5%, more than 99.9% sequence identity with the sequence set forth in SEQ ID NO: 2 and/or SEQ ID NO: 3.
15. The nucleic acid of any one of embodiments 12 to 14, wherein the ribosome skipping region comprises a sequence encoding the sequence set forth in SEQ ID NO: 2 and/or SEQ ID NO: 3.
16. The nucleic acid of any one of embodiments 12 to 15, wherein the ribosome skipping region is a 2A region.
17. The nucleic acid of embodiment 16, wherein the 2A region encodes a 2A peptide selected from the group consisting of F2A (foot-and-mouth disease virus), E2A (equine rhinitis A virus), P2A (porcine teschovirus-1 2A), and T2A (thosea asigna virus 2A).
18. The nucleic acid of any one of embodiments 12 to 17, wherein the ribosome skipping region encodes T2A.
19. The nucleic acid of any one of embodiments 12 to 18, wherein the ribosome skipping region comprises a sequence encoding an amino acid sequence having more than 95%, more than 96%, more than 97%, more than 98%, more than 99%, more than 99.5%, more than 99.9% sequence identity with the sequence set forth in SEQ ID NO: 4.
20. The nucleic acid of any one of embodiments 12 to 19, wherein the ribosome skipping region comprises or consists of a sequence encoding the sequence set forth in SEQ ID NO: 4.
21. The nucleic acid of any one of embodiments 12 to 20, wherein the ribosome skipping region comprises or consists of a sequence encoding an amino acid sequence having the same biological function as the sequence set forth in SEQ ID NO: 4.
22. The nucleic acid of any one of embodiments 12 to 21, wherein the ribosome skipping region comprises a sequence having more than 95%, more than 96%, more than 97%, more than 98%, more than 99%, more than 99.5%, more than 99.9% sequence identity with the sequence set forth in SEQ ID NO: 5.
23. The nucleic acid of any one of embodiments 12 to 22, wherein the ribosome skipping region comprises or consists of the sequence set forth in SEQ ID NO: 5.
24. The nucleic acid of any one of embodiments 12 to 23, wherein the ribosome skipping region comprises or consists of a sequence having the same biological function as the sequence set forth in SEQ ID NO: 5.
25. The nucleic acid of any one of embodiments 1 to 24, wherein the first Fe²⁺-sensitive domain comprises a degron sequence.
26. The nucleic acid of any one of embodiments 1 to 25, wherein the first coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 6.
27. The nucleic acid of any one of embodiments 1 to 26, wherein the first coding region comprises a sequence encoding the sequence set forth in SEQ ID NO: 6.
28. The nucleic acid of any one of embodiments 1 to 27, wherein the first coding region comprises a sequence encoding an amino acid sequence having the same biological function as the sequence set forth in SEQ ID NO: 6.
29. The nucleic acid of any one of embodiments 1 to 28, wherein the first coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 7.
30. The nucleic acid of any one of embodiments 1 to 29, wherein the first coding region comprises the sequence set forth in SEQ ID NO: 7.
31. The nucleic acid of any one of embodiments 1 to 30, wherein the first coding region comprises a sequence having the same biological function as the sequence set forth in SEQ ID NO: 7.
32. The nucleic acid of any one of embodiments 1 to 31, wherein first Fe²⁺-sensitive domain comprises or consists of a Hr domain of FBXL5.
33. The nucleic acid of any one of embodiments 1 to 32, wherein the first coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 8.
34. The nucleic acid of any one of embodiments 1 to 33, wherein the first coding region comprises a sequence encoding the sequence set forth in SEQ ID NO: 8.
35. The nucleic acid of any one of embodiments 1 to 34, wherein first coding region comprises a sequence encoding an amino acid sequence having the same biological function as the sequence set forth in SEQ ID NO: 8.
36. The nucleic acid of any one of embodiments 1 to 35, wherein the first coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 9.
37. The nucleic acid of any one of embodiments 1 to 36, wherein the first coding region comprises the sequence set forth in SEQ ID NO: 9.
38. The nucleic acid of any one of embodiments 1 to 37, wherein first coding region comprises a sequence having the same biological function as the sequence set forth in SEQ ID NO: 9.
39. The nucleic acid of any one of embodiments 1 to 38, wherein first reporter protein domain is a first fluorescent protein domain.
40. The nucleic acid of any one of embodiments 1 to 39, wherein second reporter protein domain is a second fluorescent protein domain.
41. The nucleic acid of embodiment 39 or 40, wherein the first fluorescent protein domain and the second fluorescent protein domain are selected from the group consisting of mTagBFP2, mNeonGreen, and mCherry.
42. The nucleic acid of any one of embodiments 39 to 41, wherein the first fluorescent protein domain is mTagBFP2 or mNeonGreen.
43. The nucleic acid of any one of embodiments 39 to 42, wherein the second fluorescent protein domain is mCherry.
44. The nucleic acid of any one of embodiments 39 to 43, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 10.
45. The nucleic acid of any one of embodiments 39 to 44, wherein the first coding region or the second coding region comprises a sequence encoding the sequence set forth in SEQ ID NO: 10.
46. The nucleic acid of any one of embodiments 39 to 45, wherein the first coding region or the second coding region comprises a sequence encoding an amino acid sequence having the same biological function as the sequence set forth in SEQ ID NO: 10.
47. The nucleic acid of any one of embodiments 39 to 46, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 11.
48. The nucleic acid of any one of embodiments 39 to 47, wherein the first coding region or the second coding region comprises the sequence set forth in SEQ ID NO: 11.
49. The nucleic acid of any one of embodiments 39 to 48, wherein the first coding region or the second coding region comprises a sequence having the same biological function as the sequence set forth in SEQ ID NO: 11.
50. The nucleic acid of any one of embodiments 39 to 49, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 12.
51. The nucleic acid of any one of embodiments 39 to 50, wherein the first coding region or the second coding region comprises a sequence encoding the sequence set forth in SEQ ID NO: 12.
52. The nucleic acid of any one of embodiments 39 to 51, wherein the first coding region or the second coding region comprises a sequence encoding an amino acid sequence having the same biological function as the sequence set forth in SEQ ID NO: 12.
53. The nucleic acid of any one of embodiments 39 to 52, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 13.
54. The nucleic acid of any one of embodiments 39 to 53, wherein the first coding region or the second coding region comprises the sequence set forth in SEQ ID NO: 13.
55. The nucleic acid of any one of embodiments 39 to 54, wherein the first coding region or the second coding region comprises a sequence having the same biological function as the sequence set forth in SEQ ID NO: 13.
56. The nucleic acid of any one of embodiments 39 to 55, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 14.
57. The nucleic acid of any one of embodiments 39 to 56, wherein the first coding region or the second coding region comprises a sequence encoding the sequence set forth in SEQ ID NO: 14.
58. The nucleic acid of any one of embodiments 39 to 57, wherein the first coding region or the second coding region comprises a sequence encoding an amino acid sequence having the same biological function as the sequence set forth in SEQ ID NO: 14.
59. The nucleic acid of any one of embodiments 39 to 58, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 15.
60. The nucleic acid of any one of embodiments 39 to 59, wherein the first coding region or the second coding region comprises the sequence set forth in SEQ ID NO: 15.
61. The nucleic acid of any one of embodiments 39 to 60, wherein the first coding region or the second coding region comprises a sequence having the same biological function as the sequence set forth in SEQ ID NO: 15.
62. The nucleic acid of any one of embodiments 1 to 61, wherein the second polypeptide is Fe²⁺-insensitive.
63. The nucleic acid of any one of embodiments 1 to 62, wherein the second polypeptide does not comprise an Fe²⁺-sensitive domain.
64. The nucleic acid of any one of embodiments 1 to 63, wherein the second polypeptide is Fe²⁺-sensitive.
65. The nucleic acid of any one of embodiments 1 to 61 and 64, wherein the second polypeptide comprises a second Fe²⁺-sensitive domain.
66. The nucleic acid of embodiment 65, wherein the second Fe²⁺-sensitive domain comprises or consists of a potential iron sulfur cluster (ISC) binding domain.
67. The nucleic acid of embodiment 65 or 66, wherein the second Fe²⁺-sensitive domain comprises or consists of a iron sulfur cluster (ISC) binding domain.
68. The nucleic acid of any one of embodiments 1 to 67, wherein the nucleic acid comprises the sequence set forth in SEQ ID NO: 30.
69. A vector comprising the nucleic acid of any one of embodiments 1 to 68.
70. The vector of embodiment 69, wherein the vector is a viral vector.
71. The vector of embodiment 70, wherein the viral vector is a lentivirus vector, an adenovirus, an adeno-associated virus (AAV), and/or a baculovirus.
72. The vector of embodiment 69, wherein the vector is a plasmid.
73. The vector of embodiment 72, wherein the plasmid is a VB240227-1627une plasmid or a PcDNA3.1-plasmid.
74. The vector of embodiment 72 or 73 comprising or consisting of the sequence set forth in SEQ ID NO: 19.
75. A cell comprising nucleic acid of any one of embodiments 1 to 68 and/or the vector of any one of embodiments 69 to 74.
76. The cell of embodiment 75, wherein the first polypeptide and the second polypeptide are stably or transiently expressed in the cell.
77. The cell of embodiment 75 or 76, wherein the cell is a HeLa cell, a macrophage, an enterocyte, a hepatocyte, a cancer cell, an immune cell and/or a neuron.
78. An *in vitro* method for measuring cellular Fe²⁺, comprising:
   a. Providing the cell of any one of embodiments 75 to 77, and
   b. Measuring a signal of the first reporter protein domain and a signal of the second reporter protein domain,
   wherein at least intensity of the signal of the first reporter protein domain is indicative of an amount of cellular Fe²⁺.
79. The method of embodiment 78, wherein the first reporter protein domain is a first fluorescent protein domain, the second reporter protein domain a second fluorescent protein domain, the signal is fluorescence, and the method comprises:
   a. Providing the cell of any one of embodiments 75 to 77 and exciting the cell, and
   b. Measuring fluorescence of the first fluorescent protein domain and fluorescence of the second fluorescent protein domain,
   wherein at least intensity of fluorescence of the first fluorescent protein domain is indicative of an amount of cellular Fe²⁺.
80. The method of embodiment 78 or 79, wherein the method further comprises prior or after, preferably after, step b):
   c. Amending an amount of extracellular and/or cellular Fe²⁺.
81. The method of embodiment 80, wherein the amount of Fe²⁺ is amended by adding to the cell a composition comprising Fe²⁺.
82. The method of embodiment 81, wherein the composition comprises FeSO₄.
83. The method of embodiment 81 or 82, wherein the composition is FeSO₄ Vitamin C.
84. The method of embodiment 80, wherein the amount of Fe²⁺ is amended by adding to the cell a Fe²⁺ chelator, preferably a cell membrane permeable Fe²⁺ chelator.
85. The method of embodiment 84, wherein the Fe²⁺ chelator is eltrombopag (ELT).
86. The method of embodiment 80, wherein the amount of Fe²⁺ is amended by adding a compound affecting or being suspected of affecting cellular Fe²⁺.
87. The method of embodiment 86, wherein the compound is an active ingredient, a drug candidate and/or a drug.
88. The method of any one of embodiments 80 to 87, wherein the method comprises after step c) another step b) referred to as step d).
89. The method of any one of embodiments 78 to 88, wherein the method comprises
   e. Comparing measurements obtained from step b) with measurements obtained from step d) and/or a control.
90. An *in vitro* screening method for a compound that affects cellular Fe²⁺, the method comprising the steps of
   a. Providing the cell of one of embodiments 75 to 77,
   b. Optionally measuring a signal of the first reporter protein domain and a signal of the second reporter protein domain,
   c. Adding a compound affecting or being suspected of affecting cellular Fe²⁺,
   d. Measuring a signal of the first reporter protein domain and a signal of the second reporter protein domain, and
   e. Comparing measurements obtained from step d) with measurements obtained from step b) and/or a control,

   wherein at least an intensity of a signal of the first reporter protein domain is indicative for an amount of cellular Fe²⁺, and
   wherein a significant change in at least the intensity of a signal of the first reporter protein domain is indicative of the compound affecting cellular Fe²⁺.
91. The method of embodiment 90, wherein the first reporter protein domain is a first fluorescent protein domain, the second reporter protein domain a second fluorescent protein domain, the signal is fluorescence, and the method comprises
   a. Providing the cell of one of embodiments 75 to 77 and exciting the cell,
   b. Optionally measuring fluorescence of the first fluorescent protein domain and fluorescence of the second fluorescent protein domain,
   c. Adding a compound affecting or being suspected of affecting cellular Fe²⁺,
   d. Measuring fluorescence of the first fluorescent protein domain and fluorescence of the second fluorescent protein domain, and
   e. Comparing measurements obtained from step d) with measurements obtained from step b) and/or a control,

   wherein at least an intensity of fluorescence of the first fluorescent protein domain is indicative for an amount of cellular Fe²⁺, and
   wherein a significant change in at least the intensity of fluorescence of the first fluorescent protein domain is indicative of the compound affecting cellular Fe²⁺.
92. An *in vitro* method for identifying a dosage and/or formulation of a compound that affects cellular Fe²⁺, the method comprising the steps of
   a. Providing the cell of one of embodiments 75 to 77,
   b. Optionally measuring a signal of the first reporter protein domain and a signal of the second reporter protein domain,
   c. Adding a compound affecting or being suspected of affecting cellular Fe²⁺ in in a dosage and/or formulation,
   d. Measuring a signal of the first reporter protein domain and a signal of the second reporter protein domain, and
   e. Comparing measurements obtained from step d) with measurements obtained from step b) and/or a control,

   wherein at least an intensity of a signal of the first reporter protein domain is indicative for an amount of cellular Fe²⁺, and
   wherein a significant change in at least the intensity of a signal of the first reporter protein domain is indicative of the dosage and/or formulation affecting cellular Fe²⁺.
93. The method of embodiment 92, wherein the first reporter protein domain is a first fluorescent protein domain, the second reporter protein domain a second fluorescent protein domain, the signal is fluorescence, and the method comprises
   a. Providing the cell of one of embodiments 75 to 77 and exciting the cell,
   b. Optionally measuring fluorescence of the first fluorescent protein domain and fluorescence of the second fluorescent protein domain,
   c. Adding a compound affecting or being suspected of affecting cellular Fe²⁺ in in a dosage and/or formulation,
   d. Measuring fluorescence of the first fluorescent protein domain and fluorescence of the second fluorescent protein domain, and
   e. Comparing measurements obtained from step d) with measurements obtained from step b) and/or a control,

   wherein at least an intensity of fluorescence of the first fluorescent protein domain is indicative for an amount of cellular Fe²⁺, and
   wherein a significant change in at least the intensity of fluorescence of the first fluorescent protein domain is indicative of the dosage and/or formulation affecting cellular Fe²⁺.
94. The method of any one of embodiments 78 to 93, wherein the method further comprises prior step a) the step of contacting the cell with the vector of any one of embodiments 69 to 74.
95. The method of any one of embodiments 78 to 94, wherein binding of cellular Fe²⁺ to the first Fe²⁺-sensitive domain results in degradation of the first reporter protein domain and/or of the first polypeptide.
96. The method of embodiment 95, wherein the first reporter protein domain and/or the first polypeptide is degraded by a proteasome.
97. The method of any one of embodiments 78 to 94, wherein binding of cellular Fe²⁺ to the first Fe²⁺-sensitive domain results in stabilization of the first reporter protein domain and/or of the first polypeptide.
98. The method of any one of embodiments 78 to 94 and 97, wherein the first reporter protein domain and/or the first polypeptide is stabilized by binding of cellular Fe²⁺ to the first Fe²⁺-sensitive domain.
99. The method of any one of embodiments 78 to 94, 97 and 98, wherein stability of the first reporter protein domain and/or of the first polypeptide is increased by binding of cellular Fe²⁺ to the first Fe²⁺-sensitive domain compared to an absence of cellular Fe²⁺ bound to the first Fe²⁺-sensitive domain.
100. The method of any one of embodiments 78 to 99, wherein the second polypeptide is Fe²⁺-insensitive.
101.The method of any one of embodiments 78 to 100, wherein the second reporter protein domain is an internal control.
102.The method of embodiment 100 or 102, wherein the method is a pseudo-ratiometric method.
103.The method of any one of embodiments 78 to 102, wherein the second polypeptide is Fe²⁺-sensitive.
104.The method of embodiment 103, wherein an intensity of the signal of the second reporter protein domain is indicative of an amount of cellular Fe²⁺.
105.The method of embodiment 103 or 104, wherein the method is a ratiometric method.
106.The method of any one of embodiments 78 to 99 and 103 to 105, wherein the second polypeptide comprises a second Fe²⁺-sensitive domain.
107.The method of embodiment 106, wherein binding of cellular Fe²⁺ to the second Fe²⁺-sensitive domain results in degradation of the second reporter protein domain and/or of the second polypeptide.
108.The method of embodiment 107, wherein the second reporter protein domain and/or the second polypeptide is degraded by a proteasome.
109.The method of any one of embodiments 106 to 108, wherein binding of cellular Fe²⁺ to the second Fe²⁺-sensitive domain results in stabilization of the second reporter protein domain and/or of the second polypeptide.
110.The method of embodiment 109, wherein the second reporter protein domain and/or the second polypeptide is stabilized by binding of cellular Fe²⁺ to the second Fe²⁺-sensitive domain.
111.The method of embodiment 109 or 110, wherein stability of the second reporter protein domain and/or of the second polypeptide is increased by binding of cellular Fe²⁺ to the second Fe²⁺-sensitive domain compared to an absence of cellular Fe²⁺ bound to the second Fe²⁺-sensitive domain.
112.The method of any one of embodiments 109 to 111, wherein the first reporter protein domain and/or the first polypeptide is stabilized by binding of cellular Fe²⁺ to the first Fe²⁺-sensitive domain and wherein the second reporter protein domain and/or the second polypeptide is degraded by binding of cellular Fe²⁺ to the second Fe²⁺-sensitive domain.
113.The method of any one of embodiments 109 to 111, wherein the first reporter protein domain and/or the first polypeptide is degraded by binding of cellular Fe²⁺ to the first Fe²⁺-sensitive domain and wherein the second reporter protein domain and/or the second polypeptide is stabilized by binding of cellular Fe²⁺ to the second Fe²⁺-sensitive domain.
114.The method of any one of embodiments 78 to 113, wherein the cell is excited at 405 nm, 470-490 nm, 561 nm, and/or 540-590 nm.
115.The method of any one of embodiments 78 to 114, wherein fluorescence is measured at 450 nm, 500-520 nm, and/or 565-650 nm.
116.The method of any one of embodiments 78 to 115, wherein fluorescence of the first fluorescent protein domain is excited at 405 nm and/or 470-490 nm and measured at 450 nm and/or 500-520 nm.
117.The method of any one of embodiments 78 to 116, wherein fluorescence of the second fluorescent protein domain is excited at 561 nm and/or 540-590 nm and measured at 565-650 nm.
118.The method of any one of embodiments 78 to 117, wherein fluorescence of the first fluorescent protein domain and fluorescence of the second fluorescent protein domain are measured by dynamic time-lapse imaging.
119.The method of any one of embodiments 78 to 118, wherein fluorescence of the first fluorescent protein domain and fluorescence of the second fluorescent protein domain are measured using a high-resolution microscopy.
120.The method of any one of embodiments 78 to 119, wherein step b) is performed repeatedly and/or continuously.
121. Use of the nucleic acid of any one of embodiments 1 to 68 to obtain the vector of any one of embodiments 69 to 74.
122. Use of the vector of any one of embodiments 69 to 74 to express the nucleic acid of any one of embodiments 1 to 68.
123. Use of the vector of any one of embodiments 69 to 74 to obtain the cell of any one of embodiments 75 to 77.
124. Use of the cell of any one of embodiments 75 to 77 for *in vitro* measuring and/or monitoring ferrous iron (Fe²⁺) in a cell, preferably according to the method of any one of embodiments 78 to 89 and 94 to 120.
125. Use of the cell any one of embodiments 75 to 77 for *in vitro* screening for a compound that affects cellular Fe²⁺, preferably according to the method of any one of embodiments 90, 91 and 94 to 120.
126. Use of the cell any one of embodiments 75 to 77 *in vitro* identifying a dosage and/or formulation of a compound that affects cellular Fe²⁺, preferably according to the method of any one of embodiments 92 to 120.
127.A nucleic acid, comprising in 5' to 3' direction:
   a. A first promoter region,
   b. A first coding region encoding
      i. an iron responsible element (IRE), and
      ii. a first reporter protein domain,
   c. A second promoter region comprising an Fe²⁺ and/or ISC sensitive region,
   d. A second coding region encoding
      i. a second reporter protein domain,
         wherein the first reporter protein domain and the second reporter protein domain are different, and
      ii. an IRE.
128.The nucleic acid of embodiment 127, wherein the first reporter protein domain is a first fluorescent protein domain, the second reporter protein domain is a second fluorescent protein domain, and the nucleic acid comprising in 5' to 3' direction:
   a. A first promoter region,
   b. A first coding region encoding
      i. an iron responsible element (IRE), and
      ii. a first fluorescent protein domain,
   c. A second promoter region comprising an Fe²⁺ and/or ISC sensitive region,
   d. A second coding region encoding
      i. a second fluorescent protein domain,
         wherein the first fluorescent protein domain and the second fluorescent protein domain are different, and
      ii. an IRE.
129.The nucleic acid of embodiment 127 or 128, wherein the nucleic acid is an expression cassette.
130. The nucleic acid of any one of embodiments 127 to 129, wherein b) is under control of a) and/or wherein d) is under control of c).
131.The nucleic acid of any one of embodiments 127 to 130, wherein the first promoter region results in a moderate expression of the first coding region operably linked thereto.
132.The nucleic acid of any one of embodiments 127 to 131, wherein the first promoter region comprises or consists of a hPGK promoter.
133.The nucleic acid of any one of embodiments 127 to 132, wherein the first promoter region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 1.
134.The nucleic acid of any one of embodiments 127 to 133, wherein the first promoter region comprises or consists of the sequence set forth in SEQ ID NO: 1.
135.The nucleic acid of any one of embodiments 127 to 134, wherein the first promoter region comprises or consists of a sequence having the same biological function as the sequence set forth in SEQ ID NO: 1.
136.The nucleic acid of any one of embodiments 128 to 135, wherein the first fluorescent protein domain and the second fluorescent protein domain are selected from the group consisting of mTagBFP2, mNeonGreen, and mCherry.
137.The nucleic acid of any one of embodiments 128 to 136, wherein the first fluorescent protein domain is mCherry.
138.The nucleic acid of any one of embodiments 128 to 137, wherein the second fluorescent protein domain is mNeonGreen.
139.The nucleic acid of any one of embodiments 127 to 138, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 10.
140.The nucleic acid of any one of embodiments 127 to 139, wherein the first coding region or the second coding region comprises a sequence encoding the sequence set forth in SEQ ID NO: 10.
141.The nucleic acid of any one of embodiments 127 to 140, wherein the first coding region or the second coding region comprises a sequence encoding an amino acid sequence having the same biological function as the sequence set forth in SEQ ID NO: 10.
142.The nucleic acid of any one of embodiments 127 to 141, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 11.
143.The nucleic acid of any one of embodiments 127 to 142, wherein the first coding region or the second coding region comprises the sequence set forth in SEQ ID NO: 11.
144.The nucleic acid of any one of embodiments 127 to 143, wherein the first coding region or the second coding region comprises a sequence having the same biological function as the sequence set forth in SEQ ID NO: 11.
145.The nucleic acid of any one of embodiments 127 to 144, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 12.
146.The nucleic acid of any one of embodiments 127 to 145, wherein the first coding region or the second coding region comprises a sequence encoding the sequence set forth in SEQ ID NO: 12.
147.The nucleic acid of any one of embodiments 127 to 146, wherein the first coding region or the second coding region comprises a sequence encoding an amino acid sequence having the same biological function as the sequence set forth in SEQ ID NO: 12.
148.The nucleic acid of any one of embodiments 127 to 147, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 13.
149.The nucleic acid of any one of embodiments 127 to 148, wherein the first coding region or the second coding region comprises the sequence set forth in SEQ ID NO: 13.
150.The nucleic acid of any one of embodiments 127 to 149, wherein the first coding region or the second coding region comprises a sequence having the same biological function as the sequence set forth in SEQ ID NO: 13.
151.The nucleic acid of any one of embodiments 127 to 150, wherein the first coding region or the second coding region comprises a sequence encoding an amino acid sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 14.
152.The nucleic acid of any one of embodiments 127 to 151, wherein the first coding region or the second coding region comprises a sequence encoding the sequence set forth in SEQ ID NO: 14.
153.The nucleic acid of any one of embodiments 127 to 152, wherein the first coding region or the second coding region comprises a sequence encoding an amino acid sequence having the same biological function as the sequence set forth in SEQ ID NO: 14.
154.The nucleic acid of any one of embodiments 127 to 153, wherein the first coding region or the second coding region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 15.
155.The nucleic acid of any one of embodiments 127 to 154, wherein the first coding region or the second coding region comprises the sequence set forth in SEQ ID NO: 15.
156.The nucleic acid of any one of embodiments 127 to 155, wherein the first coding region or the second coding region comprises a sequence having the same biological function as the sequence set forth in SEQ ID NO: 15.
157.The nucleic acid of any one of embodiments 127 to 156, wherein the second promoter region results in a moderate expression of the second coding region operably linked thereto.
158.The nucleic acid of any one of embodiments 127 to 157, wherein in c) the Fe²⁺ and/or ISC sensitive region is an HRE.
159.The nucleic acid of embodiment 158, wherein the HRE comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 20.
160.The nucleic acid of embodiment 158 or 159, wherein the HRE comprises or consists of the sequence set forth in SEQ ID NO: 20.
161.The nucleic acid of any one of embodiments 158 to 160, wherein the HRE comprises or consists of a sequence having the same biological function as the sequence set forth in SEQ ID NO: 20.
162.The nucleic acid of any one of embodiments 158 to 161, wherein the second promoter region comprises or consists of five, six, seven, eight, nine or ten HRE.
163.The nucleic acid of any one of embodiments 158 to 162, wherein the second promoter region comprises or consists of eight HRE.
164.The nucleic acid of any one of embodiments 127 to 163, wherein the second promoter region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 21.
165.The nucleic acid of any one of embodiments 127 to 164, wherein the second promoter region comprises or consists of the sequence set forth in SEQ ID NO: 21.
166.The nucleic acid of any one of embodiments 127 to 165, wherein the second promoter region comprises or consists of a sequence having the same biological function as the sequence set forth in SEQ ID NO: 21.
167.The nucleic acid of any one of embodiments 127 to 166, wherein the IRE in b) i) comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 22, and/or wherein the IRE in d) ii) comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 31, and/or wherein the IRE in d) ii) comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 32.
168.The nucleic acid of any one of embodiments 127 to 167, wherein the IRE in b) i) comprises or consists of the sequence set forth in SEQ ID NO: 22, and/or wherein the IRE in d) ii) comprises or consists of the sequence set forth in SEQ ID NO: 31, and/or wherein the IRE in d) ii) comprises or consists of the sequence set forth in SEQ ID NO: 32.
169.The nucleic acid of any one of embodiments 127 to 168, wherein the IRE in b) i) comprises or consists of a sequence having the same biological function as the sequence set forth in SEQ ID NO: 22, and/or wherein the IRE in d) ii) comprises or consists of a sequence having the same biological function as the sequence set forth in SEQ ID NO: 31, and/or wherein the IRE in d) ii) comprises or consists of a sequence having the same biological function as the sequence set forth in SEQ ID NO: 32.
170.The nucleic acid of any one of embodiments 127 to 169, wherein d) ii) comprises one, two, three, four, five, six, seven, eight, nine or ten IRE(s).
171.The nucleic acid of any one of embodiments 127 to 170, wherein d) ii) comprises five IREs, preferably wherein d) ii) comprises the sequence set forth in SEQ ID NO: 31 and the sequence set forth in SEQ ID NO: 32.
172.The nucleic acid of any one of embodiments 127 to 171, wherein an mRNA transcript of the second coding region comprises the IRE of d) ii) in a 3' untranslated region (UTR) of a sequence encoding d) i).
173.The nucleic acid of embodiment 172, wherein the 3'UTR comprises or consists of a part of a Transferrin Receptor (TfR) 3'UTR.
174.The nucleic acid of any one of embodiments 127 to 173, wherein the second coding region comprises of a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 23.
175.The nucleic acid of any one of embodiments 127 to 174, wherein the second coding region comprises or consists of the sequence set forth in SEQ ID NO: 23.
176.The nucleic acid of any one of embodiments 127 to 175, wherein the second coding region comprises or consists of a sequence having the same biological function as the sequence set forth in SEQ ID NO: 23.
177.The nucleic acid of any one of embodiments 127 to 176, wherein the first coding region comprises a 3' polyA tail region.
178.The nucleic acid of embodiment 177, wherein the polyA tail region is a BgH polyA tail region.
179.The nucleic acid of embodiment 177 or 178, wherein the polyA tail region comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 25.
180. The nucleic acid of any one of embodiments 177 to 179, wherein the polyA tail region comprises or consists of the sequence set forth in SEQ ID NO: 25.
181. The nucleic acid of any one of embodiments 177 to 180, wherein the polyA tail region comprises or consists of a sequence having the same biological function as the sequence set forth in SEQ ID NO: 25.
182.The nucleic acid of any one of embodiments 127 to 181, wherein the first coding region comprises a 3' terminator region.
183.The nucleic acid of embodiment 182, wherein the terminator region is 3' of b) ii).
184.The nucleic acid of embodiment 182 or 183, wherein the terminator region is 3' of a polyA tail region, preferably of the polyA tail region of any one of embodiments 177 to 181.
185.The nucleic acid of any one of embodiments 182 to 184, wherein the terminator region comprises a T7 terminator sequence and/or a Rrng terminator sequence.
186.The nucleic acid of any one of embodiments 182 to 185, wherein the terminator region comprises in 5' to 3'direction a T7 terminator sequence and a Rrng terminator sequence.
187.The nucleic acid of embodiment 182 or 186, wherein the T7 terminator sequence comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 26.
188.The nucleic acid of any one of embodiments 182 to 187, wherein the T7 terminator sequence comprises or consists of the sequence set forth in SEQ ID NO: 26.
189.The nucleic acid of any one of embodiments 182 to 188, wherein the T7 terminator sequence comprises or consists of a sequence having the same biological function as the sequence set forth in SEQ ID NO: 26.
190.The nucleic acid of any one of embodiments 182 to 189, wherein the Rrng terminator sequence comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 27.
191.The nucleic acid of any one of embodiments 182 to 190, wherein the Rrng terminator sequence comprises or consists of the sequence set forth in SEQ ID NO: 27.
192.The nucleic acid of any one of embodiments 182 to 191, wherein the Rrng terminator sequence comprises or consists of a sequence having the same biological function as the sequence set forth in SEQ ID NO: 27.
193.The nucleic acid of any one of embodiments 127 to 192, wherein the nucleic acid comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 28.
194.The nucleic acid of any one of embodiments 127 to 193, wherein the nucleic acid comprises the sequence set forth in SEQ ID NO: 28.
195.The nucleic acid of any one of embodiments 127 to 194, wherein the nucleic acid comprises a sequence having more than 80%, more than 85%, more than 90%, more than 95%, more than 96%, more than 97%, more than 98%, more than 99%, more than 99.5%, more than 99.9% sequence identity with the sequence set forth in SEQ ID NO: 24.
196.The nucleic acid of any one of embodiments 127 to 195, wherein the nucleic acid comprises the sequence set forth in SEQ ID NO: 24.
197.A vector comprising the nucleic acid of any one of embodiments 127 to 196.
198.The vector of embodiment 197, wherein the vector is a viral vector.
199.The vector of embodiment 198, wherein the viral vector is a lentivirus vector, an adenovirus, an adeno-associated virus (AAV), and/or a baculovirus.
200.The vector of embodiment 197, wherein the vector is a plasmid.
201.The vector of embodiment 200, wherein the plasmid is a VB240227-1627une plasmid or a PcDNA3.1-plasmid.
202.The vector of embodiment 200 or 201, comprising or consisting of the sequence set forth in SEQ ID NO: 29.
203.A cell comprising i) the nucleic acid of any one of embodiments 127 to 196 and/or the vector of any one of embodiments 197 to 202, and optionally ii) an iron responsive protein (IRP).
204.The cell of embodiment 203, wherein the first polypeptide and the second polypeptide are stably or transiently expressed in the cell.
205.The cell of embodiment 203 or 204, wherein the cell is a HeLa cell, a macrophage, an enterocyte, a hepatocyte, a cancer cell, an immune cell and/or a neuron.
206.An *in vitro* method for measuring cellular Fe²⁺ and/or cellular ISC, comprising:
   a. Providing the cell of any one of embodiments 203 to 205, and
   b. Measuring a signal of the first reporter protein domain and a signal of the second reporter protein domain,
      wherein intensity of the signal of the first reporter protein domain is indicative of an amount of cellular ISC, and/or
      wherein intensity of the signal of the second reporter protein domain is indicative of an amount of cellular Fe²⁺ and/or cellular ISC.
207.The method of embodiment 206, wherein the method further comprises prior or after step b):
   c. Amending an amount of extracellular and/or cellular Fe²⁺.
208.The method of embodiment 207, wherein the amount of Fe²⁺ and/or cellular ISC is amended by adding to the cell a composition comprising Fe²⁺.
209.The method of embodiment 208, wherein the composition comprises FeSO₄.
210.The method of embodiment 208 or 209, wherein the composition is FeSO₄ Vitamin C.
211. The method of embodiment 207, wherein the amount of Fe²⁺ and/or cellular ISC is amended by adding to the cell an Fe²⁺ chelator, preferably a cell membrane permeable Fe²⁺ chelator.
212.The method of embodiment 211, wherein the Fe²⁺ chelator is eltrombopag (ELT).
213.The method of embodiment 207, wherein the amount of Fe²⁺ and/or cellular ISC is amended by adding a compound affecting or being suspected of affecting cellular Fe²⁺ and/or cellular ISC.
214.The method of embodiment 213, wherein the compound is an active ingredient, a drug candidate and/or a drug.
215.The method of any one of embodiments 206 to 214, wherein step b) further comprises obtaining a ratio from the measured intensity of the signal of the first reporter protein domain and the measured intensity of the signal of the second reporter protein domain.
216.The method of any one of embodiments 207 to 215, wherein the method comprises after step c) another step b) referred to as step d).
217.The method of any one of embodiments 206 to 216, wherein the method further comprises
   e. Comparing measurements obtained from step d) with measurements obtained from step b) and/or a control.
218.The method of embodiment 217, wherein in step e) ratios are compared.
219.The method of any one of embodiments 206 to 218, wherein the first reporter protein domain is a first fluorescent protein domain, the second reporter protein domain is a second fluorescent protein domain, signal is fluorescence, and the method comprises:
   a. Providing the cell of any one of embodiments 203 to 205 and exciting the cell, and
   b. Measuring fluorescence of the first fluorescent protein domain and fluorescence of the second fluorescent protein domain,
      wherein intensity of fluorescence of the first fluorescent protein domain is indicative of an amount of cellular ISC, and/or
      wherein intensity of fluorescence of the second fluorescent protein domain is indicative of an amount of cellular Fe²⁺ and/or cellular ISC.
220.An *in vitro* screening method for a compound that affects cellular Fe²⁺ and/or cellular ISC the method comprising the steps of
   a. Providing the cell of any one of embodiments 203 to 205,
   b. Optionally measuring a signal of the first reporter protein domain and a signal of the second reporter protein domain and obtaining a ratio from the measured intensity of the signal of the first reporter protein domain and the measured intensity of the signal of the second reporter protein domain,
   c. Adding a compound affecting or being suspected of affecting cellular Fe²⁺ and/or cellular ISC,
   d. Measuring a signal of the first reporter protein domain and a signal of the second reporter protein domain and obtaining a ratio from the measured intensity of the signal of the first reporter protein domain and the measured intensity of the signal of the second reporter protein domain, and
   e. Comparing the ratio obtained from step d) with the ratio obtained from step b) and/or a control,
      wherein intensity of the signal of the first reporter protein domain is indicative of an amount of cellular ISC, and/or wherein intensity of the signal of the second reporter protein domain is indicative of an amount of cellular Fe²⁺ and/or cellular ISC, and
      wherein a significant change in the ratio obtained from step d) and the ratio obtained from step b) and/or a control is indicative of the compound affecting cellular Fe²⁺ and/or cellular ISC.
221.The method of embodiment 220, wherein the first reporter protein domain is a first fluorescent protein domain, the second reporter protein domain is a second fluorescent protein domain, signal is fluorescence, and the method comprises
   a. Providing the cell of any one of embodiments 203 to 205 and exciting the cell,
   b. Optionally measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain,
   c. Adding a compound affecting or being suspected of affecting cellular Fe²⁺ and/or cellular ISC,
   d. Measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain, and
   e. Comparing the ratio obtained from step d) with the ratio obtained from step b) and/or a control,
      wherein intensity of fluorescence of the first fluorescent protein domain is indicative of an amount of cellular ISC, and/or wherein intensity of fluorescence of the second fluorescent protein domain is indicative of an amount of cellular Fe²⁺ and/or cellular ISC, and
      wherein a significant change in the ratio obtained from step d) and the ratio obtained from step b) and/or a control is indicative of the compound affecting cellular Fe²⁺ and/or cellular ISC.
222.An *in vitro* method for identifying a dosage and/or formulation of a compound that affects cellular Fe²⁺ and/or cellular ISC, the method comprising the steps of
   a. Providing the cell of one of embodiments 203 to 205,
   b. Optionally measuring a signal of the first reporter protein domain and a signal of the second reporter protein domain and obtaining a ratio from the measured intensity of the signal of the first reporter protein domain and the measured intensity of the signal of the second reporter protein domain,
   c. Adding a compound affecting or being suspected of affecting cellular Fe²⁺ and/or cellular ISC in in a dosage and/or formulation,
   d. Measuring a signal of the first reporter protein domain and a signal of the second reporter protein domain and obtaining a ratio from the measured intensity of the signal of the first reporter protein domain and the measured intensity of the signal of the second reporter protein domain, and
   e. Comparing measurements obtained from step d) with measurements obtained from step b) and/or a control,
      wherein intensity of the signal of the first reporter protein domain is indicative of an amount of cellular ISC, and/or wherein intensity of the signal of the second reporter protein domain is indicative of an amount of cellular Fe²⁺ and/or cellular ISC, and
      wherein a significant change in the ratio obtained from step d) and the ratio obtained from step b) and/or a control is indicative of the dosage and/or formulation affecting cellular Fe²⁺ and/or cellular ISC.
223.The method of embodiment 222, wherein the first reporter protein domain is a first fluorescent protein domain, the second reporter protein domain is a second fluorescent protein domain, signal is fluorescence, and the method comprises
   a. Providing the cell of one of embodiments 215 to 217 and exciting the cell,
   b. Optionally measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain,
   c. Adding a compound affecting or being suspected of affecting cellular Fe²⁺ and/or cellular ISC in in a dosage and/or formulation,
   d. Measuring fluorescence of the first fluorescent protein domain and fluorescence of the second fluorescent protein domain and obtaining a ratio from the measured intensity of fluorescence of the first fluorescent protein domain and the measured intensity of fluorescence of the second fluorescent protein domain, and
   e. Comparing measurements obtained from step d) with measurements obtained from step b) and/or a control,
      wherein intensity of fluorescence of the first fluorescent protein domain is indicative of an amount of cellular ISC, and/or wherein intensity of fluorescence of the second fluorescent protein domain is indicative of an amount of cellular Fe²⁺ and/or cellular ISC, and
      wherein a significant change in the ratio obtained from step d) and the ratio obtained from step b) and/or a control is indicative of the dosage and/or formulation affecting cellular Fe²⁺ and/or cellular ISC.
224.The method of any one of embodiments 206 to 223, wherein the method further comprises prior step a) the step of contacting the cell with the vector of any one of embodiments 210 to 214.
225.The method of embodiment 206 to 224, wherein ISC is synthesized in presence of cellular Fe²⁺.
226.The method of any one of embodiments 206 to 225, wherein an ISC-IRP complex is formed by binding of ISC to IRP.
227.The method of any one of embodiments 206 to 226, wherein presence of ISC regulates translation of the first reporter protein domain.
228.The method of any one of embodiments 206 to 227, wherein translation of the first reporter protein domain is positively regulated in a high cellular Fe²⁺ condition, preferably by binding of the ISC-IRP complex to the IRE in the first coding region.
229.The method of any one of embodiments 206 to 228, wherein translation of the first reporter protein domain is negatively regulated in a low cellular Fe²⁺ condition.
230.The method of any one of embodiments 206 to 229, wherein presence of cellular Fe²⁺ and/or ISC regulates transcription and/or stability of an mRNA encoding the sequence encoding the second reporter protein domain.
231.The method of any one of embodiments 206 to 230, wherein transcription of the second reporter protein domain is positively regulated in a low cellular Fe²⁺ condition, preferably by binding of cellular Fe²⁺ and/or ISC to the Fe²⁺ and/or ISC sensitive region in the second promoter region.
232.The method of any one of embodiments 206 to 231, wherein transcription of the second reporter protein domain is negatively regulated in a high cellular Fe²⁺ condition.
233.The method of any one of embodiments 206 to 232, wherein stability of an mRNA encoding the sequence encoding the second reporter protein domain is negatively regulated in a high cellular Fe²⁺ condition, preferably by binding of the ISC-IRP complex to the IRE 3' of the sequence encoding the second reporter protein domain.
234.The method of any one of embodiments 206 to 233, wherein stability of the mRNA encoding the sequence encoding the second reporter protein domain is positively regulated in a low cellular Fe²⁺ condition.
235.The method of any one of embodiments 206 to 234, wherein the method is a ratiometric method.
236.The method of any one of embodiments 206 to 235, wherein the cell is excited at 405 nm, 470-490 nm, 561 nm, and/or 540-590 nm.
237.The method of any one of embodiments 206 to 236, wherein fluorescence is measured at 450 nm, 500-520 nm, and/or 565-650 nm.
238.The method of any one of embodiments 206 to 237, wherein fluorescence of the first fluorescent protein domain is excited at 405 nm and/or 470-490 nm and measured at 450 nm and/or 500-520 nm.
239.The method of any one of embodiments 206 to 238, wherein fluorescence of the second fluorescent protein domain is excited at 561 nm and/or 540-590 nm and measured at 565-650 nm.
240.The method of any one of embodiments 206 to 239, wherein fluorescence of the first fluorescent protein domain and fluorescence of the second fluorescent protein domain are measured by dynamic time-lapse imaging.
241.The method of any one of embodiments 206 to 240, wherein fluorescence of the first fluorescent protein domain and fluorescence of the second fluorescent protein domain are measured using a high-resolution microscopy.
242.The method of any one of embodiments 206 to 241, wherein step b) is performed repeatedly and/or continuously.
243. Use of the nucleic acid of any one of embodiments 127 to 196 to obtain the vector of any one of embodiments 197 to 202.
244. Use of the vector of any one of embodiments 197 to 202 to express the nucleic acid of any one of embodiments 127 to 196.
245. Use of the vector of any one of embodiments 197 to 202 to obtain the cell of any one of embodiments 203 to 205.
246. Use of the cell of any one of embodiments 203 to 205 for *in vitro* measuring and/or monitoring ferrous iron (Fe²⁺) in a cell, preferably according to the method of any one of embodiments 206 to 219 and 224 to 242.
247. Use of the cell any one of embodiments 203 to 205 for *in vitro* screening for a compound that affects cellular Fe²⁺, preferably according to the method of any one of embodiments 220, 221 and 224 to 242.
248. Use of the cell any one of embodiments 203 to 205 for *in vitro* identifying a dosage and/or formulation of a compound that affects cellular Fe²⁺, preferably according to the method of any one of embodiments 222 to 242.
249.A kit of nucleic acids comprising the nucleic acid of any one of embodiments 1 to 68 and the nucleic acid of any one of embodiments 127 to 196.
250.A kit of vectors comprising the vector of any one of embodiments 69 to 74 and the vector of any one of embodiments 197 to 202.
251.A vector comprising the nucleic acid of any one of embodiments 1 to 68 and the nucleic acid of any one of embodiments 127 to 196.
252.A cell comprising i) the nucleic acid of any one of embodiments 1 to 68 and the nucleic acid of any one of embodiments 127 to 196, and/or ii) the vector of any one of embodiments 69 to 74 and the vector of any one of embodiments 197 to 202.

## Claims

1. A nucleic acid, comprising
a. A promoter region;
b. A first coding region encoding a first polypeptide,
wherein the first polypeptide comprises a first reporter protein domain and a first Fe²⁺-sensitive domain;
c. A polypeptide separator region; and
d. A second coding region encoding a second polypeptide,
wherein the second polypeptide comprises a second reporter protein domain, and
wherein the first reporter protein domain and the second reporter protein domain are different.

2. The nucleic acid of claim 1, wherein
i) the polypeptide separator region is a ribosome skipping region, wherein preferably the ribosome skipping region encodes a peptide that causes ribosome skipping followed by recommencement of translation and/or wherein preferably the ribosome skipping region comprises a sequence encoding an amino acid sequence having more than 95%, 96%, 97%, 98%, or more than 99% sequence identity with the sequence set forth in SEQ ID NO: 2 and/or SEQ ID NO: 3,
ii) the first Fe²⁺-sensitive domain comprises a degron sequence, preferably wherein the first coding region comprises a sequence encoding an amino acid sequence having more than 80%, 85%, 90%, 95%, 96%, 97%, 98%, or more than 99% sequence identity with the sequence set forth in SEQ ID NO: 6,
iii) first Fe²⁺-sensitive domain comprises or consists of a Hr domain of FBXL5, preferably wherein the first coding region comprises a sequence encoding an amino acid sequence having more than 80%, 85%, 90%, 95%, 96%, 97%, 98%, or more than 99% sequence identity with the sequence set forth in SEQ ID NO: 8, and/or
iv) the second polypeptide comprises a second Fe²⁺-sensitive domain, preferably wherein the second Fe²⁺-sensitive domain comprises or consists of a potential iron sulfur cluster (ISC) binding domain.

3. A cell comprising the nucleic acid of claim 1 or 2.

4. An *in vitro* method for measuring cellular Fe²⁺, comprising:
a. Providing the cell of claim 3, and
b. Measuring a signal obtained from the first reporter protein domain and a signal obtained from the second reporter protein domain,
wherein at least intensity of the signal of the first reporter protein domain is indicative of an amount of cellular Fe²⁺.

5. The method of claim 4, wherein the first reporter protein domain is a first fluorescent protein domain and wherein the second reporter protein is a second fluorescent protein, wherein the signal obtained from the first fluorescent protein is a fluorescence of the first fluorescent protein, wherein the signal obtained from the second fluorescent protein domain is a fluorescence of the second fluorescent protein and wherein the method comprises
a. Providing the cell of claim 3 and exciting the cell, and
b. Measuring fluorescence of the first fluorescent protein domain and fluorescence of the second fluorescent protein domain,
wherein at least intensity of fluorescence of the first fluorescent protein domain is indicative of an amount of cellular Fe²⁺.

6. The method of claim 4 or 5, wherein
i) the second polypeptide comprises a second Fe²⁺-sensitive domain and/or wherein an intensity of the signal of the second reporter protein domain is indicative of an amount of cellular Fe²⁺, and/or
ii) the first reporter protein domain and/or the first polypeptide is stabilized by binding of cellular Fe²⁺ to the first Fe²⁺-sensitive domain and wherein the second reporter protein domain and/or the second polypeptide is degraded by binding of cellular Fe²⁺ to the second Fe²⁺-sensitive domain.

7. A nucleic acid, comprising in 5' to 3' direction:
a. A first promoter region,
b. A first coding region encoding
i. an iron responsible element (IRE), and
ii. a first reporter protein domain,
c. A second promoter region comprising an Fe²⁺ and/or ISC sensitive region,
d. A second coding region encoding
i. a second reporter protein domain,
wherein the first reporter protein domain and the second reporter protein domain are different, and
ii. an IRE.

8. The nucleic acid of claim 7, wherein
i) in c) the Fe²⁺ and/or ISC sensitive region is an HRE, preferably wherein the HRE comprises a sequence having more than 80%, 85%, 90%, 95%, 96%, 97%, 98%, or more than 99% sequence identity with the sequence set forth in SEQ ID NO: 20,
ii) the IRE in b) i) comprises a sequence having more than 80%, 85%, 90%, 95%, 96%, 97%, 98%, or more than 99% sequence identity with the sequence set forth in SEQ ID NO: 22,
iii) the IRE in d) ii) comprises a sequence having more than 80%, 85%, 90%, 95%, 96%, 97%, 98%, or more than 99% sequence identity with the sequence set forth in SEQ ID NO: 31,
iv) the IRE in d) ii) comprises a sequence having more than 80%, 85%, 90%, 95%, 96%, 97%, 98%, or more than 99% sequence identity with the sequence set forth in SEQ ID NO: 32, and/or
v) an mRNA transcript of the second coding region comprises the IRE of d) ii) in a 3' untranslated region (UTR) of a sequence encoding d) i), preferably wherein the 3'UTR comprises or consists of a part of a Transferrin Receptor (TfR) 3'UTR, more preferably wherein the 3'UTR comprises or consists of the sequence set forth in SEQ ID NO: 23.

9. The nucleic acid of nay one of claims 1, 2, 7 or 8, wherein
i) the nucleic acid is an expression cassette and/or
ii) the first reporter protein domain is a first fluorescent protein domain and wherein the second reporter protein domain is a second fluorescent protein domain.

10. A cell comprising i) the nucleic acid of any one of claims 7 to 9, and optionally ii) an iron responsive protein (IRP).

11. An *in vitro* method for measuring cellular Fe²⁺ and/or cellular ISC, comprising:
a. Providing the cell of claim 10, and
b. Measuring a signal obtained from the first reporter protein domain and a signal obtained from the second reporter protein domain, preferably wherein step b) further comprises obtaining a ratio from the measured intensity of the signal of the first reporter protein domain and the measured intensity of the signal of the second reporter protein domain
wherein intensity of the signal of the first reporter protein domain is indicative of an amount of cellular ISC, and/or
wherein intensity of the signal of the second reporter protein domain is indicative of an amount of cellular Fe²⁺ and/or cellular ISC.

12. The method of claim 11, wherein ISC is synthesized in presence of cellular Fe²⁺ and/or wherein an ISC-IRP complex is formed by binding of ISC to IRP.

13. The method of claim 11 or 12, wherein translation of the first reporter protein domain is positively regulated in a high cellular Fe²⁺ condition, preferably by binding of the ISC-IRP complex to the IRE in the first coding region.

14. The method of any one of claims 11 to 13, wherein
i) transcription of the second reporter protein domain is positively regulated in a low cellular Fe²⁺ condition, preferably by binding of cellular Fe²⁺ and/or ISC to the Fe²⁺ and/or ISC sensitive region in the second promoter region, and/or
ii) stability of an mRNA encoding the sequence encoding the second reporter protein domain is negatively regulated in a high cellular Fe²⁺ condition, preferably by binding of the ISC-IRP complex to the IRE 3' of the sequence encoding the second reporter protein domain.

15. The method of any one of claims 11 to 14, wherein the first reporter protein domain is a first fluorescent protein domain and wherein the second reporter protein is a second fluorescent protein, wherein the signal obtained from the first fluorescent protein is a fluorescence of the first fluorescent protein, wherein the signal obtained from the second fluorescent protein domain is a fluorescence of the second fluorescent protein and wherein the method comprises:
a. Providing the cell of claim 10 and exciting the cell, and
b. Measuring fluorescence of the first fluorescent protein domain and fluorescence of the second fluorescent protein domain, preferably wherein step b) further comprises obtaining a ratio from the measured intensity of fluorescence of the first fluorescent protein domain and the measured intensity of fluorescence of the second fluorescent protein domain
wherein intensity of fluorescence of the first fluorescent protein domain is indicative of an amount of cellular ISC, and/or
wherein intensity of fluorescence of the second fluorescent protein domain is indicative of an amount of cellular Fe²⁺ and/or cellular ISC.
